# EUROPEAN PATENT APPLICATION

(11) **EP 2 017 334 A2**
(43) Date of publication of application: **21.01.2009**
(21) Application number: 08017463.4
(22) Date of filing: 02.11.2005
(51) Int. Cl.: C12N 9/02

(54) **Method of isolating P450 Gene**

(30) Priority: 10.11.2004 JP 2004326139; 23.03.2005 JP 2005083019; 23.03.2005 JP 2005083122
(62) Divisional of application: 05799974.0
(71) Applicant: KNC Laboratories Co., Ltd, Kobe Hyogo 651-2271 (JP)
(72) Inventor: Kubota, Mitsutoshi, Hachioji-shi Tokyo 1920373 (JP); Nodate, Miho, deceased (JP); Uchiyama, Taku, Inba-gun Chiba 2701516 (JP); Misawa, Norihiko, Kamaishi-shi Iwate 026-0001 (JP)
(74) Representative: Woods, Geoffrey Corlett

(57) **Abstract**

The present invention provides a method for preparing a hybrid gene. The method includes a step of amplifying a P450 gene fragment contained in a sample using primers designed on the basis of regions of a plurality of P450 in which amino acid sequences are highly conserved and a step of preparing the hybrid gene using the amplified fragments and a known P450 gene. The method includes no culturing step or a step of normalizing extracted DNAs and is useful in isolating a P450 gene from various microbial resources.
The present invention further provides a fused cytochrome P450 monooxygenase containing a peptide which is linked to the C-terminus of a P450 protein with a linker portion disposed therebetween and which has the same function as that of a reductase domain contained in a cytochrome P450 monooxygenase originating from Rhodococcus sp. strain NCIMB 9784. This enables the construction of a high-efficiency electron transfer system useful for various P450 proteins and also enables the production of an active P450 monooxygenase.

## Description

### Technical Field

The present invention relates to a method for isolating a gene encoding a protein serving as a cytochrome P450 (P450), oligonucleotides useful for the method, a novel gene isolated by the method, a protein encoded by the gene, a fused P450 monooxygenase containing the protein, and a method for producing an oxidized compound using the enzyme. The isolation method according to the present invention can be used to isolate an enzyme gene without isolating microorganisms such as bacteria from seawater samples or soil samples, without cultivating such microorganisms, or without normalizing DNA samples extracted from the cultivated or isolated microorganisms. In particular, the isolation method is useful in isolating a novel P450 gene from genetic resources in the natural environment in the case that only a small number of microorganisms having a target gene are present in the genetic resources. P450 synthesized from a gene obtained by the isolation method according to the present invention can be used as a biocatalyst for producing products useful for the chemical industry.

### Background Art

A cytochrome P450 monooxygenase is present in various organisms such as bacteria, plants, and mammals and is an iron- and sulfur-containing enzyme that catalyzes a reaction (monooxygenation) that produces an oxidation product in such a manner that one atom of an oxygen molecule is reduced into one mole of water and the other one is introduced in to a low-molecular-weight organic compound. Although the molecular structure and catalytic function of P450s are essentially not varied, the structure of a substrate-binding site thereof is varied. Hence, P450s are suitable for not only the hydroxylation of a hydrocarbon but also various oxidation reactions such as dealkylation, epoxidation, C-C bond cleavage, aromatization, and dehydrogenation. Examples of the reaction catalyzed by P450s include various biochemical reactions such as the biosynthesis of animal hormones and secondary plant metabolites, the activation of foreign substances in organisms, and the biodegradation of such foreign substances. Since P450s are involved in the oxidation of various organic compounds, bio-industries have investigated P450s. In particular, the following techniques have been being investigated: the synthesis of new low-molecular-weight organic compounds and the decomposition of environmentally hazardous substances by the catalysis of P450, the compounds and substances being hardly oxidized by known methods. In view of industry, for the purpose of the application of bioconversion, the use of P450s originating from microorganisms such as bacteria has been recently disclosed (Non-patent Document 1). Examples of bacterial P450s usually investigated include P450cam originating from Pseudomonas putida and P450BM3 originating from Bacillus megaterium. P450cam is the first of which the crystal structure has been elucidated. P450BM3 has a structure fused with a reductase necessary for the oxidation of the P450. For these enzymes, attempts have been made to improve their activity and/or substrate specificity by molecular evolution engineering, i. e., site-directed mutagenesis (Non-patent Document 1). However, the number of types of P450s originating from known bacteria is extremely less as compared to the diversity of the bacteria. On the other hand, recent advances in genome analysis have offered new possibilities for P450 investigation. That is, the completion of the genome analysis of various bacteria has revealed the presence of various types of bacterial P450s. For example, Mycobacterium tuberculosis has 20 types of P450 sequences and Streptomyces coelicolor has 18 types of P450 sequences. Attempts have been made to use these novel P450 sequences as oxidase resources for bioconversion. Although various types of P450s have been isolated and analyzed in recent years, P450s can be obtained only from microorganisms that can be isolated and cultivated. However, the number of species of such isolatable, cultivable microorganisms is 1% or less of that of microorganisms present in soil or sea (Non-patent Document 2). Hence, the number of types of identified microbial P450s is limited and various types of P450s that are difficult to isolate or identify are probably present in environments. It cannot be denied that unidentified P450s includes ones involved in the oxidation of various organic compounds. In view of industry, it is very worthy to directly isolate various genes, such as P450 genes originating from microorganisms (uncultivable microorganisms) that cannot be isolated or cultivated or are difficult to isolate or cultivate, from the environments to analyze their functions quickly.

A method called cassette PCR is an example of a technique for isolating or recovering a target gene from the environment without isolating or cultivating microorganisms and has been developed by Okuta et al. (Non-patent Document 3). This method is as follows: from a known protein with a target function or an amino acid sequence or nucleotide sequence of a gene encoding the protein, a primer specific to the gene is designed; DNA amplification such as PCR is performed using a DNA, directly extracted from an environmental sample such as a soil sample or a brine sample, as a template; obtained DNA fragments are isolated; and both ends of the gene that cannot be amplified using the primer are supplemented with nucleotide sequences of a known gene similar to that gene. This allows the formation of a hybrid gene including a new gene sequence originating from the DNA directly extracted from the environment sample and known gene sequences located at both ends of the hybrid gene, the new gene sequence being located at a center region of the hybrid gene. Therefore, if the hybrid gene is inserted into a protein expression system parasitic on Escherichia coli, functions of an obtained gene can be efficiently analyzed. The homology between a sequence of a primer used for DNA amplification and an existing protein or gene sequence used to obtain basic information for designing the primer is critical in obtaining various novel gene sequences by this method. However, since P450s have various primary structures, any primer useful in efficiently isolating diverse P450 genes from known sequences has not been obtained yet. In the direct recovery of DNA from an environmental sample such as a soil sample, a large number of DNA fragments originating from major microorganisms of which the number is huge are obtained. If a library is prepared using the DNA fragments, the library contains a large number of genes originating from the major microorganisms. Therefore, when a target gene is in microorganisms (minor microorganisms) of which the number is small, the isolation of this gene is difficult. A technique for sequring the diversity of DNAs of genes originating from minor microorganisms is as follows: DNAs directly extracted from an environmental sample such as a soil sample are normalized using the difference in GC contents of the DNAs in the sample, and a library is then prepared (Patent Document 1). This technique cannot be used when GC contents of major microorganisms are close to those of minor microorganisms.

Examples of a compound that can be converted by the catalysis of P450s include petroleum compounds such as alkanes (linear hydrocarbons). Such alkanes are converted into diols or dicarboxylic acids by oxidizing both terminal groups of the alkanes and the diols or dicarboxylic acids are used as raw materials for producing polymers, perfumes, or medicines. Although these products are currently synthesized by petrochemical processes, the replacement of the petrochemical processes with biochemical processes is desired; hence, the biochemical oxidation of terminal groups of alkanes has been attracting much attention. Examples of P450s that can oxidize terminal groups of an alkane include a CYP52 family present in yeasts such as Candida maltosa and Candida tropicalis (Non-patent Document 4). This document discloses that Candida maltosa has four types of genes belonging to a CYP52A sub-family whose expression is induced by alkanes and the specificities of these genes are different from each other depending on the type or length of substrates such as alkanes and fatty acids. A P450, supposed to be involved in the decomposition of alkanes, originating from prokaryote has been first discovered in a strain of Acinetobacter calcoaceticus EB104 that is a bacterium that utilizes a long-chain n-alkane (Non-patent Document 5). P450s have been recently discovered in several types of bacteria, such as Alcanivorax borkumensis and Rhodococcus erythropolis, having the ability to utilize alkanes. These types of P450 form the CYP153A subfamily (Non-patent Document 6 and Dr. Nelson's homepage (http://drnelson.utmem.edu/CytchromeP450.html)). However, there is no direct evidence that these types of P450 convert terminal groups of alkanes into hydroxyl groups. It has been disclosed that the following cytochrome has an ability to oxidize alkanes with three to eight carbon atoms: P450BM-3 139-3 which is a mutant of P450 originating from Bacillus megaterium and which has been obtained by molecular evolution engineering (Non-patent Document 7). The P450 gene is of a P450/reductase-fused type and is efficiently expressed in Escherichia coli; hence, the P450 gene has been expected to be used for bioconversion processes. However, since the P450 does not convert terminal groups of alkanes but principally convert branches thereof into hydroxyl groups, the P450 has not been put to practice use. As described above, A P450 that can oxidize such alkane terminal groups is expected to be used for industrial use and have been therefore intensively investigated. However, findings about P450s are limited and the amount of information on P450s originating from bacteria is particularly small.

P450 monooxygenases are classified into two classes on the basis of the difference in electron transfer. A first class (Class I) includes P450 monooxygenases in which electrons are supplied from NAD(P)H to a P450 protein, by way of an NAD(P)H-iron-sulfur protein (ferredoxin) reductase that contains a flavin adenine dinucleotide (FAD) as a coenzyme and an iron-sulfur protein (ferredoxin) (the monooxygenases of this class are hereinafter referred to as three-component-containing P450 monooxygenases in some cases). P450 monooxygenases contained in mitochondria or bacteria usually belong to this class. A second class (Class II) includes P450 monooxygenases in which an NADPH P450 reductase containing a FAD and a flavin mononucleotide (FMN) that are coenzymes supplies a P450 protein with electrons from NADPH, in which any iron-sulfur protein is not needed (the monooxygenases of this class are hereinafter referred to as two-component-containing P450 monooxygenases in some cases). P450 monooxygenases present in microsomes from mammal liver belong to this class.

In recent years, a large number of novel genes encoding cytochromes P450 (CYP) have been discovered because of the advance in analyzing bacterial genomes. However, most of genes encoding electron transfer proteins corresponding to P450s have not been discovered. In 45 species of Streptomyces expected to be useful in synthesizing, for example, medicines, 170 or more of P450 genes have been discovered; however, only less than 20 types of electron transfer proteins corresponding to the P450 genes have been discovered (Parajuli et al., Genome analyses of Streptomyces peucetius ATCC 27952 for the identification and comparison of cytochrome P450 complement with other Streptomyces, Arch Biochem Biophys, 425: 233-241, 2004).

Some examples show that a bacterial enzyme is activated using an electron transfer protein present in the bacterium or a P450 electron transfer protein present in another bacterial strain or organism although an electron donor corresponding to the electron transfer protein is unknown. The following fact has been disclosed: some types of Streptomyces cytochromes P450 exhibit P450 monooxygenase activity in the presence of ferredoxin (putidaredoxin) and a putidaredoxin reductase originating from Pseudomonas putida (Arisawa A, et al., Bioconversion by Escherichia coli in which actinomycete cytochrome P450 and electron transfer protein are coexpressed, Annual Meeting of Japan Society for Bioscience, Biotechnology, and Agrochemistry 2003, p. 38, 2003). Furthermore, the following fact has been disclosed: P450 (PB-1) originating from Corynebacterium sp. strain EP1 exhibits activity in the presence of an electron donor originating from spinach (Kawahara N, et al., Purification and characterization of 2-ethoxyphenol-induced cytochrome P450 from Corynebacterium sp. strain EP1., Can J Microbiol. 45: 833-839, 1999). However, the electron donors and electron transfer proteins described above cannot be necessarily applied to all types of P450; hence, an electron transfer protein compatible with P450 is sought for its use. In order to find such a compatible electron transfer protein without extraordinary efforts, the investigation of P450s needs to be focused on a specific bacterial strain or a P450 family including proteins homologous to each other. Even if an electron transfer protein functioning well can be obtained, a large amount of time must be spent optimizing gene manipulation and induction conditions (for example, whether structural genes are carried on different plasmids or the same plasmid, the order of the structural genes, the timing of inducing the structural genes) for the purpose of coexpressing genes for the electron transfer protein and P450 compatible therewith in a bacterium. In the analysis of P450 functions in vitro, reductases are separately produced, purified, and then mixed such that an optimum mixing ratio can be achieved; hence, a large amount of time and resources are spent analyzing one P450 function. Therefore, the amount of information about functions of P450 genes is small although there is a vast amount of information about the structures of the P450 genes. Hence, it is difficult to perform high-throughput selection, that is, it is difficult to efficiently select an enzyme having a catalytic function or specificity to a target substrate among various types of P450s or difficult to quickly find a substrate catalyzed by P450s.

In general, P450 has a problem in that the activity per cytochrome P450 molecule is low because the rate of an enzymatic reaction depends on the transfer of electrons from an electron donor to a cytochrome P450. The following enzyme is probably a key for solving the problem: a fused P450 monooxygenase, which is a rare example, containing a single polypeptide chain including a P450 protein and reductase linked to each other. Well-known examples are P450BM3 (Non-patent Document 8) originating from Bacillus megaterium and P450foxy originating from a mold. These cytochrome P450 monooxygenases contain a P450 protein similar to that contained in a two-component P450 monooxygenase belonging to Class II and also contain a NADPH-P450 reductase containing FAD and FMN that are coenzymes. These P450s are different from the two-component P450 monooxygenase in that the two components thereof are linked together with a linker portion disposed therebetween to form a single peptide chain. The fused P450 monooxygenase has an advantage in that the enzymatic reaction rate and activity thereof are greater than those of three- or two-component P450 monooxygenases. The reason for the advantage is that the fusion between the P450 protein and the reductase probably allows the intramolecular transfer of electrons, resulting in the formation of a three-dimensional structure due to the best interaction between the P450 protein and the reductase. Various types of artificial fused P450 monooxygenases originating from eukaryotes have been prepared in imitation of the fused P450 monooxygenase. There is, for example, an artificial fusion enzyme that includes a human liver P450 protein bound to the N-terminus thereof and an NADPH-P450 reductase, originating from yeast, rat, or P450BM3 described above, bound to the C-terminus thereof. Although P450BM3 originates from a bacterium, its P450 heme domain and its NADPH-P450 reductase domain has high homology with P450s and P450 reductases, respectively, originating from eukaryotes, belonging to Class II; hence, the P450BM3 can probably act as an electron donor. P450BM3, which is bacterial P450 as described above, is apparently different from a three-component P450 monooxygenase, originating from an ordinary bacterium, belonging to Class I.

Except for the case of P450BM3, There is only one successful example of the preparation of a fusion enzyme including a P450 reductase domain prepared using a bacterial P450 protein (a successful example of the preparation of an active fusion enzyme). In this example, components of a P450 monooxygenase that are present in the same system are only fused together (Sibbesen, O., et al. Putidaredoxin reductase-putidaredoxin-cytochrome P450cam triple fusion protein. Construction of a self-sufficient Escherichia coli catalytic system. J Biol Chem. 271: 22462-9, 1996). There is no successful example of an attempt to fuse an ordinary bacterial P450 protein and a reductase domain originating from a P450 monooxygenase component together. This is probably because the function system of bacterial P450 contains the P450 protein, ferredoxin, and the ferredoxin reductase as described above and is thus complicated. In the industrial use of bacterial P450 monooxygenases, an artificial fusion enzyme enables simple enzymatic purification or intracellular reaction; however, any technique for preparing the artificial fusion enzyme has been established yet.

A P450 monooxygenase that is not similar to the P450 monooxygenases belonging to Class I or II nor the fused P450 monooxygenase has been recently disclosed (Non-patent Document 8). This P450 monooxygenase (named P450RhF) is isolated from Rhodococcus sp. strain NCIMB 9784 and is a single peptide containing a P450 protein (a heme domain), a NADH-P450 reductase domain containing a flavin mononucleotide (FMN) that is a coenzyme, and an iron-sulfur protein domain. This P450 monooxygenase (P450RhF) is novel in that the reductase domain requires the FMN only. This is because reductases present in P450 monooxygenases of Class II or known fused P450 monooxygenases requires both FAD and FMN. Accordingly, P450RhF is exceptional among P450 monooxygenases.
Patent Document 1: PCT Japanese Translation Patent Publication No. 2000-513933
Non-patent Document 1: Urlacher et al., Appl. Microbiol. Biotechnol., 64: 317-325 (2004)
Non-patent Document 2: Thomas et al., Proc. Natl. Acad. Sci., 99: 10494-10499 (2002)
Non-patent Document 3: Okuta, A. et al., Gene, 212: 221-228 (1998)
Non-patent Document 4: Ohkuma et al., J. Biol. Chem., 273: 3948-3953 (1998)
Non-patent Document 5: Thomas et al., Biochem. Biophy. Res. Comm., 286: 652-658 (2001)
Non-patent Document 6: Beilen et al., biocat 2004 Book of Abstracts, p. 233
Non-patent Document 7: Glieder et al., Nat. Biotechol., 20: 1135-1139 (2002)
Non-patent Document 8: Roberts, G.A. et al., Identification of a new class of cytochrome P450 from a Rhodococcus sp. J. Bacteriol., 184: 3898-3908, 2002.

### Disclosure of Invention

### Problems to be Solved by the Invention

It is a first object of the present invention to provide a method for efficiently isolating various P450 genes from microbial resources present in various environments and particularly from scarce microorganisms present in such environments, to provide a novel P450 gene, to provide P450 protein synthesized using the novel P450 gene, and to provide a technique for oxidizing various organic compounds using the enzyme.

Since P450 monooxygenases are variable in substrate specificity and reaction specificity, the P450 monooxygenases have been expected to be used for various useful biocatalytic reactions. However, most of P450 proteins (CYP) have no catalytic activity by theirselves. Hence, if a novel P450 gene is discovered, a large amount of time and extraordinary efforts are required to identify a substrate catalyzed by P450 encoded by the gene and to put P450 to practical use. In order to utilize enzymatic activity (catalytic activity) that the P450 proteins must have inherently, active P450 monooxygenases must be restructured. This requires electron transfer proteins for supplying electrons to the P450 proteins. However, an electron donor corresponding to a P450 and particularly to a novel P450 cannot be readily discovered and a system containing a P450 monooxygenase has low enzymatic activity and is easily inactivated. Therefore, a system, applicable to various P450 proteins, for efficiently transferring electrons has been demanded. It is a second object of the present invention to provide a method for structuring such a system to produce an active P450 monooxygenase.

### Means for Solving the Problems

In order to achieve the first object, the inventors have performed intensive investigation. The inventors have found that amino acid sequences are well conserved in regions of the CYP153A subfamily including a plurality of P450 genes originating from microorganisms utilizing petroleum components. Furthermore, the inventors have found that fragments of novel P450 genes can be amplified from various environmental DNA samples using primers designed on the basis of the regions. The inventors have developed a technique in which a hybrid gene between an amplified P450 gene and a known P450 gene is prepared by a cassette PCR method and then analyzed for function using an Escherichia coli expression system. The inventors have further performed investigation and found that products of hybrid genes containing novel P450 sequences obtained from environments have monooxygenase activity and catalyze the oxidation of organic compounds such as alkanes (linear hydrocarbons), alkenes (linear hydrocarbons containing unsaturated bonds), cyclic hydrocarbons, alkyl aromatics, and alkenyl aromatics. In particular, the products catalyze the hydroxylation of a terminal methyl group and the epoxidation of a terminal olefin group.

In order to achieve the second object, the inventors have attempted to develop a versatile, efficient system for transferring electrons to a P450. During the attempt, the inventors have paid attention to a cytochrome P450 monooxygenase (P450Rhf) from Rhodococcus sp. strain NCIMB 9784. As disclose in Section "Background Art", P450Rhf is the fused P450 monooxygenase that is present in the form of a single peptide chain formed by the fusion of the P450 protein and the P450 reductase domain (region). The fused P450 monooxygenase is different from P450BM3 or P450foxy as described above and is novel in that the reductase domain contains the iron-sulfur protein-like component and the FMN protein-like component (Non-patent Document 1). In general, Actinobacteria such as Rhodococcus have various catalytic functions involved in the conversion of various organic components and also have complicated enzyme systems. Therefore, the use of P450Rhf, which is a novel enzyme, for general purposes has been supposed to be difficult. It is usually difficult to express a gene, involved in an enzyme system from a Rhodococcus sp. strain, in a multipurpose host such as an Escherichia coli strain. Hence, in view of common sense, it has been supposed that P450Rhf cannot be used for the purpose of the inventors, that is, the development of the versatile, efficient system for transferring electrons to a P450. The inventors have investigated if the reductase domain of P450Rhf can be used to transfer electrons to various bacterial P450 proteins and if the reductase domain can be allowed to serve as a fusion protein for inducing intramolecular electron transfer. As a result, this investigation showed that a P450 monooxygenase which has catalytic activity and which is hardly inactivated can be prepared by fusing the P450Rhf reductase domain and the C-terminus of a bacterial P450 protein with an appropriate linker region disposed therebetween.

In particular, the inventors have prepared a single polypeptide gene in such a manner that a gene encoding a bacterial cytochrome P450 protein (CYP) is linked to a gene encoding the reductase domain of P450Rhf from Rhodococcus sp. strain NCIMB 9784, thereby structuring a system for expressing an artificial fusion enzyme gene encoding an artificial fusion enzyme having monooxygenase activity originating from the P450 and reducing ability originating from the P450Rhf reductase domain. Furthermore, the inventors have structured such an expression system using different bacterial P450 genes, thereby allowing an Escherichia coli strain to produce an artificial fusion enzyme. The inventors have confirmed that the produced artificial fusion enzyme, which is a single molecule, has electron transfer ability and substrate oxidation ability and that the expression system is applicable to various P450 genes. Furthermore, the inventors have discovered that if the expression system is used, the biocatalytic activity of a P450 can be determined by a simple bioconversion reaction using culture of Escherichia coli.

The present invention has been made on the basis of the above findings.

The present invention is as specified in Items (1) to (23) below.
(1) An oligonucleotide, having substantially a primer or probe function, for isolating a P450 gene fragment contains a nucleotide sequence encoding part or all of the amino acid sequence set forth in SEQ ID NO: 51 or 52 or a nucleotide sequence complementary thereto.
(2) An oligonucleotide, having substantially a primer or probe function, for isolating a P450 gene fragment contains the nucleotide sequence set forth in any one of SEQ ID NOS: 53 to 56 or part or all of a nucleotide sequence complementary thereto.
(3) A method for isolating a P450 gene fragment includes a step of using the oligonucleotide according to Item (1) or (2) as at least one primer.
(4) A method for isolating a P450 gene fragment includes a step of extracting a nucleic acid from a sample and a step of performing nucleic acid amplification using the nucleic acid and the oligonucleotide according to Item (1) or (2) as a template and a primer, respectively.
(5) A method for preparing a hybrid P450 gene includes a step of isolating a P450 gene fragment using the oligonucleotide according to Item (1) or (2) as at least one primer and a step of adding a 5'-terminal region and 3'-terminal region of a known P450 gene to the 5'-terminus and 3'-terminus, respectively, of the isolated P450 gene.
(6) In the method according to Item (5), the known P450 gene encodes the amino acid sequence set forth in SEQ ID NO: 57.
(7) A kit for isolating a P450 gene includes:
   (a) the oligonucleotide according to Item (1) or (2);
   (b) a DNA fragment containing a 5'-terminal region of a known P450 gene; and
   (c) a DNA fragment containing a 3'-terminal region of a known P450 gene.
(8) A gene encodes the protein specified in any one of Items (a) and (b) below:
   (a) a protein containing the amino acid sequence set forth in any one of SEQ ID NOS: 1 to 25; and
   (b) a protein, serving as P450, containing an amino acid sequence prepared by removing one or more amino acid residues from the amino acid sequence set forth in any one of SEQ ID NOS: 1 to 25, replacing one or more amino acid residues of this amino acid sequence with other residues, or adding one or more amino acid residues to this amino acid sequence.
(9) A gene encodes a protein, serving as P450, containing the nucleotide sequence set forth in any one of SEQ ID NOS: 26 to 50.
(10) An expression vector containing the gene according to Item (8) or (9).
(11) A protein is specified in any one of Items (a) and (b) below:
   (a) a protein containing the amino acid sequence set forth in any one of SEQ ID NOS: 1 to 25; and
   (b) a protein, serving as P450, containing an amino acid sequence prepared by removing one or more amino acid residues from the amino acid sequence set forth in any one of SEQ ID NOS: 1 to 25, replacing one or more amino acid residues of this amino acid sequence with other residues, or adding one or more amino acid residues to this amino acid sequence.
(12) A method for producing a protein serving as P450 includes a step of transforming the expression vector according to Item (10) into a host cell and then culturing the host cell and a step of recovering the protein according to Item (11) from the cultured cells.
(13) A method for producing an oxidized compound includes a step of subjecting a substrate to a reaction in the presence of the protein according to Item (11) to produce an oxidized compound.
(14) A fused cytochrome P450 monooxygenase contains a peptide which is linked to the C-terminus of a P450 protein with a linker portion disposed therebetween and which has the same function as that of a reductase domain contained in a cytochrome P450 monooxygenase originating from Rhodococcus sp. strain NCIMB 9784.
(15) In the fused cytochrome P450 monooxygenase according to Item (14), the peptide is (a) one containing the amino acid sequence set forth in SEQ ID NO: 70; (b) one containing an amino acid sequence prepared by adding one or more amino acid residues to the amino acid sequence set forth in SEQ ID NO: 70; removing one or more amino acid residues from this amino acid sequence, or replacing one or more amino acid residues of this amino acid sequence with other residues; or (c) one encoded by a DNA hybridized with a DNA containing the nucleotide sequence set forth in SEQ ID NO: 69 or a DNA complementary to this DNA under stringent conditions.
(16) In the fused cytochrome P450 monooxygenase according to Item (14) or (15), the linker portion is (d) a peptide containing the amino acid sequence set forth in SEQ ID NO: 68; (e) a peptide containing an amino acid sequence prepared by adding one or more amino acid residues to the amino acid sequence set forth in SEQ ID NO: 68; removing one or more amino acid residues from this amino acid sequence, or replacing one or more amino acid residues of this amino acid sequence with other residues; or (f) a peptide encoded by a DNA hybridized with a DNA containing the nucleotide sequence set forth in SEQ ID NO: 67 or a DNA complementary to this DNA under stringent conditions.
(17) In the fused cytochrome P450 monooxygenase according to any one of Items (14) to (16), the P450 protein originates from a bacterium.
(18) In the fused cytochrome P450 monooxygenase according to any one of Items (14) to (16), the P450 protein is identical to the protein according to Item 11 or a protein containing the amino acid sequence set forth in SEQ ID NO: 85.
(19) A DNA encodes the fused cytochrome P450 monooxygenase according to any one of Items (14) to (18).
(20) A microorganism contains the DNA according to Item (19).
(21) A method for producing a fused cytochrome P450 monooxygenase includes a step of culturing the microorganism according to Claim 20 and a step of recovering a fused cytochrome P450 monooxygenase from the cultured microorganisms or cells thereof.
(22) A method for producing an oxidized compound includes a step of subjecting a substrate to a reaction in the presence of the fused cytochrome P450 monooxygenase according to any one of Claims 14 to 18 to produce an oxidized compound.
(23) In the method according to Item (22), the substrate is n-hexane, n-heptane, n-octane, n-decane, 1-octene, cyclohexane, n-butylbenzene, 4-phenyl-1-butene, or 2-n-butylbenzofran and the oxidized compound is 1-hexanol, 1-heptanol, 1-octanol, 1-decanol, 1,2-epoxyoctane, cyclohexanol, 4-phenyl-1-butanol, 2-phenethyloxirane, or 4-benzofran-2-yl-butane-1-ol.
(24) A gene encodes the protein specified in any one of Items (a) and (b) below:
   (a) a protein containing the amino acid sequence set forth in SEQ ID NO: 85; and
   (b) a protein, serving as P450, containing an amino acid sequence prepared by removing one or more amino acid residues from the amino acid sequence set forth in SEQ ID NO:85, replacing one or more amino acid residues of this amino acid sequence with other residues, or adding one or more amino acid residues to this amino acid sequence.
(25) A gene encodes a protein, serving as P450, containing the nucleotide sequence set forth in SEQ ID NO: 86.

### Advantages

The present invention provides a method for efficiently isolating a novel P450 gene from a sample, such as an environmental sample, containing various microbial nucleic acids. Cytochromes P450 synthesized from the P450 genes obtained by the method can be used to produce various low-molecular-weight organic compounds.

Fused P450 monooxygenases according to the present invention are prepared from known P450 proteins and unknown P450 proteins. The fused P450 monooxygenases is of a single component type which has electron transfer ability and substrate oxidation ability. Hence, an experiment for reconstructing an electron transfer protein and a reaction system is not necessary although such an experiment had been necessary to achieve P450 monooxygenase activity. This leads to practical applications such as the high throughput screening of useful active biocatalysts, the simple purification of a useful P450 enzyme, a bioconversion process for producing a useful substance by use of a microorganism containing a fusion enzyme gene, and the oxidative removal of harmful substances in industrial waste water.

### Brief Description of the Drawings

[Fig. 1] Fig. 1 is an illustration showing a phylogenetic tree based on the homology between amino acid sequences (SEQ ID NOS: 1 to 25) of obtained novel P450s and amino acid sequences of known P450s.
[Fig. 2] Fig. 2 includes graphs showing changes in the absorption spectra of Crude Extract Solution A (Fig. 2A), Crude Extract Solution B (Fig. 2B), and Crude Extract Solution C (Fig. 2C) treated with carbon monoxide under reductive conditions.
[Fig. 3] Fig. 3 includes charts obtained by analyzing reaction solutions obtained by subjecting octane and 1-decene to enzyme reactions using Crude Enzyme Solutions B and D.
[Fig. 4] Fig. 4 is an illustration showing the structure of an expression plasmid for preparing fused P450.
[Fig. 5] Fig. 5 is a graph showing the comparison between the productions of 1-alcohols produced by oxidizing n-alkanes with six to eight carbon atoms.
[Fig. 6] Fig. 6 includes charts obtained by analyzing inducers by gas chromatography-mass spectrometry, the inducers being converted from reaction products prepared by subjecting n-butylbenzene to reactions using Bacterial Suspension H5, H9, or Alk or control Bacterial Suspension Non.
[Fig. 7] Fig. 7 includes charts obtained by analyzing reaction products by gas chromatography-mass spectrometry, the reaction products being obtained by subjecting 4-phenyl-1-butene to reactions using Bacterial Suspension H5, H9, or Alk or control Bacterial Suspension Non.
[Fig. 8] Fig. 8 includes graphs showing optical properties of artificial fused P450cam (Fig. 8A) and non-fused P450cam (Fig. 8B).
[Fig. 9] Fig. 9 is a graph showing the conversion of camphor to 5-hydroxycamphor by use of a strain of Escherichia coli producing fused or non-fused P450cam.
[Fig. 10] Fig. 10 is a graph showing the conversion of n-octane to 1-octanol by use of a strain of Escherichia coli producing fused or non-fused P450alk.
[Fig. 11] Fig. 11 is a graph showing the conversion of 4-hydroxybenzoic acid to 3,4-dihydroxybenzoic acid by use of a strain of Escherichia coli producing fused or non-fused P450bzo.

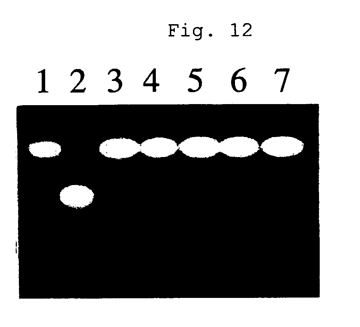

[Fig. 12] Fig. 12 is a chart showing the conversion of 7-ethoxycoumarin to 7-hydroxycoumarin by use of a strain of Escherichia coli producing fused or non-fused P450SU-1 or fused or non-fused P450SU-2, wherein Lane Number 1 corresponds to 7-ethoxycoumarin, Lane Number 2 corresponds to 7-hydroxycoumarin, Lane Number 3 corresponds to a strain containing no P450, Lane Number 4 corresponds to a strain containing pETP450SU-1, Lane Number 5 corresponds to a strain containing pSU-1RED, Lane Number 6 corresponds to a strain containing pETP450SU-2, and Lane Number 7 corresponds to a strain containing pSU-2RED.

### Best Mode for Carrying Out the Invention

### 1. P450 Monooxygenase

A P450 monooxygenase is a complex enzyme that requires an electron transfer protein in addition to a cytochrome P450 protein (CYP). In order that P450 monooxygenases are active, P450 monooxygenases present in bacteria or mitochondria require a ferredoxin reductase and ferredoxin that are proteins for transferring electrons from NAD(P)H and P450 monooxygenases present in microsomes from mammal liver require an NADPH-P450 reductase. The primary structure of the P450 monooxygenase is characterized in that a common sequence of FXXGXR/HXCXG is 50 to 60 residues apart from the C-terminus thereof. This sequence is referred to as a heme-binding region, which is characteristic of the P450 monooxygenase. When this region is present in an amino acid sequence predicted from a nucleotide sequence, a DNA thereof is defined as a gene that encodes the P450 monooxygenase (Omura et al., Molecular Biology of P450, Kodansha Scientific, 2003). P450 genes are classified according to the degree of similarity between amino acid sequences and the latest information about the classification is available on Dr. Nelson's homepage (http://drnelson.utmem.edu/CytchromeP450.html). According to the nomenclature authorized therein, a gene that belongs to the P450 superfamily has the prefix CYP followed by a family number, a subfamily number, and a gene number. Genes with amino acid sequences of which 40% or more are classified as the same family and genes with amino acid sequences of which 55% or more are classified as the same subfamily in principle.

As used herein, the term "monooxygenase activity" means the ability to catalyze a reaction in which one mole of water is produced by reducing one atom of an oxygen molecule and a low-molecular-weight organic compound is converted into an oxidation product by introducing the other one into the compound. The term "P450 monooxygenase" means a protein that has a difference spectrum (CO difference spectrum) with a maximum at 450 nm determined by applying carbon monoxide to the protein in a reduced state and also has monooxygenase activity.

### 2. P450 Monooxygenase CYP153 Family

A CYP153A1 monooxygenase belonging to the P450 monooxygenase CYP153 family has been first discovered in an Acinetobacter calcoaceticus EB104 strain (Non-patent Document 5). Thereafter, P450 monooxygenases belonging to the CYP153 family have been discovered in several species of microorganisms with the ability to utilize alkanes (Dr. Nelson's homepage (http://drnelson.utmem.edu/CytchromeP450.html). There is a report on a function of a P450 monooxygenase belonging to the CYP153 family (Non-patent Document 6). The report discloses that a CYP153A1 gene is expressed in a Pseudomonas putida host with no ability to utilize an alkane (octane) and the host is grown in a medium containing octane. From the observation of the host, the report suggests that the host has a P450 monooxygenase introducing hydroxyl groups into terminal groups of octane molecules. There is no report providing direct evidence that any P450 monooxygenase as well as the P450 monooxygenase belonging to the CYP153 family has the ability to introduce hydroxyl groups into terminal groups of alkanes. The inventors are the first to report a new P450 monooxygenase with that ability. Ordinary microbial P450 monooxygenases probably require a ferredoxin reductase and ferredoxin that are electron transfer proteins for catalyzing oxidation.

### 3. Cassette PCR Method

### (1) Preparation of oligonucleotides for isolating P450 gene fragment

Oligonucleotides (hereinafter referred to as "present oligonucleotides") for isolating a fragment of a P450 gene according to the present invention contains a nucleotide sequence encoding part or all of the amino acid sequence set forth in SEQ ID NO: 51 or 52 or a nucleotide sequence complementary thereto and has substantially a primer function. Since the present oligonucleotides are used as primers, such a P450 gene fragment can be isolated. As used herein, the term "oligonucleotide" means a deoxyribonucleotide or a ribonucleotide and the term "nucleic acid" means DNA or RNA.

The present oligonucleotides are designed on the basis of amino acid sequences each present in CYP153A1 (accession no. AJ311718), CYP153A2 (accession no. AE005680), or CYP153A13a (SEQ ID NO: 57) registered in Dr. Nelson's homepage (http://drnelson.utmem.edu/CytchromeP450.html). That is, the present oligonucleotides are designed on the basis of P450-conserved regions predicted from the alignment of these sequences.

The present oligonucleotides are designed to have substantially a primer function. As used herein, the clause "an oligonucleotide has substantially a primer function" means that an oligonucleotide meets requirements for specific annealing or hybridizing, that is, an oligonucleotide has a length and nucleotide composition (melting temperature) sufficient for specific annealing or hybridizing. This means that the present oligonucleotides are different from an oligonucleotide which has a small sequence length or an unsuitable nucleotide composition and which therefore usually promotes nonspecific annealing. The present oligonucleotides preferably have a length of ten bases or more, more preferably 16 bases or more, and most preferably 18 bases or more. Preferable examples of the present oligonucleotides include oligonucleotides containing the base sequences set forth in SEQ ID NOS: 53 to 56.

The present oligonucleotides may contain assembly sequences, linked to their 5'-terminuses, for preparing a hybrid gene described below. The assembly sequences are defined as sequences that can be annealed with a known P450 gene fragment. P450 gene fragments amplified using the present oligonucleotides as a forward primer or a reverse primer only have a sequence corresponding to a region sandwiched between the forward primer and the reverse primer. That is, the amplified P450 gene fragments lack a 5'-terminal sequence containing an initiation codon and a 3'-terminal sequence containing a termination codon. These lacking sequences can be supplemented in such a manner that sequences are annealed with the 5'- or 3'-terminal fragment of a known P450 gene and then subjected to an elongation reaction. The sequences annealed with the 5'- or 3'-terminal fragment of the known P450 gene correspond to the assembly sequences. When one of the present oligonucleotides is used as the forward primer, one of the assembly sequences is usually the 3'-terminal sequence of a known 5'-terminal fragment. When the other one of the present oligonucleotides is used as the reverse primer, the other one of the assembly sequences is usually the 5'-terminal sequence of a known 3'-terminal fragment. Examples of the present oligonucleotides include oligonucleotides containing the assembly sequences.

The assembly sequences may have such a length that the assembly sequences can be annealed with known 5'- or 3'-terminal fragments and the amplification of nucleic acids is inhibited. The assembly sequences preferably have a length of five to 50 bases and more preferably ten to 20 bases.

The present oligonucleotides can be synthesized by a method, such as a phosphite method or a phosphotriester method, known to those skilled in the art.

### (2) Isolation of fragments of novel P450 gene

Fragments of a novel P450 gene can be isolated using the oligonucleotides specified in Subsection "(1) Preparation of oligonucleotides for isolating P450 gene fragment". In usual, such novel P450 gene fragments can be isolated by an amplification reaction using the present oligonucleotides as primers (this method is hereinafter referred to as "present isolation method").

In the present isolation method, any sample containing nucleic acid originating from a prokaryote can be used to isolate the novel P450 gene fragments. Examples of such a sample include artificially cultured microorganisms and environmental samples containing a variety of unknown microorganisms. Examples of such environmental samples include brine, freshwater, and soil. In the present isolation method, a nucleic acid is extracted from the sample in usual. The extracted nucleic acid may be DNA or RNA. DNA or RNA can be extracted by a method known to those skilled in the art. In the extraction of DNA, the following method can be used: a method including phenol extraction and ethanol precipitation, a method using glass beads, or another method. In the extraction of RNA, the following method can be used: a guanidine-cesium chloride ultracentrifugal separation method, a hot phenol method, an acidguanidine thiocyanate-phenol-chloroform (AGPC) method, or another method. When the sample is brine or freshwater, the sample is preferably concentrated before nucleic acids are extracted therefrom. The sample can be concentrated by filtering the sample with a filter with an appropriate pore size. A material for forming the filter is not particularly limited and any material used to prepare ordinary filters may be used. Examples of such a material include cellulose acetate, nitrocellulose, cellulose-mixed esters, polyvinylidene difluoride, polytetrafluoroethylene, polycarbonates, polypropylene, and silver. The following method has been disclosed: a method in which a carrier bearing a culture medium is placed in water and nucleic acids from microorganisms attached to the carrier are efficiently recovered (Japanese Unexamined Patent Application Publication No. 2003-334064). After cells are lysed with a surfactant such as SDS as required, nucleic acids can be extracted from the sample obtained as described above by any one of the above methods.

When the sample is soil, nucleic acids can be extracted by a method known to those skilled in the art. Examples of a method for extracting nucleic acids from soil include the Zhou et al. method (Zhou et al., Appl. Environ. Microbiol., 62: 316-322 (1996)) and the Griffiths et al. method (Griffiths et al., Appl. Environ. Microbiol., 66: 5488-5491 (2000)).

The P450 gene fragments can be isolated from the nucleic acid obtained as described above using the oligonucleotides specified in Subsection "(1) Preparation of oligonucleotides for isolating P450 gene fragment". The P450 gene fragments can be specifically amplified by a nucleic acid amplification reaction in such a manner that the nucleic acid obtained as described above is used as a template and the oligonucleotides according to the present invention are used as primers. In usual, one of the present oligonucleotides that is designed on the basis of the amino acid sequence set forth in SEQ ID NO: 51 is used as a forward primer and the other one designed on the basis of the amino acid sequence set forth in SEQ ID NO: 52 is used as a reverse primer. The present oligonucleotides used in such a nucleic acid amplification reaction may be degenerated primers. A method for performing the nucleic acid amplification reaction is not particularly limited and any method using the present oligonucleotides as primers can be used. Examples of the most preferable method include a known method using a polymerase chain reaction (PCR) technique. The amplification reaction is continued until an amplified product is detected. The PCR technique is used to synthesize a nucleotide sequence between a pair of primers using a DNA polymerase. According to the PCR technique, amplification fragments can be exponentially amplified by repeating a cycle of denaturation, annealing, and synthesis. Those skilled in the art can determine optimum conditions of PCR. An RT-PCR technique is as follows: cDNA is prepared by a reverse transcriptase reaction using RNA as a template and PCR is performed using a pair of primers and the prepared cDNA as a template. In the case that the present oligonucleotides are used, those skilled in the art can determine conditions for specifically amplifying the P450 gene on the basis of the length and nucleotide composition of the present oligonucleotides. Amplified fragments of the P450 gene can be obtained in such a manner that PCR products are fractionated by agarose gel electrophoresis or the like, amplified bands are excised, and DNA is then extracted.

### (3) Method for preparing hybrid P450 gene

The P450 gene fragments amplified by the method specified in Subsection "(2) Isolation of fragments of novel P450 gene" each contain only a sequence corresponding to a domain sandwiched between regions annealed with the present oligonucleotides. That is, the P450 gene fragments contain no 5'-terminal region containing an initiation codon or 3'-terminal region containing a termination codon. Therefore, The P450 gene fragments amplified by the method specified in Subsection (2) have no function for forming the P450 gene in some cases. In order to solve this problem and in order to obtain DNA fragments useful in preparing the P450 gene, both terminuses of each P450 gene fragment amplified by the method specified in Subsection (2) may be supplemented with sequences contained in a known P450 gene. Any P450 gene originating from a known bacterium can be used to supplement the terminuses thereof and a known P450 gene containing nucleotide sequences encoding the amino acid sequences set forth in SEQ ID NOS: 51 and 52 is preferably used. Examples of such a known P450 gene include genes belonging to CYP153A of the 450 superfamily registered in Dr. Nelson's homepage
(http://drnelson.utmem.edu/CytchromeP450.html).

A hybrid P450 gene can be prepared by supplementing both terminuses of the P450 gene fragments amplified by the method specified in Subsection (2) with sequences contained in the known P450 gene. The hybrid P450 gene contains a nucleotide sequence, contained in an unknown P450 gene, located at a center region of the hybrid P450 gene and also contains nucleotide sequences, contained in the known P450 gene, located at both end regions thereof. A cytochrome P450 can be produced by introducing the hybrid P450 gene into a host. The hybrid P450 gene can be prepared in such a manner that the P450 gene fragments amplified by the method specified in Subsection (2) are annealed with the 5'-terminal region (corresponding to a region from an initiation codon to a portion annealed with the forward primer used in the amplification method specified in Subsection (2) and hereinafter referred to as "5-arm fragment") of the known P450 gene or the 3'-terminal region (corresponding to a region from a portion annealed with the reverse primer used in the amplification method specified in Subsection (2) to a termination codon and hereinafter referred to as "3-arm fragment") thereof and then subjected to an elongation reaction.

Primers used to prepare the hybrid P450 gene may contain the assembly sequences described in Subsection "(1) Preparation of oligonucleotides for isolating P450 gene fragment". The use of such primers containing the assembly sequence allows the amplified P450 gene fragments to be efficiently annealed with the 5- or 3-arm fragment. The 5-and 3-arm fragments can be prepared by a nucleic acid amplification reaction using DNA, containing a gene similar to the known P450 gene for supplementing both terminal regions, as a template. The primer can be designed and synthesized by a technique known to those skilled in the art. Conditions of the nucleic acid amplification reaction can be determined on the basis of data known to those skilled in the art. The hybrid P450 gene, prepared in such a manner that the P450 gene fragments amplified by the method specified in Subsection (2) are annealed with the 5-arm fragment or the 3-arm fragment and then subjected to an elongation reaction, can be amplified by a nucleic acid amplification reaction. The 5- and 3-arm fragments can be directly used as primers for this amplification reaction and oligonucleotide primers containing the following sequences are preferably used: a nucleotide sequence corresponding to a region containing an initiation codon identical to that contained in the 5-arm fragment and a nucleotide sequence corresponding to a region containing a termination codon identical to that contained in the 3-arm fragment. These primers can be designed and synthesized by a technique known to those skilled in the art. Conditions of this amplification reaction can be determined on the basis of data known to those skilled in the art. The hybrid P450 gene can be isolated in such a manner that amplified nucleic acid fragments are fractionated by agarose gel electrophoresis or the like, amplified bands are excised, and DNA is then extracted.

Whether the P450 gene fragments prepared by the method specified in Subsection (2) and the hybrid P450 gene prepared by the above method are genes encoding P450 can be determined by checking whether an amino acid sequence predicted from a nucleotide sequence has a common sequence of FXXGXR/HXCXG that is 50 to 60 residues apart from the C terminus. Alternatively, the above matter can be determined in such a manner that an expression vector containing the obtained nucleic acid fragments is introduced into an appropriate host and cells of the cultured host or a crude solution prepared by disrupting the cells and then subjecting the disrupted cells to extraction is measured for CO difference spectrum and monooxygenase activity.

The preparation of the expression vector, the selection of the host, and the introduction of the expression vector into the host are disclosed in various laboratory manuals (for example, Sambrook, J., Russel, D. W., Molecular Cloning, A Laboratory Manual, 3rd Edition, CSHL Press, 2001) and thus may be performed by a genetic engineering technique disclosed therein.

Examples of the expression vector introduced into the host include phages, phagemid vectors, and plasmid vectors. The host is not particularly limited to eukaryotic cells or prokaryotic cells and any host in which the expression vector can survive may be used. Examples of the host include animal cells, yeast cells, actinomycetes, and bacterial cells. Preferable examples of the bacterial cells include Escherichia coli cells and Bacillus subtilis cells. The expression vector needs to have an expression control region, such as a promoter, located upstream of the gene fragments or hybrid gene inserted therein. When the host is, for example, Escherichia coli, the expression control region is a lac promoter or the like. The host containing the expression vector is cultured in a culture medium suitable for the host and cells of the cultured host or a crude solution prepared by disrupting the cells and then subjecting the disrupted cells to extraction is measured for CO difference spectrum and monooxygenase activity. The measurement of CO difference spectrum can be performed in such a manner that the crude solution is treated with sodium dithinaite (Na₂S₂O₄), carbon monoxide is applied to the resulting solution, and the absorption spectrum of the resulting solution is measured, whereby a difference spectrum with a maximum at 450 nm is obtained. The measurement of monooxygenase activity can be performed in such a manner that a crude solution prepared by subjecting cells of the cultured host to extraction is allowed to react with a substrate and whether the substrate is oxidized is checked, the host being cultured in the presence of a low-molecular-weight organic compound under conditions that an expression vector containing the P450 gene fragments or the hybrid gene and an electron transfer protein is expressed or cultured such that the expression vector is expressed.

The P450 gene fragments or the hybrid P450 gene can be obtained by extracting DNA from the clone cells cultured by the above method.

### 4. P450 Gene-isolating Kit

A P450 gene-isolating kit (hereinafter referred to as "present kit") according to the present invention is used to isolate the hybrid P450 gene specified in Section "3. Cassette PCR Method". The present kit contains the oligonucleotides, 5-arm fragment, and 3-arm fragment specified in Section "3. Cassette PCR Method".

### (1) Primers for first stage of nucleic acid amplification reaction

As described in Section "3. Cassette PCR Method", in order to amplify the hybrid P450 gene, the nucleic acid amplification reaction must be performed in two stages. In the first stage of the nucleic acid amplification reaction, the present oligonucleotides specified in Subsection "(1) Preparation of oligonucleotides for isolating P450 gene fragment" are used as a forward primer or a reverse primer. In particular, one of the present oligonucleotides that contains a nucleotide sequence encoding part or all of the amino acid sequence set forth in SEQ ID NO: 51 is used as the forward primer and the other one containing a sequence complementary to a nucleotide sequence encoding part or all of the amino acid sequence set forth in SEQ ID NO: 52 is used as the reverse primer. The present kit contains at least one pair of the forward and reverse primers. The forward and reverse primers may be degenerated primers.

The forward and reverse primers contained in the present kit may contain assembly sequences.

### (2) 5-arm Fragment and 3-arm Fragment

The present kit contains the 5- and 3-arm fragments specified in Section "3. Cassette PCR Method". The 5-arm fragment is defined as a nucleic acid fragment containing a region, similar to that contained in a known P450 gene, from an initiation codon to a portion encoding the amino acid sequence set forth in SEQ ID NO: 51. The 3-arm fragment is defined as a nucleic acid fragment containing a region, similar to that contained in the known P450 gene, from a portion encoding the amino acid sequence set forth in SEQ ID NO: 52 to a termination codon. If the primers specified in Subsection "(1) Preparation of oligonucleotides for isolating P450 gene fragment" contain the assembly sequences, the 5- and 3-arm fragments need not contain the portion encoding the amino acid sequence set forth in SEQ ID NO: 51 or 52. The known P450 gene is used to prepare the 5- and 3-arm fragments and any bacterial P450 gene may be used for such a purpose. The known P450 gene preferably contains nucleotide sequences encoding the amino acid sequences set forth in SEQ ID NOS: 51 and 52. The 5- and 3-arm fragments may be a single- or double-stranded DNA.

### (3) Other Components

The present kit may contain a buffer for the nucleic acid amplification reaction, a dNTP mixture, enzymes (DNA polymerases and other enzymes), and the like. The present kit may contain a forward primer designed on the basis of a region containing an initiation codon of the 5-arm fragment and a reverse primer designed on the basis of a region containing a termination codon of the 3-arm fragment.

### 5. Novel P450 gene

A novel P450 gene (hereinafter referred to as "present P450 gene") according to the present invention encodes the protein specified in Item (a) or (b) below.
(a) A protein containing the amino acid sequence set forth in any one of SEQ ID NOS: 1 to 25 and 85.
(b) A protein, serving as P450, containing an amino acid sequence prepared by removing one or more amino acid residues from the amino acid sequence set forth in any one of SEQ ID NOS: 1 to 25 and 85, replacing one or more amino acid residues of this amino acid sequence with other residues, or adding one or more amino acid residues to this amino acid sequence.

The protein containing the amino acid sequence set forth in any one of SEQ ID NOS: 1 to 25 other than SEQ ID NO: 85 is produced by a hybrid P450 gene prepared on the basis of DNA, extracted from an environmental sample, by the method specified in Subsection "(3) Method for preparing hybrid P450 gene" of Section "3. Cassette PCR Method". The protein containing the amino acid sequence set forth in SEQ ID NO: 85 is encoded by a novel gene (SEQ ID NO: 86) isolated from a Nocardiaceae Hou Blue strain which is gram-positive. Examples of a gene encoding the protein specified in Item (a) include a gene represented by the nucleotide sequence set forth in any one of SEQ ID NOS: 26 to 50 and 86.

The protein specified in Item (b) is one that is prepared by introducing a small number of amino acid mutations into the protein specified in Item (a) such that the protein does not lose any P450 function. Examples of the amino acid mutations include natural amino acid mutations and artificial amino acid mutations. Examples of a technique for introducing such artificial amino acid mutations include a procedure for the construction of site-specific mutations (Nucleic acids res. 10, 6487-6500, 1982) and the technique is not limited to the procedure. The number of the amino acid mutations is not particularly limited and any number of the amino acid mutations may be used as long as this protein has monooxygenase activity. The number of the amino acid mutations is preferably 20 or less and more preferably 10 or less.

The present P450 gene can be prepared by the method specified in Subsection "(3) Method for preparing hybrid P450 gene" of Section "3. Cassette PCR Method" using a CYP153A13a gene cloned from Alcanivorax borkumensis SK2 encoding the amino acid sequence set forth in SEQ ID NO: 57.

The present P450 gene can be prepared by a procedure below. A central sequence originating from DNA extracted from an environmental sample is synthesized by a known DNA- synthesizing method. A 5-arm fragment and a 3-arm fragment are amplified by a nucleic acid amplification reaction using a set of primers represented by the nucleotide sequences set forth in SEQ ID NOS: 54 and 61 or a set of primers represented by the nucleotide sequences set forth in SEQ ID NOS: 56 and 62 in such a manner that DNA extracted from Alcanivorax borkumensis SK2 is used as a template. An elongation reaction is performed in such a manner that the synthesized central sequence is annealed with the 5- and 3- arm fragments. The present P450 gene can be amplified by a nucleic acid amplification reaction using a set of primers represented by the nucleotide sequences set forth in SEQ ID NOS: 61 and 62. The amplified present P450 genes can be then obtained in such a manner that reaction products are fractionated by agarose gel electrophoresis or the like and a fractionated band of about 1.4 Kbp is excised.

DNA fragments obtained by the above procedure can be determined to be identical to the present P450 gene in such a manner that the nucleotide sequences thereof are determined by a known method such as the dideoxy chain termination method or the Maxam-Gilbert method.

The present P450 gene can be prepared on the basis of nucleotide sequence data by chemical synthesis other than the above method.

### 6. Novel P450

Novel P450 according to the present invention is a protein specified in Item (a) or (b) below.
(a) A protein containing the amino acid sequence set forth in any one of SEQ ID NOS: 1 to 25 and 85.
(b) A protein, serving as P450, containing an amino acid sequence prepared by removing one or more amino acid residues from the amino acid sequence set forth in any one of SEQ ID NOS: 1 to 25 and 85, replacing one or more amino acid residues of this amino acid sequence with other residues, or adding one or more amino acid residues to this amino acid sequence.

The novel P450 can be prepared in such a manner that an expression vector containing the P450 gene specified in Section "5. Novel P450 gene" is introduced into a host cell and the resulting cell is cultured in a culture medium. The expression vector can be prepared and then introduced into the host cells by the known methods as described above. The novel P450 prepared as described above can be purified in such a manner that the cultured host cells are disrupted and then treated by a method, such as ion-exchange chromatography, partition chromatography, gel permeation chromatography, or affinity chromatography, widely used in the purification and/or production of protein. A P450 fraction treated by this method can be determined depending on the presence of CO difference spectrum.

### 7. Production of oxidized compound using P450

The present P450 serves as a catalyst and thus an oxidized compound can be produced using the present P450 (hereinafter referred to as "present production method"). Examples of the present production method include a method for producing the oxidized compound using a host cell into which an expression vector containing the present P450 gene has been introduced and a method for producing the oxidized compound using the purified present P450.

In order to allow the present P450 to catalyze oxidation, these methods usually require an electron transfer protein. For the use of a bacterium, a ferredoxin or a ferredoxin reductase is usually used as the electron transfer protein. For the production of the oxidized compound using the host cell, it is preferable that a gene encoding the electron transfer protein be co-expressed or a fused protein gene encoding the electron transfer protein and the present P450 be co-expressed. Alternatively, an electron transfer protein originating from the host cell may be used. For the production of the oxidized compound using the purified present P450, that electron transfer protein is preferably used in combination with NAD(P)H.

Any compound catalyzed by the present P450 can be used as a substrate in the present production method. According to the present production method, for example, an alcohol can be produced from an alkane and an epoxide can be produced from an alkene.

For the use of the purified enzyme, the present production method may be performed in a solution or in such a manner that the purified present P450 is immobilized on an appropriate carrier and then contacted with the substrate. The carrier for supporting the present P450 is not particularly limited. Examples of the carrier include inorganic carriers such as glass beads and silica gel beads; organic carriers made of cellulose, chitosan, Sepharose, dextran, polyvinyl alcohol, an ethylene-vinyl acetate copolymer, polyacrylamide, a polyacrylic acid, a polymethacrylic acid, polymethylmethacrylate, cross-linked polyvinyl alcohol, cross-linked polyacrylate, cross-linked polyacrylamide, cross-linked polystyrene, cross-linked cellulose, cross-linked agarose, or cross-linked dextran; and composite carriers made of these materials. The carrier preferably has a functional group, disposed thereon, for immobilizing the present P450. Examples of the functional group include a hydroxyl group, an amino group, an aldehyde group, a carboxyl group, a thiol group, a silanol group, an epoxy group, an amide group, a halogen group, a succinylimide group, and an acid anhydride group.

In the immobilization of the present P450 on the carrier, a hydrophilic spacer is preferably placed between the present P450 and the carrier such that the steric hindrance between the protein and the carrier is reduced, the efficiency of adsorption is thereby increased, and nonspecific adsorption is prevented. Examples of the hydrophilic spacer include organic molecules having terminal substituents such as carboxyl groups, amino groups, aldehyde groups, or epoxy groups.

### 8. Novel Fused Cytochrome P450 Monooxygenase

A novel fused cytochrome P450 monooxygenase has been recently disclosed (Roberts, G.A., et al, Identification of a new class of cytochrome P450 from a Rhodococcus sp. J. Bacteriol. 184: 3898-3908, 2002). This is a P450 monooxygenase (named "P450RhF") isolated from Rhodococcus sp. strain NCIMB 9784 and is a single peptide chain containing a P450 protein (heme domain), an NADH-P450 reductase domain (ferredoxin reductase-like sequence) containing a flavin mononucleotide (FMN) that is an coenzyme, and an iron-sulfur protein domain (ferredoxin-like sequence).

The fused cytochrome P450 monooxygenase contains a peptide which has a function similar to that of a reductase domain contained in the P450RhF and which is linked to the C-terminus of the P450 protein with a linker portion disposed therebetween.

Examples of the peptide include (a) a peptide containing the amino acid sequence set forth in SEQ ID NO: 70; (b) a peptide containing an amino acid sequence prepared by adding one or more amino acid residues to the amino acid sequence set forth in SEQ ID NO: 70, removing one or more amino acid residues from this amino acid sequence, or replacing one or more amino acid residues of this amino acid sequence with other residues; and (c) a peptide encoded by a DNA that is hybridized with a DNA containing the amino acid sequence set forth in SEQ ID NO: 69 or a DNA complementary to this DNA under stringent conditions.

The peptide specified in Item (a) is one containing a domain similar to the reductase domain (a region containing the NADH-P450 reductase domain and the iron-sulfur protein domain) contained in the P450RhF.

The peptide specified in Item (b) is one that is prepared by introducing a small number of amino acid mutations into the peptide specified in Item (a) such that functions of the peptide specified in Item (a) are not lost. Examples of the amino acid mutations include natural amino acid mutations and artificial amino acid mutations. Examples of a technique for introducing such artificial amino acid mutations include a procedure for the construction of site-specific mutations (Nucleic acids res. 10, 6487-6500, 1982) and the technique is not limited to the procedure. The number of the amino acid mutations is not particularly limited and any number of the amino acid mutations may be used as long as the functions of the peptide specified in Item (a) are not lost. The number of the amino acid mutations is preferably 20 or less and more preferably ten or less.

The peptide specified in Item (c) is one, prepared by the hybridization between DNAs, having the same function as that of the peptide specified in Item (a). The term "stringent conditions" specified in Item (c) means conditions under which only specific hybridization occurs but no nonspecific hybridization occurs. The conditions are usually approximately "1 × SSC, 0.1% SDS, and 37°C", preferably approximately "0.5 × SSC, 0.1% SDS, and 42°C", and more preferably approximately "0.2 × SSC, 0.1% SDS, and 65°C". A DNA prepared by hybridization usually has high homology with DNAs used for the hybridization. The term "high homology" means a homology of 60% or more, preferably a homology of 75% or more, and more preferably a homology of 90% or more.

The linker portion is used to fuse the two proteins (peptides) and any linker portion that does not remove functions of the proteins may be used. Preferable examples of the linker portion include (d) a peptide containing the amino acid sequence set forth in SEQ ID NO: 68; (e) a peptide containing an amino acid sequence prepared by adding one or more amino acid residues to the amino acid sequence set forth in SEQ ID NO: 68, removing one or more amino acid residues from this amino acid sequence, or replacing one or more amino acid residues of this amino acid sequence with other residues; and (f) a peptide encoded by a DNA that is hybridized with a DNA containing the nucleotide sequence set forth in SEQ ID NO: 67 or a DNA complementary to this DNA under stringent conditions.

The peptide specified in Item (d) is one present between the P450 protein and reductase domain of P450RhF.

The peptide specified in Item (e) is one that is prepared by introducing a small number of amino acid mutations into the peptide specified in Item (d) such that functions of the linker portion are not lost. Examples of the amino acid mutations include natural amino acid mutations and artificial amino acid mutations. Examples of a technique for introducing such artificial amino acid mutations include a procedure for the construction of site-specific mutations (Nucleic acids res. 10, 6487-6500, 1982) and the technique is not limited to the procedure. The number of the amino acid mutations is not particularly limited and any number of the amino acid mutations may be used as long as the functions of the linker portion are not lost. The number of the amino acid mutations is preferably five or less, more preferably three or less, most preferably one.

The peptide specified in Item (f) is one, prepared by the hybridization between DNAs, having the same function as that of the peptide specified in Item (d). The term "stringent conditions" specified in Item (f) has the same meaning as that of the term "stringent conditions" specified in Item (c).

The P450 protein is not particularly limited. Examples of the P450 protein include a protein produced using a DNA isolated from DNAs, extracted from a bacterial genomic DNA library or an environmental sample, by an ordinary method such as a PCR method and a protein produced using a hybrid gene or a gene prepared by introducing mutations into a known P450 gene by a PCR random mutation method or a DNA shuffling method. The P450 protein may originate from a bacterium, a plant, or an animal and preferably originates from such a bacterium in particular. A protein originating from Acinetobacter calcoaceticus is a preferable example of the P450 protein. Other examples of the P450 protein include the proteins specified in Section "6. Novel P450".

### 9. Production of oxidized compound using fused P450 monooxygenase

The P450 protein specified in Section "8. Novel Fused Cytochrome P450 Monooxygenase" serves as a catalyst and thus an oxidized compound can be produced using the P450 protein. Examples of a method for producing such an oxidized compound include a method for producing the oxidized compound using a host cell into which an expression vector containing a gene encoding the fused P450 monooxygenase has been introduced and a method for producing the oxidized compound using the purified fused P450 monooxygenase.

Both methods do not require the presence of electron transfer proteins such as ferredoxins and ferredoxin reductases (reductases) because the fused P450 monooxygenase contains the electron transfer protein. Therefore, if the oxidized compound is produced using the host cell, a gene encoding the electron transfer protein need not be coexpressed. If the oxidized compound is produced using the purified fused P450 monooxygenase, an additional electron transfer protein need not be used. The fused P450 monooxygenase can be purified by a method similar to the method specified in Section "7. Production of oxidized compound using P450".

Any compound catalyzed by the fused P450 monooxygenase can be used as a substrate in the present production method. Examples of an organic compound used as such a substrate include alkanes (linear hydrocarbons), alkenes (linear hydrocarbons containing unsaturated bonds), cyclic hydrocarbons, alkyl aromatics, and alkenyl aromatics. According to the present production method, an oxidized compound can be produced by the hydroxylation of a terminal methyl group or the epoxidation of a terminal olefin group. In particular, 1-hexanol, 1-heptanol, 1-octanol, 1-decanol, 1,2-epoxyoctane, cyclohexanol, 4-phenyl-1-butanol, 2-phenethyloxirane, or 4-benzofran-2-yl-butane-1-ol can be produced from n-hexane, n-heptane, n-octane, n-decane, 1-octene, cyclohexane, n-butylbenzene, 4-phenyl-1-butene, or 2-n-butylbenzofran, respectively.

### Examples

The present invention will now be described in detail with reference to examples. The present invention is not limited the examples.

### [Example 1] Preparation of environmental DNAs

In order to obtain P450 genes, various DNAs were extracted from environmental samples described below.

### 1. Samples obtained from the waters off Palau or Ponape

The following technique has been disclosed: a technique for immersed a carrier containing a culture medium in seawater and then efficiently recovering DNAs from microorganisms attached to the carrier (Japanese Unexamined Patent Application Publication No. 2003-334064). In this example, sterile artificial sponges were immersed in an NSW culture medium (0.1% NH₄NO₃, 0.002% ferric citrate, 0.002% K₂HPO₄, 0.05% yeast extract solution, 80% filtered seawater, and 1.5% agar), whereby the medium was allowed to permeate the artificial sponges. The agar in the medium was then solidified. The artificial sponges were placed in positions 5 to 6 m above the bottom of the waters off State of Ponape, Federated States of Micronesia, or the bottom of the waters off Republic of Palau. The artificial sponges were recovered three days later after the placement. The artificial sponges having microorganisms attached thereto were frozen and then crushed. DNAs were extracted from the microorganisms attached to the crushed sponges using Dneasy Plant Kit (Qiagen).

### 2. Freshwater samples

A sample of groundwater was obtained from an oil storage nucleotide in Kuji-shi, Iwate-ken, and a sample of spring water was obtained from Toyotomi Spring in Teshio-gun, Hokkaido. Microorganisms present in those samples were selectively concentrated using filters (GV type, 0.22 µm, 47 mm, Millipore). To each of the filters, 5 ml of a 10 mM Tris-HCl buffer solution (pH 8.0, 1mM EDTA, 0.35 M sucrose) and 200 µl of a 10 mg/ml proteinase K solution (a 10 mM Tris-HCl buffer solution) were added. The microorganisms were then incubated at 37°C for 30 minutes. After the incubation was finished, the solution mixture was vigorously stirred, whereby the microorganisms were suspended in the solution mixture. After precipitates were removed from the solution mixture, 7.5 ml of a lysing solution (a 100 mM Tris-HCl buffer solution, pH 8.0, 0.3 M NaCl, 20 mM EDTA, 2% sodium dodecyl sulfate) was added to the resulting solution mixture. The obtained mixture was vigorously stirred. To the stirred mixture, 10 ml of a solution in which the ratio of phenol to chloroform to isoamyl alcohol is 50 : 49 : 1 was added. After five minutes passed, this mixture was subjected to centrifugal separation (5000 rpm and 15 min) and the upper phase was then recovered. A twofold volume of 99% ethanol was added to the upper phase and this mixture was stirred and then cooled at -80°C for two hours. This mixture was subjected to centrifugal separation (15000 rpm and 15 min), whereby DNAs were precipitated. Cool 70% ethanol was added to this mixture, which was further subjected to centrifugal separation (15000 rpm and 15 min), whereby a precipitate fraction containing the DNAs was obtained.

### 3. Soil sample

A sample of oil-contaminated soil was obtained from an oil well in Nishiyama-cho, Niigata-ken. The following solutions were added to 5 g of the soil sample: 5 ml of a 10 mM Tris-HCl buffer solution (pH 8.0, 1mM EDTA, 0.35 M sucrose) and 200 µl of a 10 mg/ml proteinase K solution (a 10 mM Tris-HCl buffer solution). The mixture was subjected to incubation at 37°C for 30 minutes. After the incubation was finished, the mixture was vigorously stirred, whereby microorganisms were suspended in the mixture. After precipitates were removed from the mixture, 7.5 ml of a lysing solution (a 100 mM Tris-HCl buffer solution, pH 8.0, 0.3 M NaCl, 20 mM EDTA, 2% sodium dodecyl sulfate) was added to the resulting mixture. The obtained mixture was vigorously stirred. To the stirred mixture, 10 ml of a solution in which the ratio of phenol to chloroform to isoamyl alcohol is 50 : 49 : 1 was added. After five minutes passed, this mixture was subjected to centrifugal separation (5000 rpm and 15 min) and the upper phase was then recovered. A twofold volume of 99% ethanol was added to the upper phase and this mixture was stirred and then cooled at -80°C for two hours. This mixture was subjected to centrifugal separation (15000 rpm and 15 min), whereby DNAs were precipitated. Cool 70% ethanol was added to this mixture, which was further subjected to centrifugal separation (15000 rpm and 15 min), whereby a precipitate fraction containing the DNAs was obtained.

### [Example 2] Design of PCR primers

The inventors have discovered that there are two conserved regions, that is, MFIAMDPP (located close to the N-terminus) and HRCMGNRL (located close to the C-terminus) in an alignment of three amino acid sequences, that is, CYP153A1 (accession no. AJ311718), CYP153A2 (accession no. AE005680), and CYP153A13a (SEQ ID NO: 58), belonging to the CYP153A subfamily of the disclosed P450 superfamily. The inventors designed four types of primers on the basis of amino acid sequences (MFIAMDPP and HRCMGNRL) in the two conserved regions. The designed primers were 5'-ATGTTYATHGCNATGGAYCCNC-3' (SEQ ID NO: 53) located close to the conserved amino acid sequence MFIAMDPP (located close to the N-terminus) (SEQ ID NO: 51), 5'-CNGGRTCCATNGCDATRAACAT-3' (SEQ ID NO: 54) that is a complementary chain thereof, 5'-NARNCKRTTNCCCATRCANCKRTG-3' (SEQ ID NO: 55) located close to the conserved amino acid sequence HRCMGNRL (located close to the C-terminus) (SEQ ID NO: 52), and 5'-CAYMGNTGYATGGGNAAYMGNYT-3' (SEQ ID NO: 56) that is a complementary chain thereof. In these primer sequences, Y is C or T; H is A, C, or T; N is A, T, G, or C; R is A or G; D is A, G, or T; K is G or T; and M is A or C.

### [Example 3] Amplification of P450 gene

PCR amplification reactions were performed using the primer designed in Example 2 in such a manner that the DNAs prepared in Example 1 were used as templates. The primer of SEQ ID NO: 53 was used in combination with and the primer of SEQ ID NO: 55. Each reaction solution was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of one of the primers, 4 µl of a 50 pmol/µl solution of the other one, and 100 ng of one of the DNAs obtained from the environmental samples were mixed together and sterile water was then added to the mixture such that 50 µl of the reaction solution was obtained. In each reaction, denaturation was performed at 94°C for 10 minutes, a cycle of treatment was repeated 35 times, and additional treatment was then finally performed at 72°C for ten minutes, the treatment cycle being performed at 94°C for 30 seconds, 58°C for 45 seconds, and then 72°C for one minute. The amplification was confirmed by electrophoresis using a 1.5% agarose gel. The amplified DNAs with a predicted length (about 0.85 Kbp) were recovered from the agarose and then purified with a QIAquick Gel Extraction kit (Qiagen).

### [Example 4] Isolation of P450 gene from Alcanivorax borkumensis SK2

The inventors isolated DNA fragments containing CYP153A13a (SEQ ID NO: 58) from a strain of Alcanivorax borkumensis SK2 (DSM 11573) that was a marine bacterium utilizing an alkane by the procedure below.

Two primer sequences, SEQ ID NO: 59 (5'-GCCGATGGAGTTTATGGGCAAT-3') AND SEQ ID NO: 60 (5'-GTACCACATCACCACCTTGTCGCC-3'), were designed on the basis of the sequence CYP153A1 (accession no. AJ311718) contained in a P450 monooxygenase present in a strain of Acinetobacter calcoaceticus EB104 that is a bacterium utilizing a long-chain n-alkane. A PCR amplification reaction was then performed using a genomic DNA extracted from the Alcanivorax borkumensis SK2 strain as a template. In the reaction, 16 types of buffer solutions included in a PCR Optimizer kit (Invitrogen) were used and a cycle of treatment was repeated 40 times, the treatment cycle being performed at 94°C for one minute, 55°C for two minutes, and then 72°C for three minutes. The amplification was confirmed by electrophoresis using a 2% agarose gel. This resulted in the confirmation of amplified P450 gene fragments (about 250 bp). A P450 monooxygenase gene was cloned by hybridization using the amplified P450 gene fragments as probes. The Alcanivorax borkumensis-derived genomic DNA was partially cleaved into fragments with the restriction enzyme Sau3AI and the fragments were linked to the BamHi sites of the cosmid vector SuperCos 1 and then packaged into phages. Strains of Escherichia coli XL1-Blue MR were infected with the phages and colonies resistant to antibody Ampicillin were picked up, whereby a cosmid library was prepared. The colonies were hybridized using AlkPhos Direct Labelling and Detection System (Amersham Biosciences Corp.) according to a protocol attached thereto. Labeled positive clones were recovered and then subjected to Southern hybridization using AlkPhos Direct Labelling and Detection System (Amersham Biosciences Corp.) according to a protocol attached thereto, resulting in the detection of bands hybridized with the P450 monooxygenase gene fragments. The analysis of the DNA sequence of one of the obtained clones showed the presence of a full length of a cloned P450 monooxygenase gene (CYP153A13a containing the DNA sequence set forth in SEQ ID NO: 58 and the amino acid sequence set forth in SEQ ID NO: 57) belonging to the CYP153 family.

### [Example 5] Preparation of hybrid gene

The following fragments were amplified from a CYP153A13a gene (SEQ ID NO: 58) cloned from a strain of Alcanivorax borkumensis SK2 by a PCR: a DNA fragment (hereinafter referred to as "5-arm") ranging from an initiation codon to a portion encoding a conserved region (the amino acid sequence MFIAMDPP) and a DNA fragment (hereinafter referred to as "3-arm") ranging from a termination codon to a portion encoding a second conserved region (the amino acid sequence HRCMGNRL). The 5-arm was PCR-amplified as described below. A reaction solution used was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of the primer of SEQ ID NO: 61 (5'-CACCATGTCAACAGTTCAAGTA-3'), 4 µl of a 50 pmol/µl solution of the primer of SEQ ID NO: 54, and 100 ng of a cosmid vector containing a cloned CYP153A13a gene were mixed together and sterile water was then added to the mixture such that 50 µl of the reaction solution was obtained. In the reaction, denaturation was performed at 94°C for 10 minutes, a cycle of treatment was repeated 30 times, and additional treatment was then finally performed at 72°C for ten minutes, the treatment cycle being performed at 94°C for 30 seconds, 58°C for 45 seconds, and then 72°C for one minute. The amplified 5-arm fragments were confirmed by electrophoresis using a 1.5% agarose gel. For the amplification of the 3-arm fragment, the primer of SEQ ID NO: 62 (5'-TGATTATTTTTTAGCCGTCAACT-3') and the primer of SEQ ID NO: 56 were used instead of the primer of SEQ ID NO: 61 and the primer of SEQ ID NO: 54. The amplification was confirmed by electrophoresis using a 1.5% agarose gel. DNAs with a predicted length were recovered from the agarose and then purified with a QIAquick Gel Extraction kit (Qiagen). Each hybrid gene was prepared, by PCR, from the 5- and 3-arm fragments and a PCR product based on the DNA prepared from one of the environmental samples in Example 3. A reaction solution used was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of the primer of SEQ ID NO: 61, 4 µl of a 50 pmol/µl solution of the primer of SEQ ID NO: 62, 20 ng of the PCR product based on the DNA prepared from the environmental sample in Example 3, 5 mg of the 5-arm fragment, and 5 mg of the 3-arm fragment were mixed together and sterile water was then added to the mixture such that 50 µl of the reaction solution was obtained. In the reaction, denaturation was performed at 94°C for 10 minutes, a cycle of treatment was repeated 30 times, and additional treatment was then finally performed at 72°C for ten minutes, the treatment cycle being performed at 94°C for 30 seconds, 58°C for 45 seconds, and then 72°C for one minute. The amplification was confirmed by electrophoresis using a 1.5% agarose gel. DNAs with a predicted length (about 1.4 Kbp) were recovered from the agarose and then purified with a QIAquick Gel Extraction kit (Qiagen). Purified hybrid genes were cloned into sequence vectors (pENTR/SD/D-TOPO) (Invitrogen) using GATEWAY System pENTR Directional TOPO Cloning Kits (Invitrogen).

### [Example 6] Analysis of DNA sequence

The plasmid vectors containing the hybrid genes prepared in Example 5 were transformed into strains of Escherichia coli (One Shot TOP10 Chemically Component E. coli available from Invitrogen). Colonies of the Escherichia coli strains were randomly selected and then inoculated into LB liquid culture media (1% tryptone, 0.5% yeast extract, and 0.5% NaCl). Plasmids were extracted therefrom with a QIAprep Spin Miniprep kit (Qiagen). Sequence reactions were performed by the dideoxy chain termination method using a Big Dye Terminator Cycle Sequencing FS Ready Reaction kit (Applied Science). The nucleotide sequences of reaction products were determined with ABI prism 3730 DNA Analyzer (Applied Science). Gene sequences having the common sequence FXXGXR/HXCXG, characteristic of P450, 50 to 60 residues apart from the C-terminuses of predicted amino acid sequences were selected from obtained gene sequences. The hybrid genes had the amino acid sequences set forth in SEQ ID NOS: 1 to 25 and the nucleotide sequences set forth in SEQ ID NOS: 26 to 50 in addition to the common sequence.

### [Example 7] Analysis of sequences

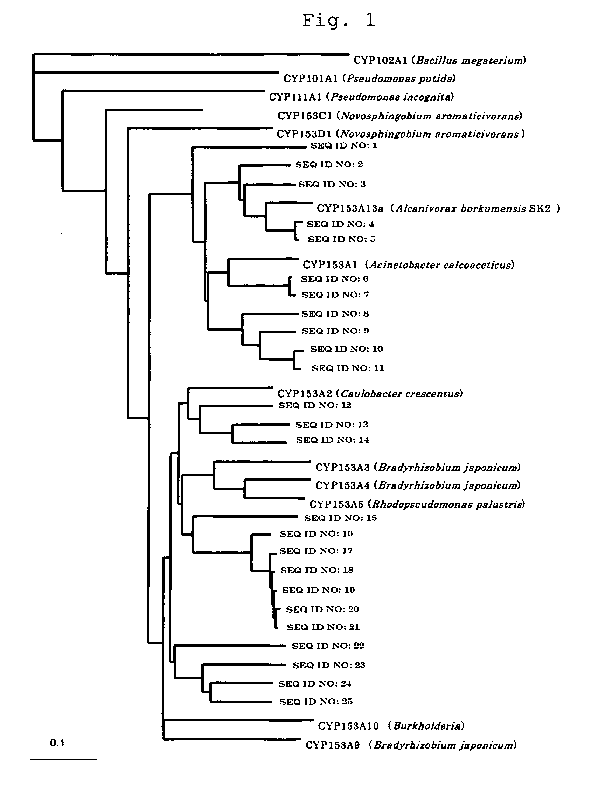

The amino acid sequences (SEQ ID NOS: 1 to 25) of the hybrid P450 genes obtained in Example 6 were compared with amino acid sequences between conserved regions (SEQ ID NOS: 51 and 52) in known P450 amino acid sequences. The comparison between these amino acid sequences were performed using an alignment software, Clustal W (Higgins, D. G., Thompson, J. D., and Gibson, T. J., Methods Enzymol. 266, 383-402, 1996). The known P450 sequence was obtained by NCBI Blast searching (http://ncbi.nlm.nih.gov/). Fig. 1 shows the compared sequences in a genealogical tree. Table 1 summarizes the homology between the sequences and four types of known CYP153A subfamilies. As is clear from Fig. 1 and Table 1, the amino acid sequences between the conserved regions in the amino acid sequences of SEQ ID NOS: 1 to 25 are different from the sequences between the conserved regions in the known P450 amino acid sequences and thus belong to new classes. Furthermore, as is clear from Table 1, novel P450 genes can be obtained by PCR using DNAs, originating from the microorganisms extracted from the various environmental samples obtained from Ponape seawater, Palau seawater, Kuji underwater, Hokkaido spring water, or oil-contaminated soil in Niigata, as templates.

**[Table 1]**

| Homology (%) | | | | | |
|---|---|---|---|---|---|
| SEQ ID NOS | CYP153A13a | CYP153A1 | CYP153A2 | CYP153A3 | SourcesofTemplate DNAs |
| 1 | 65 | 67 | 55 | 49 | Niigata Soil |
| 2 | 79 | 71 | 57 | 51 | Niigata Soil |
| 3 | 79 | 70 | 57 | 51 | Niigata Soil |
| 4 | 87 | 73 | 56 | 49 | Ponape Seawater |
| 5 | 88 | 74 | 57 | 50 | Ponape Seawater |
| 6 | 72 | 79 | 59 | 52 | Ponape Seawater |
| 7 | 72 | 79 | 57 | 51 | Ponape Seawater |
| 8 | 72 | 70 | 57 | 49 | Palau Seawater |
| 9 | 73 | 75 | 58 | 51 | Niigata Soil |
| 10 | 71 | 73 | 56 | 51 | Niigata Soil |
| 11 | 71 | 73 | 56 | 50 | Niigata Soil |
| 12 | 53 | 56 | 75 | 66 | Niigata Soil |
| 13 | 55 | 54 | 69 | 66 | Kuji Underwater |
| 14 | 52 | 55 | 71 | 64 | Niigata Soil |
| 15 | 52 | 52 | 66 | 64 | Palau Seawater |
| 16 | 54 | 57 | 72 | 65 | Kuji Underwater |
| 17 | 56 | 57 | 69 | 63 | Kuji Underwater |
| 18 | 55 | 57 | 70 | 64 | Kuji Underwater |
| 19 | 55 | 57 | 69 | 63 | Palau Seawater |
| 20 | 54 | 57 | 70 | 63 | Kuji Underwater |
| 21 | 55 | 57 | 70 | 63 | Kuji Underwater |
| 22 | 57 | 58 | 67 | 57 | Hokkaido Spring Water |
| 23 | 52 | 55 | 68 | 59 | Niigata Soil |
| 24 | 53 | 56 | 70 | 62 | Niigata Soil |
| 25 | 54 | 57 | 71 | 63 | Kuji Underwater |

### [Example 8] Expression of hybrid genes

Three of the hybrid genes containing the novel sequences shown in Example 7 were expressed in strains of Escherichia coli and functions thereof were then investigated. Three types of genes (SEQ ID NOS: 4, 5, and 9) encoding P450 were inserted into protein-expressing plasmid vectors (pDEST14 produced by Invitrogen) by the LR Clonase reaction using a Gateway system. The resulting vectors were transformed into strains of Escherichia coli DH5α (Invitrogen). The strains were inoculated into LB liquid culture media. Plasmids were extracted from the strains using QIAprep Spin Miniprep Kits (Qiagen). The plasmids obtained were transformed into strains of Escherichia coli BL21-Ai (Invitrogen). These strains were inoculated into 5 ml of LB liquid culture media supplemented with a solution of Ampicillin (Wako Pure Chemical Industries) with a final concentration of 50 µg/ml and then cultured overnight. Subsequently, 1 ml of culture solutions obtained from these media were added to 100 ml of 100 LB liquid culture media supplemented with an Ampicillin solution with a final concentration of 50 µg/ml and these strains were further cultured at 37°C. At the beginning of logarithmic growth, a 1 mM solution of 5-aminolevulin acid hydrochloride (Wako Pure Chemical Industries) and a 0.5 mM FeCl₃ solution were added to these media. After these strains were cultured at 25°C for 30 minutes, a 0.2% solution of arabinose (Wako Pure Chemical Industries) was added to these media and these strains were further cultured. After the culture (at 25°C for 16 hours) was finished, these strains were collected with a centrifugal separator, suspended in PBS (phosphate-buffered saline; 137 mM NaCl, 10 mM Na₂HPO₄, and 2.68 mM KCl) containing 20% glycerol (Wako Pure Chemical Industries), disrupted with an ultrasonic disruptor, and then subjected to centrifugal separation, whereby supernatant liquids were obtained. One of the supernatant liquids that was obtained from the Escherichia coli strains containing the plasmid vector containing the gene encoding SEQ ID NO: 4 was named Crude Extract Solution A, another one obtained from the Escherichia coli strains containing the plasmid vector containing the gene encoding SEQ ID NO: 5 was named Crude Extract Solution B, and the other one obtained from the Escherichia coli strains containing the plasmid vector containing the gene encoding SEQ ID NO: 9 was named Crude Extract Solution C.

### [Example 9] Analysis of functions by CO difference spectrometry

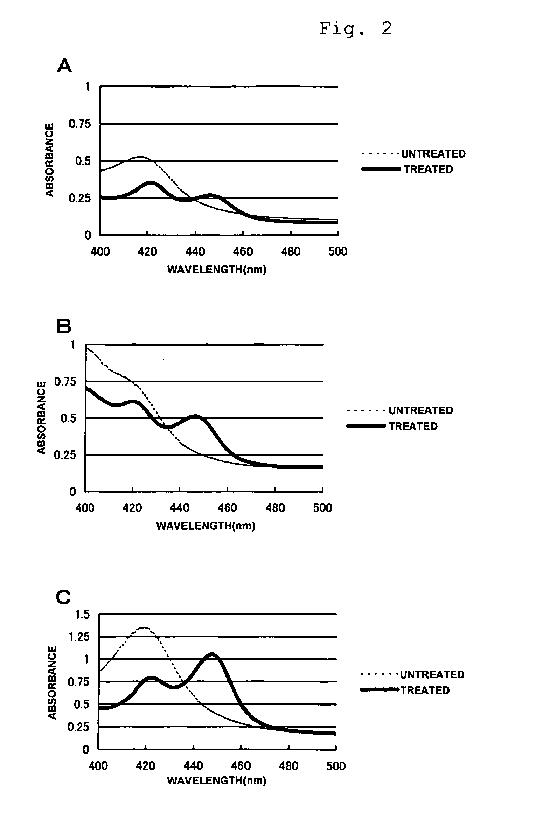

It is known that P450 has a CO difference spectrum with a maximum at 450 nm after P450 is treated with sodium dithinaite (Na₂S₂O₄) and then carbon monoxide. Thus, carbon monoxide was applied to 1 ml of Crude Extract Solutions A to C prepared in Example 8 for one minute such that several bubbles per second are created, 2 mg of sodium dithinaite was mixed with each of Crude Extract Solutions A to C, the mixtures were allowed to stand for five minutes, and the absorption spectra of the mixtures were then measured, whereby untreated or treated Crude Extract Solutions A to C were subjected to spectrum analysis. As shown in Fig. 2, the 450 nm absorbance of Crude Extract Solutions A to C treated with carbon monoxide under reductive conditions is greater than that of untreated Crude Extract Solutions A to C. This confirms the presence of soluble, functional P450 in Crude Extract Solutions A to C. This shows that the three amino acid sequences (SEQ ID NOS: 4, 5, and 9) are proteins serving as P450.

### [Example 10] Isolation of putidaredoxin and putidaredoxin reductase originating from Pseudomonas putida

In order to catalyze oxidation, the novel P450, as well as ordinary microbial P450, prepared as described above probably requires a ferredoxin reductase and ferredoxin that are electron transfer proteins. The inventors have developed a technique for using a putidaredoxin and putidaredoxin reductase originating from Pseudomonas putida as electron transfer proteins and cloned genes encoding such electron transfer proteins.

For the putidaredoxin, primers (the sequence 5'-CGTCTCCCATGTCTAAAGTAGTGTATGTGT-3' set forth in SEQ ID NO: 63 and the sequence 5'-CGTCTCATCGATTACCATTGCCTATCGGGA-3' set forth in SEQ ID NO: 64) containing restriction enzyme sites were designed from the gene (accession no. D00528) encoding the putidaredoxin and then cloned by PCR using a genomic DNA originating from Pseudomonas putida as a template. A reaction solution used was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of one of the primers, 4 µl of a 50 pmol/µl solution of the other one, and 100 ng of the prepared DNA were mixed together and sterile water was then added to the mixture such that 50 µl of the reaction solution was obtained. In the reaction, denaturation was performed at 94°C for 10 minutes, a cycle of treatment was repeated 30 times, and additional treatment was then finally performed at 72°C for ten minutes, the treatment cycle being performed at 94°C for 30 seconds, 58°C for 45 seconds, and then 72°C for one minute. The amplification was confirmed by electrophoresis using a 1.5% agarose gel. The amplified DNAs with a predicted length were recovered from the agarose and then purified with a QIAquick Gel Extraction kit (Qiagen).

For the putidaredoxin reductase, primers (the sequence 5'-CGTCTCCCATGAACGCAAACGACAACGTGG-3' set forth in SEQ ID NO: 65 and the sequence 5'-CGTCTCATCGATCAGGCACTACTCAGTTCA-3' set forth in SEQ ID NO: 66) containing restriction enzyme sites were designed from the gene (accession no. D00528) encoding the putidaredoxin reductase and then cloned by PCR using a genomic DNA originating from Pseudomonas putida as a template. A reaction solution used was prepared as follows: 0.5 unit of DNA polymerase Amplitaq Gold (Applied Biosystems), 5 µl of an attached polymerase buffer solution, 5 µl of a 2 mM dNTP solution, 4 µl of a 50 pmol/µl solution of one of the primers, 4 µl of a 50 pmol/µl solution of the other one, and 100 ng of the prepared DNA were mixed together and sterile water was then added to the mixture such that 50 µl of the reaction solution was obtained. In the reaction, denaturation was performed at 94°C for 10 minutes, a cycle of treatment was repeated 30 times, and additional treatment was then finally performed at 72°C for ten minutes, the treatment cycle being performed at 94°C for 30 seconds, 58°C for 45 seconds, and then 72°C for one minute. The amplification was confirmed by electrophoresis using a 1.5% agarose gel. The amplified DNAs with a predicted length were recovered from the agarose and then purified with a QIAquick Gel Extraction kit (Qiagen).

The full-length genes, isolated by PCR, encoding the putidaredoxin or the putidaredoxin reductase were treated with the restriction enzyme BsmBI. The expression vectors pET-28a (Novagen) were treated with the restriction enzymes Sal I and Nco I. The genes and vectors were ligated (Ligation High produced by Toyobo) and then transformed into strains of Escherichia coli JM109 (Takara Shuzo). The strains were inoculated into an LB liquid culture medium and plasmids were extracted therefrom with a QIAprep Spin Miniprep kit (Qiagen).

### [Example 11] Activity to oxygenate substrate

The three amino acid sequences (SEQ ID NOS: 4, 5, and 9), which were the novel P450 expressing the soluble, functional proteins as described in Example 9, were investigated if the amino acid sequences have the activity to oxygenate a substrate.

The expression vectors containing the putidaredoxin, originating from Pseudomonas putida, isolated in Example 10 were transformed into strains of Escherichia coli Rosseta DE3 (Novagen) and the expression vectors containing the putidaredoxin reductase originating from Pseudomonas putida were transformed into strains of Escherichia coli BL21 DE3 pLysS (Novagen). These strains were inoculated into 5 ml of LB liquid culture media and then cultured overnight. The following liquid was added to 100 ml of a TB liquid culture medium (1.2% tryptone, 2.4% yeast extract, 0.4% glycerol, 0.231% KH₂PO₄, and 1.254% K₂HPO₄) supplemented with a kanamycin solution with a final concentration of 25 µg/ml: 1 ml of a culture liquid obtained from the culture medium inoculated with the strains transformed from the expression vectors containing the putidaredoxin. The following liquid was added to 100 ml of a TB liquid culture medium supplemented with a chloramphenicol solution with a final concentration of 50 µg/ml and a kanamycin solution with a final concentration of 25 µg/ml: 1 ml of a culture liquid obtained from the culture medium inoculated with the strains transformed from the expression vectors containing the putidaredoxin reductase. The strains in the culture liquids were cultured at 37°C. At the beginning of logarithmic growth, an isopropyl-β-D-thiogalactopyranoside (IPTG) solution was added to these media such that the these media had a final IPTG concentration of 0.5 mM. The strains were further cultured. After the culture (at 30°C for 16 hours) was finished, these strains were collected with a centrifugal separator, suspended in PBS containing 20% glycerol, disrupted with an ultrasonic disruptor, and then subjected to centrifugal separation, whereby supernatant liquids, that is, crude extract solutions, were obtained. These crude extract solutions were mixed with crude extract solutions each containing one of the three types of novel P450 (SEQ ID NOS: 4, 5, and 9) in nearly equal proportions, whereby crude enzyme solutions were obtained. The crude enzyme solution consisting of the crude extract solution containing the novel P450 of SEQ ID NO: 4 and the crude extract solutions containing the two electron transfer proteins was named Crude Enzyme Solution A, the crude enzyme solution consisting of the crude extract solution containing the novel P450 of SEQ ID NO: 5 and the crude extract solutions containing the two electron transfer proteins was named Crude Enzyme Solution B, the crude enzyme solution consisting of the crude extract solution containing the novel P450 of SEQ ID NO: 9 and the crude extract solutions containing the two electron transfer proteins was named Crude Enzyme Solution C, and the crude enzyme solution consisting of the crude extract solutions containing the two electron transfer proteins was named Crude Enzyme Solution D. Crude Enzyme Solutions A to D were used for an enzyme reaction. Before the enzyme reaction was started, a substrate was added to each crude enzyme solution such that the crude enzyme solution had a final substrate concentration of 1 mM, the mixture was allowed to stand for 10 minutes at 25°C, NADP was then added to the mixture such that the mixture had a final NADP concentration of 5 mM. The substrate was octane, decane, or 1-decene. The enzyme reaction was performed at 25°C for 12 hours and then terminated in such a manner that hydrochloric acid was added to the reaction mixture such that the reaction mixture had a final hydrochloric acid concentration of 0.2 N. Ethyl acetate was added to the resulting crude enzyme solution and a fraction extracted from the crude enzyme solution with ethyl acetate was analyzed with a gas chromatography mass spectrometer (GCMS-QP5050A manufactured by Shimadzu Corporation), whereby the presence of an oxidized compound derived from the substrate was investigated.

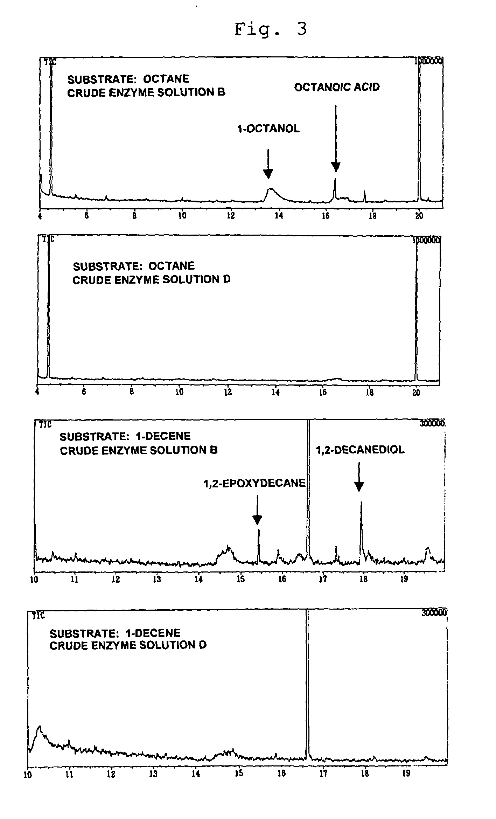

Fig. 3 shows the analysis of fractions extracted from reaction mixtures obtained by subjecting to octane or 1-decene to the enzyme reaction using Crude Enzyme Solution B or D. The fraction extracted from the reaction mixture obtained by the reaction of octane using Crude Enzyme Solution B contains 1-octanol and octanoic acid, which are oxidized compounds derived from octane. In contrast, the fraction extracted from that using Crude Enzyme Solution D, which contains no P450, contains no oxidized compound derived from octane. The fraction extracted from the reaction mixture obtained by the reaction of 1-decene using Crude Enzyme Solution B contains 1,2-epoxydecane and 1,2-decanediol, which are oxidized compounds derived from octane. In contrast, the fraction extracted from that obtained using Crude Enzyme Solution D, which contains no P450, contains no oxidized compound derived from 1-decene. These show that the novel P450 (SEQ ID NO: 5), obtained as described above, contained in Crude Enzyme Solution B has the activity to catalyze the oxidation of organic compounds such as octane and 1-decene.

Octane, decane, and 1-decene were subjected to the enzyme reaction using Crude Enzyme Solution A, B, C, or D and the reaction mixtures were analyzed. Table 2 shows detected oxidized compounds derived from the substrates.

**[Table 2]**

| | | | | |
|---|---|---|---|---|
| Substrate | Crude Enzyme | Crude Enzyme | Crude Enzyme | Crude Enzyme |
| | Solution A | Solution B | Solution C | Solution D |
| Octane | 1-octanol and | 1-octanol and | Octanoic acid | Not detected |
| | octanoic acid | octanoic acid | | |
| Decane | Not detected | Decanoic acid | Not detected | Not detected |
| 1-decene | Not detected | 1,2-epoxydecane | Not detected | Not detected |
| | | and 1,2-decanediol | | |

The oxidized compounds derived from octane were detected in the extract fractions obtained from Crude Enzyme Solutions A, B, and C. This shows that all the three types of novel P450 (SEQ ID NOS: 4, 5, and 9) contained in the crude enzyme solutions have oxygenation activity. The table shows that the novel P450 has the ability to catalyze the oxidation and/or epoxidation of alkanes, such as octane and decane, having different chain lengths.

### [Example 12] Preparation of plasmid for producing fusion protein having RhF reductase domain

In order to readily investigate the activity of the obtained hybrid P450 genes to oxidize various substrates by bioconversion using Escherichia coli hosts, an expression plasmid for producing a fusion protein containing P450 and an electron transfer protein domain (reductase domain) was prepared.

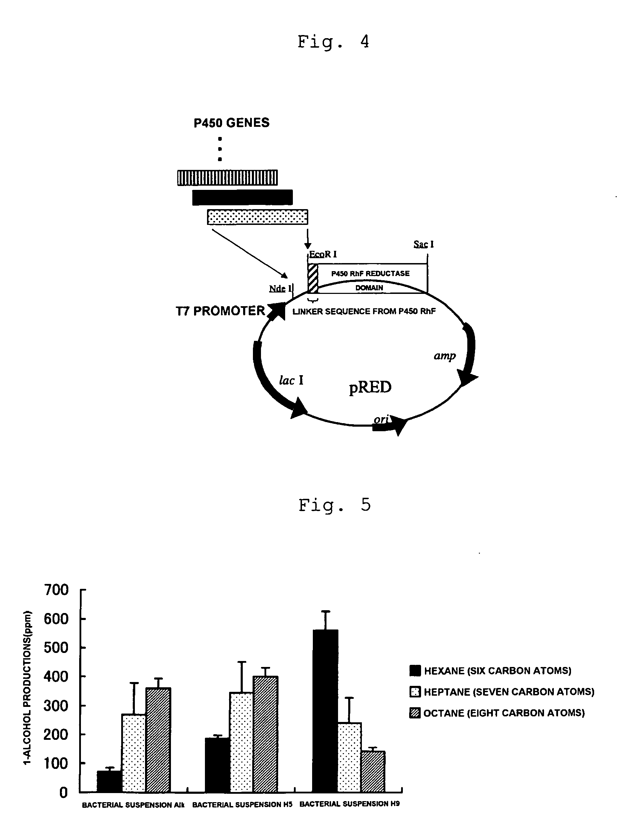

The following gene has been isolated from Rhodococcus sp. strain NCIMB 9784: a fused P450 monooxygenase (P450RhF) gene containing a single polypeptide chain containing a P450 protein domain (heme domain) and reductase domain fused with each other (Non-patent Document 8). A 1.0 kb DNA fragment containing the following gene was PCR-amplified using a primer (5'-GGGAATTCGTGCTGCACCGCCATCAACCG-3', SEQ ID NO: 71) containing a restriction enzyme domain originating from a template DNA and a primer (5'-GGGAGCTCTCAGAGGCGCAGGGCCAGGCG-3', SEQ ID NO: 72): a gene encoding a linker sequence (the nucleotide sequence set forth in SEQ ID NO: 67 and the amino acid sequence set forth in SEQ ID NO: 68) and reductase domain (the nucleotide sequence set forth in SEQ ID NO: 69 and the amino acid sequence set forth in SEQ ID NO: 70) contained in the P450RhF gene originating from Rhodococcus sp. strain NCIMB 9784. The amplified 1.0 kb DNA fragments were excised with EcoRI and SacI and then inserted into the EcoRI and SacI sites in the Escherichia coli vectors pET21a (Novagen). The plasmids are hereinafter referred to as pRED. The map of pRED is shown in Fig. 4.

### [Example 13] Preparation of artificial fusion enzymes containing hybrid P450 genes and RhF reductase domains

In order to investigate the activity of the obtained hybrid P450 genes to oxidize various substrates by bioconversion using Escherichia coli hosts, artificial fusion enzymes containing the hybrid P450 genes were prepared by inserting the hybrid P450 genes into plasmid vectors pRED for producing fusion proteins. The fusion proteins have structures in which the hybrid P450 genes are linked to RhF reductase domains with linker portions.

Primers (5'-CACTAGTCATATGTCAACGATGTCAAGTACA-3' SEQ ID NO: 73 and 5'-CAGCACAATTGTTTTTTAGCCGTCAACTT-3' SEQ ID NO: 74) containing restriction enzyme sites were designed. Fragments of the P450 genes were PCR-amplified in such a manner that sequence vectors containing genes encoding the five types of novel P450 (SEQ ID NOS: 3, 5, 7, 9, and 11) prepared in Example 5 were used as templates. Termination codons were removed from the amplified P450 gene fragments. The obtained 1.4 kb DNA fragments were cleaved with restriction enzymes NdeI and MfeI and then inserted into the NdeI-EcoRI sites of a plasmid pRED. A plasmid vector containing the gene encoding SEQ ID NO: 3 was named pH3RED, a plasmid vector containing the gene encoding SEQ ID NO: 5 was named pH5RED, a plasmid vector containing the gene encoding SEQ ID NO: 7 was named pH7RED, a plasmid vector containing the gene encoding SEQ ID NO: 9 was named pH9RED, and a plasmid vector containing the gene encoding SEQ ID NO: 11 was named pH11RED. A plasmid vector pAlkRED containing a CYP153A13a cloned from a strain of Alcanivorax borkumensis SK2 was prepared.

### [Example 14] Experiments of converting alkanes (linear hydrocarbons) by use of fused P450 monooxygenases

The plasmid vectors pH3RED, pH5RED, pH7RED, pH9RED, pH11RED, and pAlkRED were transformed into strains of Escherichia coli BL21-AI. The strains were inoculated into 5 ml of LB liquid culture media with a final Ampicillin concentration of 50 µg/ml and then cultured overnight. To 100 ml of LB liquid culture media with a final Ampicillin concentration of 50 µg/ml, 1 ml of culture liquids obtained from those LB liquid culture media were added. The strains were cultured at 30°C. At the beginning of logarithmic growth, a 1 mM solution of 5-aminolevulin acid hydrochloride and a 0.5 mM FeCl₃ solution were added to these media. After these strains were cultured at 20°C for 30 minutes, a 0.2% arabinose solution was added to these media and these strains were further cultured. After the culture (at 20°C for 16 hours) was finished, these strains were collected with a centrifugal separator and then suspended in 10 ml of 100 mM phosphate buffers (pH 7.5). A bacterial suspension containing the pH3RED-expressed strains was named H3, a bacterial suspension containing the pH5RED-expressed strains was named H5, a bacterial suspension containing the pH7RED-expressed strains was named H7, a bacterial suspension containing the pH9RED-expressed strains was named H9, a bacterial suspension containing the pH11RED-expressed strains was named H11, and a bacterial suspension containing the pAlkRED-expressed strains was named HAlk.

To 1 ml of the bacterial suspensions, 0.25 ml of substrates were added and the mixtures were subjected to reactions. The substrates were n-hexane, n-heptane, n-octane, and n-decane. Hydrochloric acid was added to the mixtures such that the resulting mixtures had a final hydrochloric acid concentration of 0.2 N, whereby the reactions were terminated. To the mixtures, 0.25 ml of hexane was added. Hexane extract fractions were obtained from the mixtures and then analyzed with a gas chromatography mass spectrometer, whereby oxidized compounds derived from the substrates were identified and the amounts of the oxidized compounds were determined. Table 3 shows the amounts (contents) of the oxidized compounds produced by the oxidation using the bacterial suspensions.

**[Table 3]**

| (Substrates) Oxides | Bacterial Suspensio Alk | Bacterial Suspension H3 | Bacterial Suspension H5 | Bacterial Suspension H7 | Bacterial Suspension H9 | Bacterial Suspension H11 |
|---|---|---|---|---|---|---|
| (n-Hexane) 1-Hexamol | 71±14 ppm | 148±8 ppm | 187±11 ppm | 232±26 ppm | 559±66 ppm | 48±2 ppm |
| (n-Heptane) 1-Heptanol | 269±109 ppm | 257±34 ppm | 345±106 ppm | 49±11 ppm | 239±88 ppm | 43±6 ppm |
| (n-Octane) 1-Octanol | 359±34 ppm | 301±39 ppm | 399±31 ppm | 163±14 ppm | 141±14 ppm | 129±6 ppm |
| (n-Decane) 1-Decanol | 75±53 ppm | 43±30 ppm | 28±31 ppm | 2.5±4.4 ppm | 42±14 ppm | 35±22 ppm |

The experiments of converting the linear hydrocarbons by bioconversion, as well as the experiments performed using the crude enzyme solutions prepared from the bacterial solutions (Example 11), show that the hybrid P450 monooxygenases catalyze the alcoholization of the n-alkanes such as hexane, octane, and decane.

Fig. 5 shows the comparison between the productions of 1-alcohols produced by oxidizing the n-alkanes with six to eight carbon atoms. For the bacterial suspensions Alk and H5, a decrease in the number of carbon atoms in the n-alkanes reduces the productions of the 1-alcohols. For the bacterial suspension H9, a decrease in the number of carbon atoms in the n-alkanes increases the productions of the 1-alcohols.

As described above, the productions of the oxidized compounds converted from the n-alkanes using the novel P450 monooxygenase are different from those converted from the n-alkanes using the P450 monooxygenases prepared using the known P450 gene CYP15313A for the arms of hybrid genes. That is, the novel P450 monooxygenase has specificity and specific activity to substrates different from those of other monooxygenases.

### [Example 15] Experiment of converting alkene by use of fused P450 monooxygenases

A bioconversion experiment was performed using the bacterial suspensions H3, H5, H7, H9, H11, and Alk prepared in Example 14 in such a manner that 1-octene, which is one of alkenes (unsaturated linear hydrocarbons), is used as a substrate. In particular, 0.25 ml of the substrate was added to 1 ml of each bacterial suspension and the mixture was subjected to a reaction at 20°C for 24 hours. Hydrochloric acid was added to the resulting mixture such that the mixture had a final hydrochloric acid concentration of 0.2 N, whereby the reaction was terminated. To the mixture, 0.25 ml of hexane was added. A hexane extract fraction obtained from this mixture was analyzed with a gas chromatography mass spectrometer, whereby 1,2-epoxyoctane produced by the oxidation of the substrate was identified and the amount thereof was determined. Table 4 shows the amounts (contents) of the oxidized compound produced by the oxidation using the bacterial suspensions.

**[Table 4]**

| (Substrate) Oxide | Bacterial Suspension Alk | Bacterial Suspension H3 | Bacterial Suspension H5 | Bacterial Suspension H7 | Bacterial Suspension H9 | Bacterial Suspension H11 |
|---|---|---|---|---|---|---|
| (1-Octene) 1,2-Epoxyoctane | 1122±129 ppm | 657±33 ppm | 1197±26 ppm | 115±14 ppm | 630±61 ppm | 2+1 ppm |

Table 4 shows that the hybrid P450 monooxygenases catalyze the epoxidation of 1-octene, which is one of unsaturated hydrocarbons.

### [Example 16] Experiment of converting cyclic hydrocarbon by use of fused P450 monooxygenases

A bioconversion experiment was performed using the bacterial suspensions H3, H5, H7, H9, H11, and Alk prepared in Example 14 in such a manner that cyclohexane, which is one of cyclic hydrocarbons (cycloalkanes), is used as a substrate. In particular, 0.25 ml of the substrate was added to 1 ml of each bacterial suspension and the mixture was subjected to a reaction at 20°C for 24 hours. Hydrochloric acid was added to the resulting mixture such that the mixture had a final hydrochloric acid concentration of 0.2 N, whereby the reaction was terminated. To the mixture, 0.25 ml of hexane was added. A hexane extract fraction obtained from this mixture was analyzed with a gas chromatography mass spectrometer, whereby cyclohexanol produced by the oxidation of the substrate was identified and the amount thereof was determined. Table 5 shows the amounts (contents) of the oxidized compound produced by the oxidation using the bacterial suspensions.

**[Table 5]**

| (Substrate) Oxide | Bacterial Suspension Alk | Bacterial Suspension H3 | Bacterial Suspension H5 | Bacterial Suspension H7 | Bacterial Suspension H9 | Bacterial Suspension H11 |
|---|---|---|---|---|---|---|
| (Cyclohexane) Cyclohexanol | 453±64 ppm | 117±7 ppm | 299±37 ppm | 7±2 ppm | 77±11 ppm | 290±1 ppm |

Table 5 shows that the hybrid P450 monooxygenases catalyze the alcoholization of cyclohexane, which is one of cyclic hydrocarbons.

### [Example 17] Experiments of converting aromatic hydrocarbons by use of fused P450 monooxygenases

The bioconversion of aromatic hydrocarbons (alkyl aromatics and alkenyl aromatics) were performed using the bacterial suspensions H5, H9, and Alk prepared in Example 14 and a bacterial suspension Non that was prepared in such a manner that plasmid vectors pAlkRED were transformed into strains of Escherichia coli BL21-AI and the strains were cultured without treatment for protein expression. The substrates were n-butylbenzene and 4-phenyl-1-butene. Each substrate was added to 1 ml of each bacterial suspension such that the mixture had a final substrate concentration of 1 mM. The mixture was subjected to a reaction at 20°C for eight hours. Hydrochloric acid was added to the resulting mixture such that the mixture had a final hydrochloric acid concentration of 0.2 N, whereby the reaction was terminated. To the mixture, 0.25 ml of ethyl acetate was added. An ethyl acetate extract fraction obtained from this mixture was analyzed for composition with a gas chromatography mass spectrometer. In the experiment using n-butylbenzene, the ethyl acetate extract fraction was treated with a sililating agent, TMSI-H (GL Science), such that a derivative was produced. The resulting fraction was analyzed for composition.

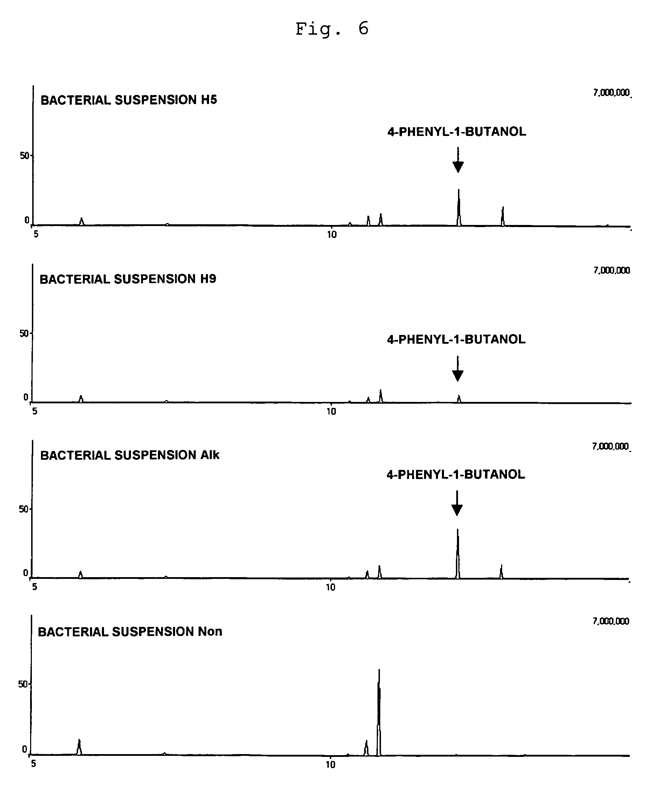

Fig. 6 shows the analysis of the fractions obtained by the reaction of n-butylbenzene. The fractions obtained from the bacterial suspensions H5, H9, and Alk contain 4-phenyl-1-butanol produced by the oxidation of n-butylbenzene. In contrast, the fraction obtained from the bacterial suspension Non containing no P450 monooxygenase contain no oxidized compound derived from n-butylbenzene. This experiment is the first to demonstrate the conversion of n-butylbenzene into 4-phenyl-1-butanol by the use of the P450 monooxygenases belonging to the CYP153 family.

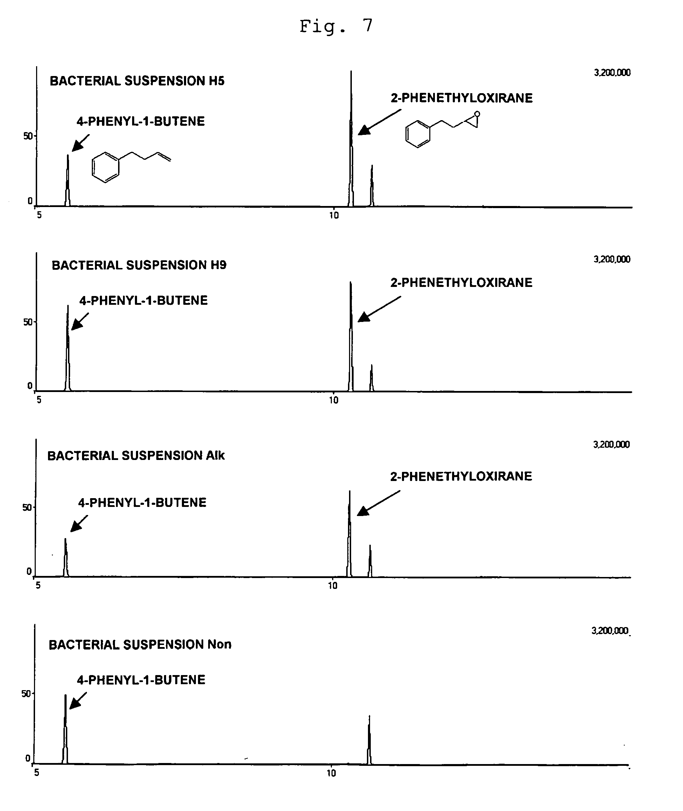

Fig. 7 shows the analysis of the fractions obtained by the reaction of 4-phenyl-1-butene. The fractions obtained from the bacterial suspensions H5, H9, and Alk contain 2-phenethyl-oxirane produced by the oxidation of 4-phenyl-1-butene. In contrast, the fraction obtained from the bacterial suspension Non containing no P450 monooxygenase contain no oxidized compound derived from 4-phenyl-1-butene. This experiment is the first to demonstrate the conversion of 4-phenyl-1-butene into 2-phenethyl-oxirane by the use of the P450 monooxygenases belonging to the CYP153 family.

### [Example 18] Preparation of artificial fusion enzyme containing P450 gene originating from Pseudomonas putida

The following fragment was PCR-amplified from a genomic DNA extracted from a strain of Pseudomonas putida ATCC 17453 using Primer 3 (5'-GACTAGTCATATGACGACTGAAACCATACA -3', SEQ ID NO: 75) and Primer 4 (5'-GGGAATTCTACCGCTTTGGTAGTCGCCGGA-3', SEQ ID NO: 76): a 1.3 kb DNA fragment containing a P450cam gene (Nucleotide sequence of the Pseudomonas putida cytochrome P450cam gene and its expression in Escherichia coli., J. Biol. Chem., 261: 1158-1163, 1986 and NCBI accession No. M12546) originating from Pseudomonas putida. The underlined sequences indicate an NdeI site (Primer 3) and an EcoRI site (Primer 4). A termination codon of the P450 gene was removed. The amplified 1.3 kb DNA fragments were cleaved at the NdeI and EcoRI sites and then subcloned into the NdeI-EcoRI sites of the plasmid pRED. The resulting plasmid is hereinafter referred to as pCAMRED. A plasmid pETP450cam for comparison was prepared by cloning the P450cam gene into the NdeI-EcoRI sites of a vector pET21a.

### [Example 19] Analysis of functions of fused P450 monooxygenase

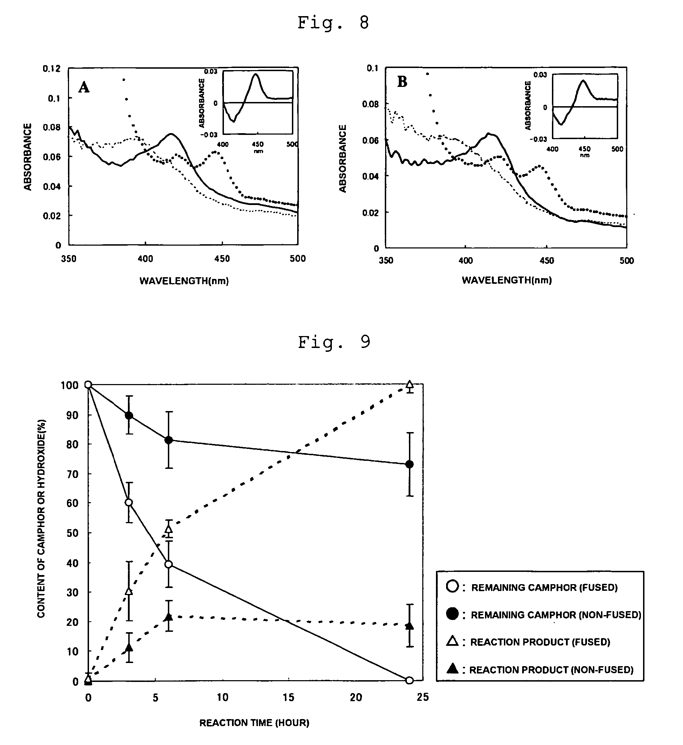

The plasmid pCAMRED prepared in Example 18 was transformed into a strain of Escherichia coli BL21(DE3) and the resulting strain was cultured in 3 mL of an LB liquid culture medium (containing 100 µg/ml of Ampicillin) at 30°C for 16 hours. Subsequently, 1 mL of a culture liquid obtained from the medium was added to 50 mL of an M9-glucose culture medium (containing 100 µg/ml of Ampicillin) and the culture was continued until the OD600 value reached about 0.8. IPTG serving as an inducer was added to the culture liquid such that the culture liquid had an IPTG concentration of 0.2 mM. The culture was further continued at 25°C for 16 hours. The cultured strains were collected and then suspended in 10 mL of a 10 mM phosphate buffer (pH 7.0). The suspension was subjected to ultrasonic disruption. The resulting suspension was subjected to centrifugal separation (10000xg for 20 minutes), whereby a cell-free extract was obtained from a supernatant portion of the suspension. Into two cuvettes, 0.8 ml of the cell-free extract was pipetted. After carbon monoxide was introduced into the sample-side cuvette, 5 to 10 mg of sodium thiosulfate was placed into both cuvettes. The cell-free extract in each cuvette was agitated and then subjected to spectrum analysis using light with a wavelength of 400 to 500 nm, whereby the cell-free extract was investigated if the cell-free extract contained an active P450 monooxygenase. Camphor serving as a substrate was added to 0.8 ml of the cell-free extract such that the mixture had a camphor concentration of 0.5 mM. The mixture was subjected to spectrum analysis. As shown in Fig. 8A, an artificial fused P450 monooxygenase has a spectrum pattern similar to that of a non-fused P450cam monooxygenase (Fig. 8B). That is, peaks are shifted from 420 nm to 450 nm because the P450 and P450cam monooxygenases in a reduced state are bound to carbon monoxide (dotted lines shown in Fig. 8). The CO difference spectra measured in the presence or absence of carbon monoxide have a maximum absorbance at 450 nm (inserted graphs in Fig. 8). In the spectrum of the mixture of the cell-free extract and the substrate, a maximum peak is shifted to 390 nm (a broken line in Fig. 8A). These show that the artificial fusion of the P450 monooxygenase and a reductase domain causes no adverse effects (the binding inhibition of the substrate due to steric hindrance or the like) and the fused P450cam monooxygenase has enzymatic activity.

### [Example 20] Conversion of camphor by use of Escherichia coli producing fused P450cam monooxygenase

A strain of Escherichia coli BL21 (DE3) containing the plasmid pCAMRED was cultured in the same manner as that described in Example 19. The cultured strains were subjected to gene expression. The resulting strains were collected and then suspended in 5 mL of a 10 mM phosphate buffer (pH 7.0). Camphor serving as a substrate for a P450cam monooxygenase was added to the suspension such that the mixture had a final camphor concentration of 0.5 mM. The strains were incubated at 25°C for 24 hours. After three, six, or 24 hours elapsed since the start of the reaction, each sample was obtained from the reaction solution. A liquid extract containing the substrate and a reaction product (5'-exo-hydroxide) was obtained from the reaction solution using ethyl acetate and then analyzed with a gas chromatography mass spectrometer (GC-MS, Shimadzu QP-5050), whereby identification was performed. As shown in Fig. 9, in the strains containing an artificial fusion enzyme gene, the sample obtained after 24 hours elapsed since the start of the reaction contains no camphor but camphor 5'-exo-hydroxide. In contrast, in strains of Escherichia coli BL21 (DE3) containing a non-fused plasmid pETP450cam (containing no RhF reductase domain) for comparison, about 20% of camphor is consumed during hydroxylation. The vertical axes of the graph in Fig. 9 show the consumption of the substrate or the accumulation of the reaction product. The content of the substrate at the start of the reaction is defined as 100% and the theoretical content of the reaction product at the complete consumption of the substrate is defined as 100%.

### [Example 21] Fusion of P450alk gene originating from Alcanivorax borkumensis

A 1.3 kb DNA fragment containing a P450alk (CYP153A13a) gene originating from Alcanivorax borkumensis was PCR-amplified from a genomic DNA extracted from a strain of Alcanivorax borkumensis SK2 (DSM11573) using Primer 5 (5'-CACTAGTCATATGTCAACGAGTTCAAGTACA-3', SEQ ID NO: 73) and Primer 6 (5'-CAGCACCAATTGTTTTTTAGCCGTCAACTT-3', SEQ ID NO: 74). The underlined sequences indicate an NdeI site (Primer 5) and an MfeI site (Primer 6). A termination codon of the P450 gene was removed. The amplified 1.3 kb DNA fragments were cleaved at the NdeI and MfeI sites and then subcloned into the NdeI-EcoRI sites of the plasmid pRED prepared in Example. The resulting plasmid is hereinafter referred to as pAlkRED. The nucleotide sequence of the P450alk (CYP153A13a) gene originating from Alcanivorax borkumensis SK2 (DSM11573) is set forth in SEQ ID NO: 58.

A plasmid pETP450alk for comparison was prepared by cloning only the P450alk gene into the NdeI-EcoRI sites of a vector pET21a.

### [Example 22] Conversion of alkane by use of Escherichia coli producing fused P450alk monooxygenase

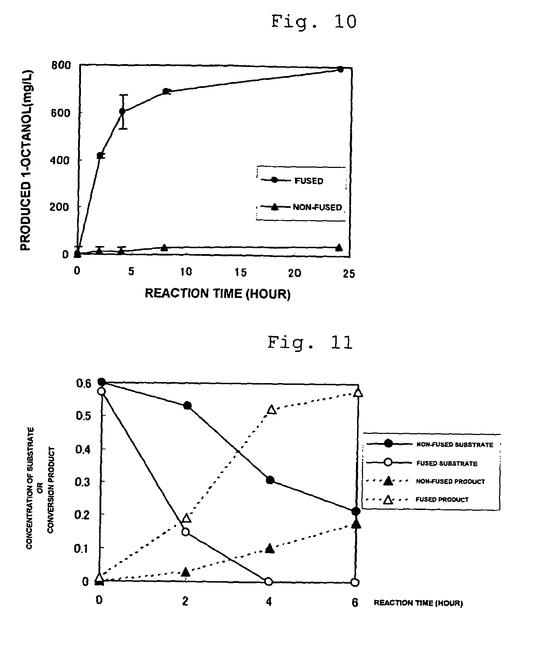

Strains of Escherichia coli BL21 (DE3) containing the plasmid pAlkRED or pETP450alk were cultured in the same manner as that described in Example 19. The cultured strains were subjected to gene expression. The resulting strains were collected and then suspended in 5 mL of 10 mM phosphate buffers (pH 7.0). To 1.5 mL of each suspension, 0.5 mL of octane serving as a substrate for a P450cam monooxygenase was added. The strains were incubated at 20°C for 24 hours. Samples were obtained from the reaction solution until 24 hours elapsed since the start of the reaction. A liquid extract containing a reaction product (1-octanol) was obtained from the reaction solution using octane and then analyzed with a gas chromatography mass spectrometer (GC-MS, Shimadzu QP-5050), whereby identification and determination were performed. Fig. 10 shows that the sample obtained from the reaction solution prepared using the strains containing the plasmid pAlkRED for expressing an artificial fusion enzyme gene contains 790.8 ppm of 1-octanol. In contrast, the sample obtained from the reaction solution prepared using the strains containing the non-fused plasmid pETP50cam (containing no RhF reductase domain) for comparison contains 40.3 ppm of 1-octanol. The content of 1-octanol in the sample obtained from the reaction solution prepared using these strains is about 5% of that in the sample obtained from the reaction solution prepared using those strains in which the artificial fusion enzyme gene has been expressed.

### [Example 23] Fusion of P450bzo gene obtained from environmental DNA sample

Uchiyama T, et al. have obtained a P450bzo gene from an environmental metagenome library by the SIGEX method (Uchiyama T, et al. Substrate-induced gene-expression screening of environmental metagenome libraries for isolation of catabolic genes, Nat. Biotechnol., 23: 88-93, 2005 and GenBank code: ABI86504). The P450bzo gene was subcloned. A 1.2 kb DNA fragment located close to the N-terminus of the P450 gene amplified using Primer 7 (5'-CGATATACATATGTTCAGTTTTGACCCCTAT-3', SEQ ID NO: 77) and Primer 8 (5'-GGATTGCGTTTGACGAATTTCACAAA-3', SEQ ID NO: 78). A 110 bb DNA fragment located close to the C-terminus of the P450 gene amplified using Primer 9 (5'-GGGCTCACTTTGTGAAATTCGTCAA3', SEQ ID NO: 79) and Primer 10 (5'-GGGAATTCCGAAACGGCCAATTCCAG-3', SEQ ID NO: 80). The 20th T in Primer 8 and the 15th A in Primer 9 are mutations introduced to remove an EcoRI site of the gene. There were no substitutes due to the mutations in amino acid sequences. The amplified 1.2 kb and 110 bp DNA fragments were mixed together. Full-length P450bzo gene fragments were prepared from the mixture by PCR using Primers 7 and 10. The underlined sequences in Primers 7 and 10 indicate an NdeI site and an EcoRI site. A termination codon of the P450 gene was removed. The amplified 1.3 kb fragments were cleaved at the NdeI and EcoRI sites and then subcloned into the NdeI-EcoRI sites of the plasmid pRED prepared in Example. The resulting plasmid is referred to as pBzoRED.

A plasmid pETP450bzo for comparison was prepared by cloning the P450bzo gene into the NdeI-EcoRI sites of an Escherichia coli vector pET21a.

### [Example 24] Conversion of 4-hydroxybenzoic acid by use of Escherichia coli producing fused P450bzo monooxygenase

Strains of Escherichia coli BL21 (DE3) containing the plasmid pBzoRED or pETP450bzo were cultured in the same manner as that described in Example 19. The cultured strains were subjected to gene expression. The resulting strains were collected and then suspended in 5 mL of 10 mM phosphate buffers (pH 7.0). To each suspension, 4-hydroxybenzoic acid serving as a substrate for a P450bzo monooxygenase was added. The strains were incubated at 20°C for six hours. After two, four, or six hours elapsed since the start of the reaction, each sample was obtained from the reaction solution. A liquid extract containing the substrate and a reaction product (3,4-dihydroxybenzoic acid) was obtained from the reaction solution using ethyl acetate and then analyzed with a gas chromatography mass spectrometer (GC-MS, Shimadzu QP-5050), whereby identification were performed. Fig. 11 shows that the sample obtained from the reaction solution prepared using the strains containing the plasmid pBzoRED for expressing an artificial fusion enzyme gene contains no substrate but about 0.6 mM of 3,4-dihydroxybenzoic acid, which is a product of hydroxylation, because of the rapid conversion of the substrate, the sample being obtained after four hours elapsed since the start of the reaction. In contrast, the sample obtained from the reaction solution prepared using the strains containing the non-fused plasmid pETP450bzo (containing no RhF reductase domain) for comparison has a substrate concentration (2.5 mM) that is half of that of that sample and also has a product concentration of about 0.1 mM, the sample being obtained after four hours elapsed since the start of the reaction.

### [Example 25] Fusion of P450SU-1 or P450SU-2 gene originating from Streptomyces griseolus

A 1.3 kb DNA fragment containing a P450SU-1 (CYP105A1) or P450SU-2 (CYP105B1) gene (Genes for two herbicideinducible cytochromes P-450 from Streptomyces griseolus, J. Bacteriol., 172: 3335-45, 1990 and NCBI accession No. M32238 or M32239) originating from Streptomyces griseolus was PCR-amplified from a genomic DNA extracted from a strain of Streptomyces griseolus ATCC 11796 using a pair of Primer 11 (5'-GGACTCCATATGACCGATACCGCCACGACG-3', SEQ ID NO: 81) and Primer 12 (5'-CTGAATTCCCAGGTGACCGGGAGTTCGTTGAC-3', SEQ ID NO: 82) or a pair of Primer 13 (5'-GGACTCCATATGACGACCGCAGAACGCACC-3', SEQ ID NO: 83) and Primer 14 (5'-CTGAATTCCCAGGCGATCGGCAGCGAGTGGAC-3', SEQ ID NO: 84). The underlined sequences indicate NdeI sites (Primers 11 and 13) and EcoRI sites (Primers 12 and 14). A termination codon of the P450 gene was removed. The amplified 1.3 kb DNA fragments were cleaved at the NdeI and EcoRI sites and then inserted into the NdeI-EcoRI sites of the vector pRED, prepared in Example 12, for expressing function, whereby plasmids for expressing a fused P450SU-1 or P450SU-1 gene were prepared. The prepared plasmids are hereinafter referred to as pSU-1RED or pSU-2RED. A plasmid pETP450SU-1 and plasmid pETP450SU-2 for comparison were prepared by inserting only the P450SU-1 or P450SU-2 gene into the NdeI-EcoRI sites of a vector pET21a.

### [Example 26] Conversion of 7-ethoxycoumarin by use of Escherichia coli producing fused P450SU-1 or P450SU-2 monooxygenase

Strains of Escherichia coli BL21 (DE3) containing the plasmid pSU-1RED, pSU-2RED, pETP450SU-1, or pETP450SU-2 were cultured in the same manner as that described in Example 19. The cultured strains were subjected to gene expression. To culture media for culturing the strains, 7-ethoxycoumarin serving as a substrate for a P450SU-1 monooxygenase and a P450SU-2 monooxygenase was added simultaneously with the gene expression such that the culture media had a final 7-ethoxycoumarin concentration of 1 mM. The strains were incubated at 20°C for 74 hours. Each liquid extract containing the substrate and a reaction product (7-ethoxycoumarin) was obtained from a reaction solution, obtained from each culture medium, using ethyl acetate and then analyzed by thin-layer chromatography. The analysis showed that the extracts obtained from the reaction solutions prepared using the strains containing the plasmid pSU-1RED or pSU-2RED for expressing an artificial fusion enzyme gene contained 7-ethoxycoumarin, which was a reaction product. In contrast, the extract obtained from the reaction solution prepared using the strains containing the non-fused plasmid pETP450SU-1 contained no reaction product. Although the extract obtained from the reaction solution prepared using the strains containing the non-fused plasmid pETP450SU-2 contained a small amount of the reaction product, the content of the reaction product in this extract was less than that in the extract obtained from the reaction solution prepared using the strains containing the plasmid pSU-2RED.

This example shows that a P450 monooxygenase originating from actinomycete can be used for a function expression system according to the present invention.

### [Example 27] Isolation and fusion of P450HB153 gene

Nocardiaceae strain Hou_blue is a novel gram-positive bacterium and has been isolated from soil (oil-contaminated soil obtained in Niigata-ken) obtained from an oil field located in Nishiyama-cho, Niigata-ken on the basis of the ability to utilize an alkane, which is a petroleum hydrocarbon. The Hou_blue strain was deposited with Department of Biotechnology (2-5-8, Kazusa Kamatari, Kisarazu-shi, Chiba-ken), National Institute of Technology and Evaluation, on June 8, 2004 and the deposit number thereof was NITE P-3.

The following gene was isolated from the Nocardiaceae Hou_blue strain: a novel P450 gene (hereinafter referred to as a P450HB153 gene (the nucleotide sequence thereof is set forth in SEQ ID NO: 86 and the amino acid sequence of a P450 protein encoded by the gene is set forth in SEQ ID NO: 85)) belonging to the CYP153 family. A 1.3 kb DNA fragment containing the P450HB153 gene was PCR-amplified from a genomic DNA extracted from the strain using Primer Hou-1 (5'-GTAGGCCATATGAACGTAATCGGTGCAGGT-3', SEQ ID NO: 87) and Primer Hou-2 (5'-CATGAATTCGACCTGTTCCATCTCGGTCTC-3', SEQ ID NO: 88). The underlined sequences indicate an NdeI site and an EcoRI site. A termination codon of the P450HB153 gene was removed. The amplified 1.3 kb DNA fragments were cleaved at the NdeI and EcoRI sites and then inserted into the NdeI-EcoRI sites of the vector pRED prepared in Example 12, whereby a plasmid pHB153-Red was prepared.

### [Example 28] Conversion of 2-n-butylbenzofuran by use of Escherichia coli producing fused P450HB153 monooxygenase

An experiment of biochemically converting 2-n-butylbenzofuran was performed using a strain of Escherichia coli BL21 (DE3) containing a plasmid pHB153-Red. That is, the plasmid pHB153-Red prepared in Example 27 was transformed into a strain of Escherichia coli BL21(DE3) and the resulting strain was cultured in 3 mL of an LB liquid culture medium (containing 100 µg/ml of Ampicillin) at 30°C for 16 hours. Subsequently, 1 mL of a culture liquid obtained from the medium was added to 50 mL of an M9-glucose culture medium (containing 100 µg/ml of Ampicillin) and the culture was continued until the OD600 value reached about 0.8. IPTG serving as an inducer was added to the culture liquid such that the culture liquid had an IPTG concentration of 0.2 mM. The culture was further continued at 25°C for 16 hours. The culture liquid was subjected to centrifugal separation (8000rpm for 5 min.), whereby the cultured strains were precipitated. After a supernatant portion was removed from the culture liquid, 10 mL of a 50 mM phosphate buffer (pH 7.0) was added to the resulting culture liquid and the mixture was then vigorously stirred. The cultured strains were recovered from the mixture into 50 ml Falcon tubes. To each Falcon tube, 1.74 mg of the substrate dissolved in a small amount of DMDS was added (a final substrate concentration of 1 mM). The resulting strains were subjected to shaking culture at 20°C for 24 hours.

From 10 of the Falcon tubes, 100 ml (corresponding to 1 L of the original culture liquid) of a suspension containing the culture liquid used to convert 2-n-butylbenzofuran was recovered. The suspension was mixed with ethyl acetate and the mixture was partitioned. The ethyl acetate phase was recovered and then concentrated. The ethyl acetate extract (18.6 mg) was subjected to TLC analysis (a developer containing hexane and ethyl acetate (5 : 1). The analysis showed the presence of a product (hereinafter referred to as Compound 28-1) with an Rf value of 0.21. The ethyl acetate extract was purified by silica gel chromatography (1 cm diameter, 20 cm length, a developer containing hexane and ethyl acetate (5 : 1), whereby pure Compound 28-1 (0.4 mg) was obtained. The analysis of Compound 28-1 by EI-MS showed a molecular ion peak M^{ϕ} at m/z 190 (substrate + one oxygen atom). The analysis of Compound 28-1 by ¹H NMR spectrometry showed that a signal corresponding to an aromatic ring portion thereof was identical to that of the substrate. The analysis also showed that there was no terminal methyl group of the substrate and Compound 28-1 had three methylene groups and an oxymethylene group with a triplet at δ 3.70. The analysis of the DQF-COSY spectrum thereof showed that these four methylene groups were linked to one another. From the analysis of the DQF-COSY spectrum and the analysis by EI-MS, it was confirmed that this compound had a hydroxyl group inserted into a terminal methyl group. That is, Compound 28-1 was identified to be 4-benzofran-2-yl-butan-1-ol.

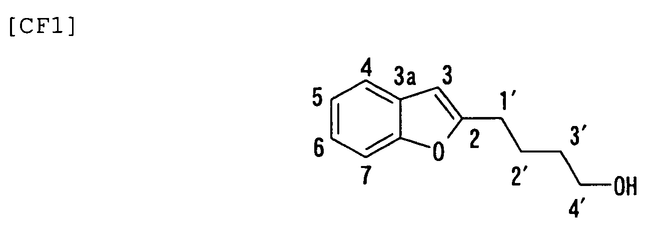

This compound is specified in CAS and is the first produced by microbial conversion.

**[Table 6]**

| 400-MHz ¹H NMR Data of Compound 28-1 (4-benzofuran-2-yl-butan-1-ol) (in CDCl₃) | | |
|---|---|---|
| Position | δ_{H} | |
| 3 | 6.40 (s) | |
| 4 | 7.47 (d7.0) | |
| 5 | 7.21 | |
| 6 | 7.21 | |
| 7 | 7.40 (d 7.1) | |
| 1' | 2.82 (t) | |
| 2' | 1.85 (m) | |
| 3' | 1.70 (m) | |
| 4' | 3.70 (t 6.3) | |

### Industrial Applicability

The present invention provides a method for efficiently isolating a novel P450 gene from a sample, such as an environmental sample, containing various microbial nucleic acids. P450 produced from the P450 gene obtained by the method has specificity and activity to various substrates and can be used for the oxidation of various low-molecular-weight organic compounds such as alkanes, alkenes, cyclic hydrocarbons (cycloalkanes), and aromatics (alkyl or alkenyl aromatics).

This specification includes the matters specified in the specifications and/or drawings of Japanese patent applications (No. 2004-326139, No. 2005-83019, and No. 2005-83122) to which this application claims priority. The contents of the publications, patents, and patent applications cited in this specification are incorporated herein by reference.

## Claims

1. A fused cytochrome P450 monooxygenase containing a peptide which is linked to the C-terminus of a P450 protein with a linker portion disposed therebetween and which has the same function as that of a reductase domain contained in a cytochrome P450 monooxygenase originating from Rhodococcus sp. strain NCIMB 9784.

2. The fused cytochrome P450 monooxygenase according to claim 1, wherein the peptide is:
(a) one containing the amino acid sequence set forth in SEQ ID NO: 70;
(b) one containing an amino acid sequence prepared by adding one or more amino acid residues to the amino acid sequence set forth in SEQ ID NO: 70; removing one or more amino acid residues from this amino acid sequence, or replacing one or more amino acid residues of this amino acid sequence with other residues; or
(c) one encoded by a DNA capable of hybridizing with a DNA containing the nucleotide sequence set forth in SEQ ID NO: 69 or a DNA complementary to this DNA under stringent conditions.

3. The fused cytochrome P450 monooxygenase according to claim 1 or 2, wherein the linker portion is:
(a) a peptide containing the amino acid sequence set forth in SEQ ID NO: 68;
(b) a peptide containing an amino acid sequence prepared by adding one or more amino acid residues to the amino acid sequence set forth in SEQ ID NO: 68; removing one or more amino acid residues from this amino acid sequence, or replacing one or more amino acid residues of this amino acid sequence with other residues; or
(c) a peptide encoded by a DNA capable of hybridizing with a DNA containing the nucleotide sequence set forth in SEQ ID NO: 67 or a DNA complementary to this DNA under stringent conditions.

4. A fused cytochrome P450 monooxygenase according to any one of claims 1 to 3, wherein the P450 protein originates from a bacterium.

5. A fused cytochrome P450 monooxygenase according to any one of claims 1 to 3, wherein the P450 protein is a protein containing the amino acid sequence set forth in SEQ ID NO: 57 or 85.

6. A fused cytochrome P450 monooxygenase according to any one of claims 1 to 3, wherein the P450 protein is a protein containing:
(a) the amino acid sequence set forth in any one of SEQ ID NOS: 1 to 25; or
(b) an amino acid sequence prepared by removing one or more amino acid residues from the amino acid sequence set forth in any one of SEQ ID NOS: 1 to 25, replacing one or more amino acid residues of this amino acid sequence with other residues, or adding one or more amino acid residues to this amino acid sequence, wherein said protein is a P450 protein.

7. A DNA encoding a fused cytochrome P450 monooxygenase according to any one of claims 1 to 6.

8. A microorganism containing a DNA according to claim 7.

9. A method for producing a fused cytochrome P450 monooxygenase, comprising:
culturing a microorganism according to claim 8; and
recovering a fused cytochrome P450 monooxygenase from the cultured microorganisms or cells thereof.

10. A method for producing an oxidized compound, comprising a step of subjecting a substrate to a reaction in the presence of a fused cytochrome P450 monooxygenase according to any one of claims 1 to 6 to produce an oxidized compound.

11. The method according to claim 10, wherein the substrate is n-hexane, n-heptane, n-octane, n-decane, 1-octene, cyclohexane, n-butylbenzene, 4-phenyl-1-butene, or 2-n-butylbenzofran and the oxidized compound is 1-hexanol, 1-heptanol, 1-octanol, 1-decanol, 1,2-epoxyoctane, cyclohexanol, 4-phenyl-1-butanol, 2-phenethyloxirane, or 4-benzofran-2-yl-butane-1-ol.


SEQUENCE LISTING

<110> MARINE BIOTECHNOLOGY INSTITUTE CO., LTD.

<120> METHOD FOR ISOLATION OF P450 GENES

<130> N.101336 GCW

<140> EP 05799974.0
<141> 2005-11-02

<150> JP 2004/326139
<151> 2004-11-10

<150> JP 2005/83019
<151> 2005-03-23

<150> JP 2005/83122
<151> 2005-03-23

<160> 88

<170> PatentIn Ver. 2.1

<210> 1
<211> 470
<212> PRT
<213> Artificial Sequence

<220>
<223> Description of Artificial Sequence: hybrid protein

<400> 1
Met Ser Thr Ser Ser Ser Thr Ser Asn Asp Ile Gln Ala Lys Ile Ile
 1               5                  10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Met His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Gln His Asp Glu Gln Arg Asn Ala Val Gln
145                 150                 155                 160

Gly Val Val Ala Pro Lys Asn Leu Lys Glu Met Glu Ser Leu Ile Arg
                165                 170                 175

Ser Arg Val Arg Glu Val Leu Asp Gly Leu Pro Val Gly Glu Pro Phe
            180                 185                 190

Asn Trp Val Asp Lys Val Ser Ile Glu Leu Thr Gly Arg Met Leu Ala
        195                 200                 205

Thr Leu Leu Asp Tyr Leu Tyr Glu Asp Arg Arg Glu Leu Thr Glu Ile
    210                 215                 220

Ser Asp Leu Ala Ser Gly Val Pro Gly Ala Thr Gly Gly Glu Ser Asp
225                 230                 235                 240

Leu Asp Glu Thr Phe Ser Leu Ala Val Pro Leu Leu Thr Lys Phe Leu
                245                 250                 255

Arg Leu Trp Asn Glu Lys Lys Ala Arg Phe Glu Ala Gly Gln Pro Leu
            260                 265                 270

Gly Phe Asp Leu Ile Ser Leu Met Leu Ala Asp Pro Ala Thr Arg Asn
        275                 280                 285

Leu Ile Asp Glu Arg Pro Met Glu Phe Leu Gly Asn Ala Val Leu Leu
    290                 295                 300

Ile Val Gly Gly Asn Asp Thr Thr Arg Asn Ser Met Ser Gly Gly Val
305                 310                 315                 320

Tyr Ala Leu Asn Lys Trp Pro Asp Gln Phe Ala Lys Leu Lys Asp Asn
                325                 330                 335

Pro Lys Leu Ile Pro Asn Met Val Ser Glu Ile Ile Arg Trp Gln Thr
            340                 345                 350

Pro Leu Ala Tyr Met Ala Arg Arg Ala Lys Glu Asp Thr Val Val Gly
        355                 360                 365

Gly Gln Glu Ile Lys Lys Gly Glu Lys Leu Ile Met Trp Tyr Ser Ser
    370                 375                 380

Ala Asn Arg Asp Glu Arg His Phe Glu Asn Pro Glu Gln Leu Ile Ile
385                 390                 395                 400

Asp Arg Lys Asn Ala Arg Asn His Ile Ala Phe Gly Phe Gly Ile His
                405                 410                 415

Arg Cys Met Gly Asn Arg Phe Ala Glu Leu Gln Leu Arg Ile Leu Trp
            420                 425                 430

Glu Glu Leu Leu Pro Arg Phe Glu Asn Ile Glu Val Ile Gly Glu Pro
        435                 440                 445

Glu Arg Val Gln Ser Asn Phe Val Arg Gly Tyr Ser Lys Met Met Val
    450                 455                 460

Lys Leu Thr Ala Lys Lys
465                 470



<210> 2
<211> 470
<212> PRT
<213> Artificial Sequence

<220>
<223> Description of Artificial Sequence: hybrid protein

<400> 2
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
             20                  25                  30

Lys Asn Leu Met Lys Val Lys Arg Lys Thr Ile Gly Thr Ser Arg Pro
         35                  40                  45

Gln Val His Phe Val Glu Thr Asp Leu Pro Asp Val Asn Asp Leu Ala
     50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Arg Ser Val Gln
145                 150                 155                 160

Gly Val Val Ala Pro Gln Asn Leu Lys Glu Met Glu Gly Leu Ile Arg
                165                 170                 175

Ser Arg Thr Gly Glu Val Leu Asp Gly Leu Pro Leu Gly Glu Ala Phe
            180                 185                 190

Asn Trp Val Pro Ala Val Ser Ile Glu Leu Thr Gly Arg Met Leu Ala
        195                 200                 205

Thr Leu Leu Asp Phe Pro Tyr Glu Glu Arg His Lys Leu Thr Tyr Trp
    210                 215                 220

Ser Asp Arg Leu Ala Asn Thr Ala Glu Thr Thr Gly Gly Glu Phe Asp
225                 230                 235                 240

Asp Asp Asp Gly Leu Phe Asp Thr Ala Ala Asp Met Ala Arg Ala Phe
                245                 250                 255

Ser Glu Leu Trp Arg Asp Lys Gln Ala Arg Arg Ala Ala Gly Glu Ala
            260                 265                 270

Pro Ser Phe Asp Leu Ile Ser Leu Met Gln Ser Ser Glu Asp Thr Ala
        275                 280                 285

Asp Met Ile Lys Arg Pro Met Glu Phe Leu Gly Asn Leu Ala Leu Leu
    290                 295                 300

Ile Ile Gly Gly Asn Asp Thr Thr Arg Asn Ser Met Ser Gly Gly Val
305                 310                 315                 320

Leu Ala Leu Asn Gln Phe Pro Lys Glu Phe Ala Lys Leu Lys Ala Asn
                325                 330                 335

Pro Glu Leu Ile Pro Asn Met Val Ser Glu Ile Ile Arg Trp Gln Thr
            340                 345                 350

Pro Leu Ala His Met Arg Arg Ile Ala Thr Gln Asp Val Glu Leu Cys
        355                 360                 365

Gly Gln Thr Ile Lys Lys Gly Asp Arg Val Leu Met Trp Tyr Ala Ser
    370                 375                 380

Gly Asn Arg Asp Glu Arg Lys Phe Glu Asn Pro Asp Asp Phe Ile Ile
385                 390                 395                 400

Asp Arg Glu Gly Ala Arg His His Met Ser Phe Gly Tyr Gly Ile His
                405                 410                 415

Arg Cys Met Gly Asn Arg Leu Ala Glu Leu Gln Leu Arg Ile Leu Trp
            420                 425                 430

Glu Glu Leu Leu Pro Arg Phe Glu Asn Ile Glu Val Ile Gly Glu Pro
        435                 440                 445

Glu Arg Val Gln Ser Asn Phe Val Arg Gly Tyr Ser Lys Met Met Val
    450                 455                 460

Lys Leu Thr Ala Lys Lys
465                 470

<210> 3
<211> 470
<212> PRT
<213> Artificial Sequence

<220>
<223> Description of Artificial Sequence: hybrid protein

<400> 3
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro

35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Arg Ser Val Gln
145                 150                 155                 160

Asn Val Val Ala Pro Gln Asn Leu Lys Glu Met Glu Gly Leu Ile Arg
                165                 170                 175

Thr Arg Thr Ala Glu Val Leu Glu Ser Leu Pro Arg Asn Glu Val Phe
            180                 185                 190

Asn Trp Val Pro Ala Val Ser Lys Glu Leu Thr Gly Arg Met Leu Ala
        195                 200                 205

Thr Leu Leu Asp Phe Pro Tyr Glu Glu Arg His Lys Leu Ile Glu Trp
    210                 215                 220

Ser Asp Gln Met Ala Gly Thr Ala Ala Ala Thr Gly Gly Glu Phe Glu
225                 230                 235                 240

Asp Glu Glu Ser Met Phe Glu Ala Ala Ala Asp Met Ala Trp Ser Phe
                245                 250                 255

Ser Arg Leu Trp Arg Asp Lys Lys Ala Arg Arg Ala Ala Gly Glu Ala
            260                 265                 270

Pro Gly Phe Asp Leu Ile Ser Leu Leu Gln Ser Gly Glu Asp Thr Gln
        275                 280                 285

Asp Leu Ile Asn Arg Pro Met Glu Phe Ile Gly Asn Leu Ala Leu Leu

290                 295                 300

Ile Val Gly Gly Asn Asp Thr Thr Arg Asn Ser Met Ser Gly Gly Val
305                 310                 315                 320

Leu Ala Met Asn Gln Phe Pro Ala Glu Phe Ala Lys Leu Lys Ala Asn
                325                 330                 335

Pro Lys Leu Leu Pro Asn Met Val Ser Glu Ile Ile Arg Trp Gln Thr
            340                 345                 350

Pro Leu Ala His Met Arg Arg Val Ala Thr Gln Asp Val Glu Leu Arg
        355                 360                 365

Gly Gln Thr Ile Lys Lys Gly Asp Arg Val Leu Met Trp Tyr Ala Ser
    370                 375                 380

Gly Asn Arg Asp Glu Arg Lys Phe Glu Lys Pro Asp Asp Phe Ile Ile
385                 390                 395                 400

Asp Arg Glu Gly Ala Arg Asn His Ile Ala Phe Gly Tyr Gly Ile His
                405                 410                 415

Arg Cys Met Gly Asn Arg Leu Ala Glu Leu Gln Leu Arg Ile Leu Trp
            420                 425                 430

Glu Glu Leu Leu Pro Arg Phe Glu Asn Ile Glu Val Ile Gly Glu Pro
        435                 440                 445

Glu Arg Val Gln Ser Asn Phe Val Arg Gly Tyr Ser Lys Met Met Val
    450                 455                 460

Lys Leu Thr Ala Lys Lys
465                 470

<210> 4
<211> 470
<212> PRT
<213> Artificial Sequence

<220>
<223> Description of Artificial Sequence: hybrid protein

<400> 4
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Arg Ala Val Gln
145                 150                 155                 160

Gly Val Val Ala Pro Lys Asn Leu Lys Glu Met Glu Gly Leu Ile Arg
                165                 170                 175

Ser Arg Ala Ala Glu Val Leu Asp Ser Leu Pro Leu Asp Lys Pro Phe
            180                 185                 190

Asp Trp Val Pro Ala Val Ser Lys Glu Leu Thr Gly Arg Met Leu Ala
        195                 200                 205

Thr Leu Leu Asp Phe Pro Tyr Glu Asp Arg His Lys Leu Val Glu Trp
    210                 215                 220

Ser Asp Arg Leu Ser Gly Ala Ala Ser Ala Thr Gly Gly Glu Phe Thr
225                 230                 235                 240

Asp Glu Asp Val Met Phe Asp Asp Ala Ala Asp Met Ala Arg Ala Phe
                245                 250                 255

Ser Arg Leu Trp Arg Asp Lys Glu Ala Arg Arg Ala Ser Gly Glu Glu
            260                 265                 270

Pro Gly Phe Asp Leu Ile Ser Met Leu Gln Ser Asn Asp Asp Thr Lys
        275                 280                 285

Asp Leu Ile Asn Arg Pro Met Glu Phe Ile Gly Asn Leu Ala Leu Leu
    290                 295                 300

Ile Val Gly Gly Asn Asp Thr Thr Arg Asn Ser Met Ser Gly Gly Val
305                 310                 315                 320

Leu Ala Leu Asn Gln Phe Pro Glu Glu Phe Ser Lys Leu Lys Ala Asn
                325                 330                 335

Pro Glu Leu Ile Pro Asn Met Val Ser Glu Ile Ile Arg Trp Gln Thr
            340                 345                 350

Pro Leu Ala Asn Met Arg Arg Val Ala Thr Gln Asp Val Glu Leu Arg
        355                 360                 365

Gly Gln Thr Ile Lys Lys Gly Asp Arg Val Leu Met Trp Tyr Ala Ser
    370                 375                 380

Gly Asn Arg Asp Glu Arg Lys Phe Asp Asn Pro Asp Gln Leu Ile Ile
385                 390                 395                 400

Asp Arg Lys Asp Ala Arg Asn His Ile Ser Phe Gly Tyr Gly Ile His
                405                 410                 415

Arg Cys Met Gly Asn Ser Leu Ala Glu Leu Gln Leu Arg Ile Leu Trp
            420                 425                 430

Glu Glu Leu Leu Pro Arg Phe Glu Asn Ile Glu Val Ile Gly Glu Pro
        435                 440                 445

Glu Arg Val Gln Ser Asn Phe Val Arg Gly Tyr Ser Lys Met Met Val
    450                 455                 460

Lys Leu Thr Ala Lys Lys
465                 470

<210> 5
<211> 470
<212> PRT
<213> Artificial Sequence

<220>
<223> Description of Artificial Sequence: hybrid protein

<400> 5
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Arg Ala Val Gln
145                 150                 155                 160

Gly Val Val Ala Pro Lys Asn Leu Lys Glu Met Glu Gly Leu Ile Arg
                165                 170                 175

Ser Arg Ala Ala Glu Val Leu Asp Ser Leu Pro Leu Asp Lys Pro Phe
            180                 185                 190

Asp Trp Val Pro Ala Val Ser Lys Glu Leu Thr Gly Arg Met Leu Ala
        195                 200                 205

Thr Leu Leu Asp Phe Pro Tyr Glu Asp Arg His Lys Leu Val Glu Trp
    210                 215                 220

Ser Asp Arg Leu Ser Gly Ala Ala Ser Ala Thr Gly Gly Glu Phe Thr
225                 230                 235                 240

Asp Glu Asp Val Met Phe Asp Asp Ala Ala Asp Met Ala Arg Ala Phe
                245                 250                 255

Ser Arg Leu Trp Arg Asp Lys Glu Ala Arg Arg Ala Ser Gly Glu Glu
            260                 265                 270

Pro Gly Phe Asp Leu Ile Ser Met Leu Gln Ser Asn Glu Asp Thr Lys
        275                 280                 285

Asp Leu Ile Asn Arg Pro Met Glu Phe Ile Gly Asn Leu Ala Leu Leu
    290                 295                 300

Ile Val Gly Gly Asn Asp Thr Thr Arg Asn Ser Met Ser Gly Gly Val
305                 310                 315                 320

Leu Ala Leu Asn Gln Phe Pro Glu Glu Phe Arg Lys Leu Lys Ala Lys
                325                 330                 335

Pro Glu Leu Ile Pro Asn Met Val Ser Glu Ile Ile Arg Trp Gln Thr
            340                 345                 350

Pro Leu Ala Asn Met Arg Arg Val Ala Thr Gln Asp Val Glu Leu Arg
        355                 360                 365

Gly Gln Thr Ile Lys Lys Gly Asp Arg Val Leu Met Trp Tyr Ala Ser
    370                 375                 380

Gly Asn Arg Asp Glu Arg Lys Phe Glu Asn Pro Asp Gln Leu Ile Ile
385                 390                 395                 400

Asp Arg Lys Asp Ala Arg Asn His Ile Ser Phe Gly Tyr Gly Ile His
                405                 410                 415

Arg Cys Met Gly Asn Arg Leu Ala Glu Leu Gln Leu Arg Ile Leu Trp
            420                 425                 430

Glu Glu Leu Leu Pro Arg Phe Glu Asn Ile Glu Val Ile Gly Glu Pro
        435                 440                 445

Glu Arg Val Gln Ser Asn Phe Val Arg Gly Tyr Ser Lys Met Met Val
    450                 455                 460

Lys Leu Thr Ala Lys Lys
465                 470

<210> 6
<211> 469
<212> PRT
<213> Artificial Sequence

<220>
<223> Description of Artificial Sequence: hybrid protein

<400> 6
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Glu Gln Arg Lys Ala Val Gln
145                 150                 155                 160

Asn Val Val Ala Pro Lys Asn Leu Lys Glu Met Glu Ser Leu Ile Arg
                165                 170                 175

Glu Arg Thr Arg Glu Val Leu Asp Ser Leu Pro Val Asp Gln Pro Phe
            180                 185                 190

Asp Trp Val Gln Gln Val Ser Ile Glu Leu Thr Ala Arg Met Leu Ala
        195                 200                 205

Thr Leu Leu Asp Phe Pro Tyr Glu Gln Arg His Lys Leu Val Tyr Trp
    210                 215                 220

Ser Asp Val Pro Thr Ala Ser Pro Glu Thr Thr Gly Asp Lys Val Gly

225                 230                 235                 240

Pro Asp Glu Ile Phe Ala Ala Ala Ala Asp Ala Gly Lys His Phe Val
                245                 250                 255

Ser Leu Trp His Glu Lys Ala Ala Arg Lys Ala Ala Gly Glu Ala Pro
            260                 265                 270

Gly Phe Asp Leu Ile Ser Leu Met Gln Ala Asn Glu Glu Thr Lys Asp
        275                 280                 285

Met Val Lys Arg Pro Met Glu Phe Met Gly Asn Leu Val Leu Leu Ile
    290                 295                 300

Val Gly Gly Asn Asp Thr Thr Arg Asn Ser Met Thr Gly Gly Val Leu
305                 310                 315                 320

Ala Leu Asn Gln Phe Pro Gly Glu Phe Ile Lys Leu Lys Glu Asn Pro
                325                 330                 335

Asp Leu Ile Pro Asn Met Val Ser Glu Ile Ile Arg Trp Gln Thr Pro
            340                 345                 350

Leu Ala Tyr Met Arg Arg Ile Ala Lys Gln Asp Val Glu Leu Asn Gly
        355                 360                 365

Glu Thr Ile Arg Lys Gly Asp Lys Val Val Met Trp Tyr Ala Ser Gly
    370                 375                 380

Asn Arg Asp Glu Arg Lys Ile Glu Asn Pro Asp Val Phe Gln Ile Asp
385                 390                 395                 400

Arg Lys Gly Ala Arg Asn His Leu Ser Phe Gly Phe Gly Val His Arg
                405                 410                 415

Cys Met Gly Asn Arg Leu Ala Glu Leu Gln Leu Arg Ile Leu Trp Glu
            420                 425                 430

Glu Leu Leu Pro Arg Phe Glu Asn Ile Glu Val Ile Gly Glu Pro Glu
        435                 440                 445

Arg Val Gln Ser Asn Phe Val Arg Gly Tyr Ser Lys Met Met Val Lys
    450                 455                 460

Leu Thr Ala Lys Lys
465

<210> 7
<211> 469
<212> PRT
<213> Artificial Sequence

<220>
<223> Description of Artificial Sequence: hybrid protein

<400> 7
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Glu Gln Arg Lys Ala Val Gln
145                 150                 155                 160

Asn Val Val Ala Pro Lys Asn Leu Lys Glu Met Glu Ser Leu Ile Arg
                165                 170                 175

Glu Arg Thr Arg Glu Val Leu Asp Ser Leu Pro Val Asp Gln Pro Phe
            180                 185                 190

Asp Trp Val Gln Gln Val Ser Ile Glu Leu Thr Ala Arg Met Leu Ala
        195                 200                 205

Thr Leu Leu Asp Phe Pro Tyr Glu Gln Arg His Lys Leu Val Tyr Trp
    210                 215                 220

Ser Asp Val Ser Thr Ala Ser Pro Glu Thr Thr Gly Gly Lys Val Gly
225                 230                 235                 240

Pro Asp Glu Ile Phe Ala Ala Ala Ala Asp Ala Gly Lys His Phe Val
                245                 250                 255

Ser Leu Trp His Glu Lys Ala Ala Arg Lys Ala Ala Gly Glu Ala Pro
            260                 265                 270

Gly Phe Asp Leu Ile Ser Leu Met Gln Ala Asn Glu Glu Thr Lys Asp
        275                 280                 285

Met Val Lys Arg Pro Met Glu Phe Met Gly Asn Leu Val Leu Leu Ile
    290                 295                 300

Val Gly Gly Asn Asp Thr Thr Arg Asn Ser Met Thr Gly Gly Val Leu
305                 310                 315                 320

Ala Leu Asn Gln Phe Pro Gly Glu Phe Ile Lys Leu Lys Glu Asn Pro
                325                 330                 335

Asp Leu Ile Pro Asn Met Val Ser Glu Ile Ile Arg Trp Gln Thr Pro
            340                 345                 350

Leu Ala Tyr Met Arg Arg Ile Ala Lys Gln Asp Val Glu Leu Asn Gly
        355                 360                 365

Glu Thr Ile Arg Lys Gly Asp Lys Val Val Met Trp Tyr Ala Ser Gly
    370                 375                 380

Asn Arg Asp Glu Arg Lys Ile Glu Asn Pro Asp Val Phe Gln Ile Asp
385                 390                 395                 400

Arg Lys Gly Ala Arg Asn His Leu Ser Phe Gly Phe Gly Val His Arg
                405                 410                 415

Cys Met Gly Asn Ser Phe Ala Glu Leu Gln Leu Arg Ile Leu Trp Glu
            420                 425                 430

Glu Leu Leu Pro Arg Phe Glu Asn Ile Glu Val Ile Gly Glu Pro Glu
        435                 440                 445

Arg Val Gln Ser Asn Phe Val Arg Gly Tyr Ser Lys Met Met Val Lys
    450                 455                 460

Leu Thr Ala Lys Lys
465

<210> 8
<211> 469
<212> PRT
<213> Artificial Sequence

<220>
<223> Description of Artificial Sequence: hybrid protein

<400> 8
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Glu His Asp Arg Gln Arg Ala Ala Val Gln
145                 150                 155                 160

Gly Val Val Ala Pro Lys Asn Leu Arg Glu Met Glu Ser Leu Ile Arg
                165                 170                 175

Glu Arg Ala Arg Asp Val Leu Asp Gly Leu Pro Leu Gly Glu Pro Phe
            180                 185                 190

Asn Trp Val Gln His Val Ser Ile Glu Leu Thr Ala Arg Met Leu Ala
        195                 200                 205

Thr Leu Leu Asp Phe Pro Phe Glu Gln Arg Arg Lys Leu Val Glu Trp
    210                 215                 220

Ser Asp Leu Ala Thr Ser Met Glu Gln Ala Val Gly Ala Pro Ser Asp
225                 230                 235                 240

Asn Asp Glu Ile Phe Arg Gly Met Val Asp Met Ala Arg Gly Leu Ser
                245                 250                 255

Ala His Trp Arg Asp Lys Ala Ala Arg Thr Ala Ala Gly Glu Glu Pro
            260                 265                 270

Gly Phe Asp Leu Ile Thr Met Leu Gln Arg Asp Glu Ser Thr Lys Asp
        275                 280                 285

Leu Leu Asp Arg Pro Met Glu Phe Leu Gly Asn Leu Thr Leu Leu Ile
    290                 295                 300

Val Gly Gly Asn Asp Thr Thr Arg Asn Ser Met Ser Gly Gly Val Leu
305                 310                 315                 320

Ala Leu Asn Gln Phe Pro Asp Gln Phe Glu Lys Leu Lys Ala Asn Pro
                325                 330                 335

Gly Leu Ile Pro Asn Met Val Ser Glu Ile Ile Arg Trp Gln Thr Pro
            340                 345                 350

Leu Ala His Met Arg Arg Ile Ala Lys Ala Asp Thr Met Leu Asn Gly
        355                 360                 365

Gln Phe Ile Arg Lys Gly Asp Lys Val Leu Met Trp Tyr Ala Ser Gly
    370                 375                 380

Asn Arg Asp Glu Arg Val Phe Glu Arg Pro Asp Glu Leu Ile Ile Asp
385                 390                 395                 400

Arg Pro Asn Ala Arg Asn His Val Ser Phe Gly Phe Gly Ile His Arg
                405                 410                 415

Cys Met Gly Asn Ser Leu Ala Glu Leu Gln Leu Arg Ile Leu Trp Glu
            420                 425                 430

Glu Leu Leu Pro Arg Phe Glu Asn Ile Glu Val Ile Gly Glu Pro Glu
        435                 440                 445

Arg Val Gln Ser Asn Phe Val Arg Gly Tyr Ser Lys Met Met Val Lys
    450                 455                 460

Leu Thr Ala Lys Lys
465

<210> 9
<211> 469
<212> PRT
<213> Artificial Sequence

<220>
<223> Description of Artificial Sequence: hybrid protein

<400> 9
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Ile Gln Arg Gln Ala Val Gln
145                 150                 155                 160

Gly Val Val Ala Pro Lys Asn Leu Lys Glu Met Glu Gly Leu Ile Arg

                165                 170                 175

Gln Arg Val Gln Asp Val Leu Asp Lys Leu Pro Val Asp Gln Pro Phe
            180                 185                 190

Asp Trp Val Asn Arg Val Ser Ile Glu Leu Thr Ser Arg Met Leu Ala
        195                 200                 205

Thr Leu Leu Asp Phe Pro Tyr Glu Gln Arg His Lys Leu Val Tyr Trp
    210                 215                 220

Ser Asp Met Ala Ala Ala Ser Ala Glu Thr Thr Gly Gly Ser Gly Asp
225                 230                 235                 240

Met Asp Glu Leu Phe His Gly Ile Ala Asp Met Ala Arg His Phe Ser
                245                 250                 255

Ala Leu Trp His Asp Lys Ala Ala Arg Arg Ala Ala Gly Glu Glu Pro
            260                 265                 270

Gly Phe Asp Leu Ile Thr Met Leu Gln Ser Asn Glu Ser Thr Lys Asp
        275                 280                 285

Leu Ile Lys Arg Pro Met Glu Phe Leu Gly Asn Leu Val Leu Leu Ile
    290                 295                 300

Val Gly Gly Asn Asp Thr Thr Arg Asn Ser Met Ser Gly Gly Val Leu
305                 310                 315                 320

Ala Leu Asn Arg Tyr Pro Asp Gln Phe Glu Lys Leu Lys Ala Asn Pro
                325                 330                 335

Glu Leu Ile Pro Asn Met Val Ser Glu Val Ile Arg Trp Gln Thr Pro
            340                 345                 350

Leu Ala Tyr Met Arg Arg Ile Ala Lys Ala Asp Thr Met Leu Ser Gly
        355                 360                 365

Gln Phe Ile Arg Lys Gly Asp Lys Val Val Met Trp Tyr Ala Ser Gly
    370                 375                 380

Asn Arg Asp Glu Arg Val Phe Asp Arg Pro Asp Asp Leu Ile Ile Asp
385                 390                 395                 400

Arg Ala Asn Ala Arg Asn His Ile Ser Phe Gly Phe Gly Ile His Arg
                405                 410                 415

Cys Met Gly Asn Arg Leu Ala Glu Leu Gln Leu Arg Ile Leu Trp Glu

420                 425                 430

Glu Leu Leu Pro Arg Phe Glu Asn Ile Glu Val Ile Gly Glu Pro Glu
        435                 440                 445

Arg Val Gln Ser Asn Phe Val Arg Gly Tyr Ser Lys Met Met Val Lys
    450                 455                 460

Leu Thr Ala Lys Lys
465

<210> 10
<211> 469
<212> PRT
<213> Artificial Sequence

<220>
<223> Description of Artificial Sequence:hybrid protein

<400> 10
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
             20                  25                  30

Lys Asn Leu Met Lys Val Lys Arg Lys Thr Ile Gly Thr Ser Arg Pro
         35                  40                  45

Gln Val His Phe Val Glu Thr Asp Leu Pro Asp Val Asn Asp Leu Ala
     50                  55                  60

Ile Glu Asp Ile Asp Thr Ser Asn Pro Phe Leu Tyr Arg Gln Gly Lys
 65                  70                  75                  80

Ala Asn Ala Tyr Phe Lys Arg Leu Arg Asp Glu Ala Pro Val His Tyr
                 85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Arg His Asp Lys Gln Arg Ala Ala Val Gln
145                 150                 155                 160

Gly Val Val Ala Pro Lys Asn Leu Arg Glu Met Glu Gly Leu Ile Arg
                165                 170                 175

Ser Arg Val Gln Glu Val Leu Asp Asn Leu Pro Val Asp Gln Pro Phe
            180                 185                 190

Asp Trp Ile Gln Asn Val Ser Ile Glu Leu Thr Ala Arg Met Leu Ala
        195                 200                 205

Thr Leu Leu Asp Phe Pro Tyr Glu Gln Arg Arg Lys Leu Val Glu Trp
    210                 215                 220

Ser Asp Leu Ala Thr Ser Met Glu Gln Ala Asn Gly Gly Pro Ser Asp
225                 230                 235                 240

Leu Asp Asp Thr Phe Ala Gly Met Arg Asp Met Ala Arg Gly Leu Ser
                245                 250                 255

Glu His Trp His Asp Lys Ala Ala Arg Arg Ala Ala Gly Glu Glu Pro
            260                 265                 270

Gly Phe Asp Leu Ile Thr Met Leu Gln Ser Asn Glu Ser Thr Lys Asp
        275                 280                 285

Leu Ile Lys Arg Pro Met Glu Phe Leu Gly Asn Leu Val Leu Leu Ile
    290                 295                 300

Val Gly Gly Asn Asp Thr Thr Arg Asn Ser Met Ser Gly Gly Val Leu
305                 310                 315                 320

Ala Leu Asn Arg Tyr Pro Asp Gln Phe Glu Lys Leu Lys Ala Asn Pro
                325                 330                 335

Asp Leu Ile Pro Asn Met Val Ser Glu Val Ile Arg Trp Gln Thr Pro
            340                 345                 350

Leu Ala Tyr Met Arg Arg Ile Ala Lys Ala Asp Thr Met Leu Asn Gly
        355                 360                 365

Gln Phe Ile Arg Lys Gly Asp Lys Val Val Met Trp Tyr Ala Ser Gly
    370                 375                 380

Asn Arg Asp Glu Arg Val Phe Asp Arg Pro Asp Asp Leu Ile Ile Asp
385                 390                 395                 400

Arg Ala Asn Ala Arg Asn His Ile Ser Phe Gly Phe Gly Ile His Arg
                405                 410                 415

Cys Met Gly Asn Arg Leu Ala Glu Leu Gln Leu Arg Ile Leu Trp Glu
            420                 425                 430

Glu Leu Leu Pro Arg Phe Glu Asn Ile Glu Val Ile Gly Glu Pro Glu
        435                 440                 445

Arg Val Gln Ser Asn Phe Val Arg Gly Tyr Ser Lys Met Met Val Lys
    450                 455                 460

Leu Thr Ala Lys Lys
465

<210> 11
<211> 469
<212> PRT
<213> Artificial Sequence

<220>
<223> Description of Artificial Sequence:hybrid protein

<400> 11
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Arg Gln Arg Ala Ala Val Gln
145                 150                 155                 160

Gly Val Val Ala Pro Lys Asn Leu Arg Glu Met Glu Gly Leu Ile Arg
                165                 170                 175

Ser Arg Val Gln Glu Val Leu Asp Asn Leu Pro Val Asp Gln Pro Phe
            180                 185                 190

Asp Trp Val Gln Asn Val Ser Ile Glu Leu Thr Ala Arg Met Leu Ala
        195                 200                 205

Thr Leu Leu Asp Phe Pro Tyr Glu Gln Arg Arg Lys Leu Val Glu Trp
    210                 215                 220

Ser Asp Leu Ala Thr Ser Met Glu Gln Ala Asn Gly Gly Pro Ser Asp
225                 230                 235                 240

Leu Asp Glu Thr Phe Val Gly Met Arg Asp Met Ala Arg Gly Leu Ser
                245                 250                 255

Glu His Trp His Asp Lys Ala Ala Arg Arg Ala Ala Gly Glu Glu Pro
            260                 265                 270

Gly Phe Asp Leu Ile Thr Met Leu Gln Ser Asn Glu Ser Thr Lys Asp
        275                 280                 285

Leu Ile Lys Arg Pro Met Glu Phe Leu Gly Asn Leu Val Leu Leu Ile
    290                 295                 300

Val Gly Gly Asn Asp Thr Thr Arg Asn Ser Met Ser Gly Gly Val Leu
305                 310                 315                 320

Ala Leu Asn Arg Tyr Pro Asp Gln Phe Glu Lys Leu Lys Ala Asn Pro
                325                 330                 335

Glu Leu Ile Pro Asn Met Val Ser Glu Val Ile Arg Trp Gln Thr Pro
            340                 345                 350

Leu Ala Tyr Met Arg Arg Ile Ala Lys Ala Asp Thr Met Leu Asn Gly
        355                 360                 365

Gln Phe Ile Arg Lys Gly Asp Lys Val Val Met Trp Tyr Ala Ser Gly
    370                 375                 380

Asn Arg Asp Glu Arg Val Phe Asp Arg Pro Asp Asp Leu Ile Ile Asp
385                 390                 395                 400

Arg Ala Asn Ala Arg Asn His Ile Ser Phe Gly Phe Gly Ile His Arg
                405                 410                 415

Cys Met Gly Asn Ser Leu Ala Glu Leu Gln Leu Arg Ile Leu Trp Glu
            420                 425                 430

Glu Leu Leu Pro Arg Phe Glu Asn Ile Glu Val Ile Gly Glu Pro Glu
        435                 440                 445

Arg Val Gln Ser Asn Phe Val Arg Gly Tyr Ser Lys Met Met Val Lys
    450                 455                 460

Leu Thr Ala Lys Lys
465

<210> 12
<211> 461
<212> PRT
<213> Artificial Sequence

<220>
<223> Description of Artificial Sequence:hybrid protein

<400> 12
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
             20                  25                  30

Lys Asn Leu Met Lys Val Lys Arg Lys Thr Ile Gly Thr Ser Arg Pro
         35                  40                  45

Gln Val His Phe Val Glu Thr Asp Leu Pro Asp Val Asn Asp Leu Ala
     50                  55                  60

Ile Glu Asp Ile Asp Thr Ser Asn Pro Phe Leu Tyr Arg Gln Gly Lys
 65                  70                  75                  80

Ala Asn Ala Tyr Phe Lys Arg Leu Arg Asp Glu Ala Pro Val His Tyr
                 85                  90                  95

Gln Lys Asn Ser Ala Phe Gly Pro Phe Trp Ser Val Thr Arg Tyr Glu

            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Lys Thr Val Ser
145                 150                 155                 160

Pro Ile Val Ser Pro His Ser Leu Met Gln Leu Glu Pro Ile Ile Arg
                165                 170                 175

Glu Arg Ala Ala Gly Leu Leu Asp Ser Leu Pro Ile Gly Glu Thr Phe
            180                 185                 190

Asp Trp Val Asp Arg Ile Ser Ile Glu Leu Thr Thr Gln Met Leu Val
        195                 200                 205

Thr Leu Phe Asp Phe Pro Phe His Asp Arg Arg Lys Leu Thr Arg Trp
    210                 215                 220

Ser Asp Val Ala Thr Ala Ala Pro Gly Met Gly Val Ile Glu Thr Glu
225                 230                 235                 240

Glu Gln Arg Arg Glu Glu Leu Phe Glu Cys Leu Ala Tyr Phe Thr Asn
                245                 250                 255

Leu Trp Asn Glu Arg Val Asn Ala Pro Pro Ala Asn Asn Leu Ile Ser
            260                 265                 270

Leu Leu Ala His Gly Glu Gln Thr Arg Asn Met Glu Pro Met Glu Tyr
        275                 280                 285

Leu Gly Asn Leu Ile Leu Leu Ile Val Gly Gly Asn Asp Thr Thr Arg
    290                 295                 300

Asn Thr Met Thr Gly Ser Val Leu Ala Leu Asn Gln Asn Pro Asp Gln
305                 310                 315                 320

Tyr Ala Lys Leu Lys Ala Asn Pro Glu Leu Ile Pro Ser Met Val Ser
                325                 330                 335

Glu Thr Ile Arg Trp Gln Thr Pro Leu Ala His Met Arg Arg Thr Ala
            340                 345                 350

Leu Ala Asp Tyr Glu Ile Gly Gly Lys Thr Ile Lys Lys Gly Asp Lys

        355                 360                 365

Val Ala Met Trp Tyr Val Ser Gly Asn Arg Asp Glu Thr Ala Ile Glu
    370                 375                 380

Asn Pro Asp Ala Tyr Ile Ile Asp Arg Glu Arg Pro Arg Gln His Leu
385                 390                 395                 400

Ser Phe Gly Phe Gly Ile His Arg Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460

<210> 13
<211> 464
<212> PRT
<213> Artificial Sequence

<220>
<223> Description of Artificial Sequence:hybrid protein

<400> 13
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Lys Thr Val Ser
145                 150                 155                 160

Pro Ile Val Ser Pro Ala Asn Leu His Leu Met Glu Pro Leu Ile Arg
                165                 170                 175

Ser Arg Ile Thr Lys Thr Leu Asp Glu Leu Pro Ile Gly Glu Pro Phe
            180                 185                 190

Asp Trp Val Asp Arg Val Ser Ile Glu Leu Thr Thr Gln Met Leu Ala
        195                 200                 205

Thr Leu Phe Asp Phe Pro Trp Asp Glu Arg Arg Lys Leu Thr Arg Trp
    210                 215                 220

Ser Asp Ile Ala Thr Ala Gly Pro Gln Ser Gly Leu Phe Leu Thr Asp
225                 230                 235                 240

Asp Tyr Glu Thr Glu Arg Arg Met Glu Leu Phe Ala Cys Val Asp Tyr
                245                 250                 255

Phe Thr Arg Leu Trp Asn Glu Arg Val Asn Ala Pro Pro Lys Gly Asp
            260                 265                 270

Leu Ile Ser Met Leu Ala His Gly Glu Ala Thr Arg Asn Met Asp Arg
        275                 280                 285

Met Glu Tyr Leu Gly Asn Leu Leu Leu Leu Ile Ile Gly Gly Asn Asp
    290                 295                 300

Thr Thr Arg Asn Thr Met Thr Gly Ser Ile Leu Ala Met Asn Glu Asn
305                 310                 315                 320

Pro Glu Gln Leu Arg Lys Leu Gln Ala Lys Pro Asp Leu Ile Pro Ser
                325                 330                 335

Met Val Ser Glu Thr Ile Arg Trp Gln Thr Pro Leu Ser Asn Met Arg
            340                 345                 350

Arg Thr Ala Thr Gln Asp Phe Glu Leu Gly Gly Lys Leu Ile Lys Lys
        355                 360                 365

Gly Asp Lys Val Leu Ile Trp Tyr Ala Ser Gly Asn Arg Asp Glu Glu
    370                 375                 380

Ala Ile Glu Asn Pro Glu Ala Tyr Ile Ile Asp Arg Glu Arg Pro Arg
385                 390                 395                 400

Asn His Leu Ser Phe Gly Phe Gly Ile His Arg Cys Met Gly Asn Arg
                405                 410                 415

Leu Ala Glu Leu Gln Leu Arg Ile Leu Trp Glu Glu Leu Leu Pro Arg
            420                 425                 430

Phe Glu Asn Ile Glu Val Ile Gly Glu Pro Glu Arg Val Gln Ser Asn
        435                 440                 445

Phe Val Arg Gly Tyr Ser Lys Met Met Val Lys Leu Thr Ala Lys Lys
    450                 455                 460

<210> 14
<211> 464
<212> PRT
<213> Artificial Sequence

<220>
<223> Description of Artificial Sequence:hybrid protein

<400> 14
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Ile Gln Arg Lys Thr Val Ser
145                 150                 155                 160

Pro Ile Val Ser Pro Gln Asn Leu His Leu Leu Glu Pro Leu Ile Arg
                165                 170                 175

Ser Arg Ile Thr Lys Thr Leu Asp Glu Leu Pro Ile Gly Gln Pro Phe
            180                 185                 190

Asp Trp Val Asp Arg Val Ser Ile Glu Leu Thr Thr Gln Met Leu Ala
        195                 200                 205

Thr Leu Phe Asp Phe Pro Phe Glu Glu Arg Arg Lys Leu Thr Phe Trp
    210                 215                 220

Ser Asp Val Ala Thr Ala Pro Pro Glu Ser Lys Leu Phe Lys Thr Asp
225                 230                 235                 240

Asp Pro Glu Thr Glu Arg Lys Met Ile Leu Phe Glu Cys Val Asp Tyr
                245                 250                 255

Phe Thr Arg Leu Trp Asn Glu Arg Val Asn Ala Pro Pro Lys Gly Asp
            260                 265                 270

Leu Ile Ser Met Leu Ala His Gly Glu Ala Thr Arg Asn Met Asp Arg
        275                 280                 285

Met Glu Tyr Leu Gly Asn Leu Ile Leu Leu Ile Val Gly Gly Asn Asp
    290                 295                 300

Thr Thr Arg Asn Thr Met Ser Gly Ser Ile Val Ala Met His Gln Asn
305                 310                 315                 320

Pro Asp Gln Leu Ala Lys Leu Gln Ala Asp His Ser Leu Val Pro Ser
                325                 330                 335

Met Val Ser Glu Thr Ile Arg Trp Gln Thr Pro Leu Ala His Met Arg
            340                 345                 350

Arg Thr Ala Thr Gln Asp Phe Glu Leu Gly Gly Lys Thr Ile Lys Lys
        355                 360                 365

Gly Asp Lys Val Val Met Trp Tyr Val Ser Gly Asn Arg Asp Asp Glu
    370                 375                 380

Val Ile Glu Arg Ala Asp Glu Phe Leu Ile Asp Arg Lys Asn Pro Arg
385                 390                 395                 400

His His Ala Ser Phe Gly Phe Gly Ile His Ser Cys Met Gly Asn Arg
                405                 410                 415

Leu Ala Glu Leu Gln Leu Arg Ile Leu Trp Glu Glu Leu Leu Pro Arg
            420                 425                 430

Phe Glu Asn Ile Glu Val Ile Gly Glu Pro Glu Arg Val Gln Ser Asn
        435                 440                 445

Phe Val Arg Gly Tyr Ser Lys Met Met Val Lys Leu Thr Ala Lys Lys
    450                 455                 460

<210> 15
<211> 461
<212> PRT
<213> Artificial Sequence

<220>
<223> Description of Artificial Sequence:hybrid protein

<400> 15
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala

50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Asp Gln Arg Lys Val Val Ser
145                 150                 155                 160

Pro Ala Leu Gly Pro Glu Asn Leu Ala Lys Leu Ala Asp Gly Ile Arg
                165                 170                 175

Thr Arg Thr Gly Gln Leu Leu Asp Gln Leu Pro Ile Asn Glu Thr Phe
            180                 185                 190

Asp Trp Val Asp Arg Val Ser Ile Glu Leu Thr Thr Gln Met Leu Ala
        195                 200                 205

Ile Leu Phe Asp Phe Pro Trp Glu Glu Arg Arg Lys Leu Thr His Trp
    210                 215                 220

Ser Asp Thr Ala Thr Ala Met Thr Ala His Pro Thr Asp Ala Glu Lys
225                 230                 235                 240

Ala Ala Gly Leu Ala Glu Leu Gly Glu Cys Ala Ala Tyr Phe Thr Arg
                245                 250                 255

Leu Trp Asn Glu Arg Val Asn Ala Glu Pro Arg Ala Asp Leu Leu Ser
            260                 265                 270

Met Leu Ala His Ser Glu Ala Thr Arg Asn Met Gly Pro Lys Glu Phe
        275                 280                 285

Leu Gly Asn Leu Ile Leu Leu Ile Val Gly Gly Asn Asp Thr Thr Arg
    290                 295                 300

Asn Ser Met Thr Gly Gly Leu Leu Ala Leu His Asp Asn Pro Asp Gln

305                 310                 315                 320

Tyr Arg Lys Leu Cys Glu Asn Pro Lys Leu Val Asp Ser Leu Val Pro
                325                 330                 335

Glu Ile Ile Arg Trp Gln Thr Pro Val Thr Ser Met Arg Arg Thr Ala
            340                 345                 350

Leu Arg Asp Thr Val Leu Gly Gly Lys Val Ile His Glu Gly Asp Arg
        355                 360                 365

Val Ile Met Trp Tyr Ala Ser Ala Asn Arg Asp Pro Asp Ala Ile Glu
    370                 375                 380

Asn Pro Asn Ala Phe Ile Ile Asp Arg Ala Arg Pro Arg Gln His Leu
385                 390                 395                 400

Ser Phe Gly Phe Gly Ile His Arg Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460


<210> 16
<211> 461
<212> PRT
<213> Artificial Sequence

<220>
<223> Description of Artificial Sequence:hybrid protein

<400> 16
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
             20                  25                  30

Lys Asn Leu Met Lys Val Lys Arg Lys Thr Ile Gly Thr Ser Arg Pro
         35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Ile Gln Arg Lys Thr Val Ser
145                 150                 155                 160

Pro Ile Val Ser Pro Met Asn Leu Ala Lys Leu Glu Gly Val Ile Arg
                165                 170                 175

Glu Arg Ala Gly Leu Ile Leu Asp Gly Leu Pro Ile Gly Glu Pro Phe
            180                 185                 190

Asp Trp Val Asp Arg Val Ser Ile Glu Leu Thr Thr Gln Met Leu Ala
        195                 200                 205

Thr Leu Phe Asp Phe Pro Trp Glu Asp Arg Ala Lys Leu Thr Arg Trp
    210                 215                 220

Ser Asp Val Ala Thr Ala Leu Val Gly Thr Gly Ile Ile Asp Ser Glu
225                 230                 235                 240

Glu Gln Arg Met Glu Glu Leu Lys Gly Cys Ala Gln Tyr Met Thr Arg
                245                 250                 255

Leu Trp Asn Glu Arg Val Asn Val Pro Pro Gly Asn Asp Leu Ile Ser
            260                 265                 270

Met Met Ala His Thr Glu Ser Met Arg Asn Met Thr Pro Glu Glu Phe
        275                 280                 285

Leu Gly Asn Leu Ile Leu Leu Ile Val Gly Gly Asn Asp Thr Thr Arg
    290                 295                 300

Asn Ser Met Thr Gly Gly Val Leu Ala Leu Asn Glu Asn Pro Asp Glu
305                 310                 315                 320

Tyr Arg Lys Leu Cys Ala Asn Pro Ala Leu Ile Ala Ser Met Val Pro
                325                 330                 335

Glu Ile Val Arg Trp Gln Thr Pro Leu Ser His Met Arg Arg Thr Ala
            340                 345                 350

Leu Gln Asp Thr Glu Leu Gly Gly Lys Ser Ile Arg Lys Gly Asp Lys
        355                 360                 365

Val Ile Met Trp Tyr Val Ser Gly Asn Arg Asp Pro Glu Ala Ile Glu
    370                 375                 380

Asn Pro Asp Ala Phe Ile Ile Asp Arg Ala Arg Pro Arg His His Leu
385                 390                 395                 400

Ser Phe Gly Phe Gly Ile His Arg Cys Met Gly Asn Ser Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460

<210> 17
<211> 461
<212> PRT
<213> Artificial Sequence

<220>
<223> Description of Artificial Sequence:hybrid protein

<400> 17
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
             20                  25                  30

Lys Asn Leu Met Lys Val Lys Arg Lys Thr Ile Gly Thr Ser Arg Pro
         35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Met Ala Val Ser
145                 150                 155                 160

Pro Ile Val Ala Pro Glu Asn Leu Ala Lys Leu Glu Gly Leu Ile Arg
                165                 170                 175

Glu Arg Thr Gly Arg Ala Leu Asp Gly Leu Pro Ile Asn Glu Thr Phe
            180                 185                 190

Asp Trp Val Lys Arg Val Ser Ile Asn Leu Thr Thr Gln Met Leu Ala
        195                 200                 205

Thr Leu Phe Asp Phe Pro Trp Glu Asp Arg Ala Lys Leu Thr Arg Trp
    210                 215                 220

Ser Asp Val Ala Thr Ala Leu Val Gly Thr Gly Ile Ile Asp Ser Glu
225                 230                 235                 240

Glu Gln Arg Met Glu Glu Leu Lys Gly Cys Ala Gln Tyr Met Thr Arg
                245                 250                 255

Leu Trp Asn Glu Arg Val Asn Val Pro Pro Gly Asn Asp Leu Ile Ser
            260                 265                 270

Met Met Ala His Thr Glu Ser Met Arg Asn Met Thr Pro Glu Glu Phe
        275                 280                 285

Leu Gly Asn Leu Leu Leu Leu Ile Val Gly Gly Asn Asp Thr Thr Arg
    290                 295                 300

Asn Ser Met Thr Gly Gly Val Leu Ala Leu Asn Glu Asn Pro Asp Glu
305                 310                 315                 320

Tyr Arg Lys Leu Cys Ala Asn Pro Ala Leu Ile Ala Ser Met Val Pro
                325                 330                 335

Glu Ile Val Arg Trp Gln Thr Pro Leu Ala His Met Arg Arg Thr Ala
            340                 345                 350

Leu Gln Asp Thr Glu Leu Gly Gly Lys Ser Ile Arg Lys Gly Asp Lys
        355                 360                 365

Val Ile Met Trp Tyr Val Ser Gly Asn Arg Asp Pro Glu Ala Ile Glu
    370                 375                 380

Asn Pro Glu Ala Phe Ile Ile Asp Arg Ala Lys Pro Arg His His Leu
385                 390                 395                 400

Ser Phe Gly Phe Gly Ile His Ser Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460

<210> 18
<211> 461
<212> PRT
<213> Artificial Sequence

<220>
<223> Description of Artificial Sequence:hybrid protein

<400> 18
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
             20                  25                  30

Lys Asn Leu Met Lys Val Lys Arg Lys Thr Ile Gly Thr Ser Arg Pro

35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Met Ala Val Ser
145                 150                 155                 160

Pro Ile Val Ala Pro Glu Asn Leu Ala Lys Leu Glu Gly Leu Ile Arg
                165                 170                 175

Glu Arg Thr Gly Arg Ala Leu Asp Gly Leu Pro Ile Asn Glu Thr Phe
            180                 185                 190

Asp Trp Val Lys Arg Val Ser Ile Asn Leu Thr Thr Gln Met Leu Ala
        195                 200                 205

Thr Leu Phe Asp Phe Pro Trp Glu Asp Arg Ala Lys Leu Thr Arg Trp
    210                 215                 220

Ser Asp Val Ala Thr Ala Leu Val Gly Thr Gly Ile Ile Asp Ser Glu
225                 230                 235                 240

Glu Gln Arg Met Glu Glu Leu Lys Gly Cys Ala Gln Tyr Met Thr Arg
                245                 250                 255

Leu Trp Asn Glu Arg Val Asn Val Pro Pro Gly Asn Asp Leu Ile Ser
            260                 265                 270

Met Met Ala His Thr Glu Ser Met Arg Asn Met Thr Pro Glu Glu Phe
        275                 280                 285

Leu Gly Asn Leu Ile Leu Leu Ile Val Gly Gly Asn Asp Thr Thr Arg

290                 295                 300

Asn Ser Met Thr Gly Gly Val Leu Ala Ile Asn Glu Asn Pro Asp Glu
305                 310                 315                 320

Tyr Arg Lys Leu Cys Ala Asn Pro Ala Leu Ile Ala Ser Met Val Pro
                325                 330                 335

Glu Ile Val Arg Trp Gln Thr Pro Leu Ala His Met Arg Arg Thr Ala
            340                 345                 350

Leu Gln Asp Thr Glu Leu Gly Gly Lys Ser Ile Arg Lys Gly Asp Lys
        355                 360                 365

Val Ile Met Trp Tyr Val Ser Gly Asn Arg Asp Pro Glu Ala Ile Glu
    370                 375                 380

Asn Pro Asp Ala Phe Ile Ile Asp Arg Ala Arg Pro Arg His His Leu
385                 390                 395                 400

Ser Phe Gly Phe Gly Ile His Arg Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460

<210> 19
<211> 461
<212> PRT
<213> Artificial Sequence

<220>
<223> Description of Artificial Sequence:hybrid protein

<400> 19
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
             20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Met Ala Val Ser
145                 150                 155                 160

Pro Ile Val Ala Pro Glu Asn Leu Ala Lys Leu Glu Gly Leu Ile Arg
                165                 170                 175

Glu Arg Thr Gly Arg Ala Leu Asp Gly Leu Pro Ile Asn Glu Thr Phe
            180                 185                 190

Asp Trp Val Lys Arg Val Ser Ile Asn Leu Thr Thr Gln Met Leu Ala
        195                 200                 205

Thr Leu Phe Asp Phe Pro Trp Glu Asp Arg Ala Lys Leu Thr Arg Trp
    210                 215                 220

Ser Asp Val Ala Thr Ala Leu Val Gly Thr Gly Ile Ile Asp Ser Glu
225                 230                 235                 240

Glu Gln Arg Met Glu Glu Leu Lys Gly Cys Ala Gln Tyr Met Thr Arg
                245                 250                 255

Leu Trp Asn Glu Arg Val Asn Val Pro Pro Gly Asn Asp Leu Ile Ser
            260                 265                 270

Met Met Ala His Thr Glu Ser Met Arg Asn Met Thr Pro Glu Glu Phe
        275                 280                 285

Leu Gly Asn Leu Ile Leu Leu Ile Val Gly Gly Asn Asp Thr Thr Arg
    290                 295                 300

Asn Ser Met Thr Gly Gly Val Leu Ala Ile Asn Glu Asn Pro Asp Glu
305                 310                 315                 320

Tyr Arg Lys Leu Cys Ala Asn Pro Ala Leu Ile Ala Ser Met Val Pro
                325                 330                 335

Glu Ile Val Arg Trp Gln Thr Pro Leu Ser His Met Arg Arg Thr Ala
            340                 345                 350

Leu Gln Asp Thr Glu Leu Gly Gly Lys Ser Ile Arg Lys Gly Asp Lys
        355                 360                 365

Val Ile Met Trp Tyr Val Ser Gly Asn Arg Asp Pro Glu Ala Ile Glu
    370                 375                 380

Asn Pro Glu Ala Phe Ile Ile Asp Arg Ala Arg Pro Arg His His Leu
385                 390                 395                 400

Ser Phe Gly Phe Gly Ile His Arg Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460

<210> 20
<211> 461
<212> PRT
<213> Artificial Sequence

<220>
<223> Description of Artificial Sequence:hybrid protein

<400> 20
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
             20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Met Ala Val Ser
145                 150                 155                 160

Pro Ile Val Ala Pro Glu Asn Leu Ala Lys Leu Glu Gly Leu Ile Arg
                165                 170                 175

Glu Arg Thr Cys Arg Ala Leu Asp Gly Leu Pro Ile Asn Glu Thr Phe
            180                 185                 190

Asp Trp Val Lys Leu Val Ser Ile Asn Leu Thr Thr Gln Met Leu Ala
        195                 200                 205

Thr Leu Phe Asp Phe Pro Trp Glu Asp Arg Ala Lys Leu Thr Arg Trp
    210                 215                 220

Ser Asp Val Ala Thr Ala Leu Val Gly Thr Gly Ile Ile Asp Ser Glu
225                 230                 235                 240

Glu Gln Arg Met Glu Glu Leu Lys Gly Cys Ala Gln Tyr Met Thr Arg
                245                 250                 255

Leu Trp Asn Glu Arg Val Asn Val Pro Pro Gly Asn Asp Leu Ile Ser
            260                 265                 270

Met Met Ala His Thr Glu Ser Met Arg Asn Met Thr Pro Glu Glu Phe
        275                 280                 285

Leu Gly Asn Leu Ile Leu Leu Ile Val Gly Gly Asn Asp Thr Thr Arg
    290                 295                 300

Asn Ser Met Thr Gly Gly Val Pro Ala Ile Asn Glu Asn Pro Asp Glu
305                 310                 315                 320

Tyr Arg Lys Leu Cys Ala Asn Pro Ala Leu Ile Ala Ser Met Val Pro
                325                 330                 335

Glu Ile Val Arg Trp Gln Thr Pro Leu Ser His Met Arg Arg Thr Ala
            340                 345                 350

Leu Gln Asp Thr Glu Leu Gly Gly Lys Ser Ile Arg Lys Gly Asp Lys
        355                 360                 365

Val Ile Met Trp Tyr Val Ser Gly Asn Arg Asp Pro Glu Ala Ile Glu
    370                 375                 380

Asn Pro Asn Ala Phe Ile Ile Asp Arg Ala Lys Pro Arg His His Leu
385                 390                 395                 400

Ser Phe Gly Phe Gly Ile His Arg Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460


<210> 21
<211> 461
<212> PRT
<213> Artificial Sequence

<220>
<223> Description of Artificial Sequence:hybrid protein

<400> 21
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu

            20                  25                  30

Lys Asn Met Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Met Ala Val Ser
145                 150                 155                 160

Pro Ile Val Ala Pro Glu Asn Leu Ala Lys Leu Glu Gly Leu Ile Arg
                165                 170                 175

Glu Arg Thr Cys Arg Ala Leu Asp Gly Leu Pro Ile Asn Glu Thr Phe
            180                 185                 190

Asp Trp Val Lys Leu Val Ser Ile Asn Leu Thr Thr Gln Met Leu Ala
        195                 200                 205

Thr Leu Phe Asp Phe Pro Trp Glu Asp Arg Ala Lys Leu Thr Arg Trp
    210                 215                 220

Ser Asp Val Ala Thr Ala Leu Val Gly Thr Gly Ile Ile Asp Ser Glu
225                 230                 235                 240

Glu Gln Arg Met Glu Glu Leu Lys Gly Cys Ala Gln Tyr Met Thr Arg
                245                 250                 255

Leu Trp Asn Glu Arg Val Asn Val Pro Pro Gly Asn Asp Leu Ile Ser
            260                 265                 270

Met Met Ala His Thr Glu Ser Met Arg Asn Met Thr Pro Glu Glu Phe

275                 280                 285

Leu Gly Asn Leu Ile Leu Leu Ile Val Gly Gly Asn Asp Thr Thr Arg
    290                 295                 300

Asn Ser Met Thr Gly Gly Val Leu Ala Ile Asn Glu Asn Pro Asp Glu
305                 310                 315                 320

Tyr Arg Lys Leu Cys Ala Asn Pro Ala Leu Ile Ala Ser Met Val Pro
                325                 330                 335

Glu Ile Val Arg Trp Gln Thr Pro Leu Ser His Met Arg Arg Thr Ala
            340                 345                 350

Leu Gln Asp Thr Glu Leu Gly Gly Lys Ser Ile Arg Lys Gly Asp Lys
        355                 360                 365

Val Ile Met Trp Tyr Val Ser Gly Asn Arg Asp Pro Glu Ala Ile Glu
    370                 375                 380

Asn Pro Glu Ala Phe Ile Ile Asp Arg Ala Arg Pro Arg His His Leu
385                 390                 395                 400

Ser Phe Gly Phe Gly Ile His Arg Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460

<210> 22
<211> 465
<212> PRT
<213> Artificial Sequence

<220>
<223> Description of Artificial Sequence:hybrid protein

<400> 22
Met Ser Thr Ser Ser Ser Thr Ser Asn Asp Ile Gln Ala Lys Ile Ile
  1               5                  10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val His Arg Ala Ala Val Gln
145                 150                 155                 160

Pro Val Val Ala Pro Ala Asn Leu Ala Lys Leu Glu Pro Ile Ile Arg
                165                 170                 175

Glu Arg Ala Gly Arg Ile Leu Asp Glu Leu Pro Ile Gly Glu Thr Leu
            180                 185                 190

Asn Trp Val Asp Arg Val Ser Ile Glu Leu Thr Thr Gln Met Leu Ala
        195                 200                 205

Thr Leu Phe Asp Phe Pro Phe Glu Gln Arg Arg Lys Leu Thr Phe Trp
    210                 215                 220

Ser Asp Ala Thr Thr Gly Ala Pro Asp Ile Ser Gly Gly Asp Phe Ile
225                 230                 235                 240

Thr Pro Glu Glu Arg Asn Lys Ala Leu Ala Glu Cys Gly Glu Thr Phe
                245                 250                 255

Thr Arg Leu Trp Tyr Glu Arg Val Asn Gly Pro Pro Gly Asn Asp Leu
            260                 265                 270

Ile Ser Met Leu Ala His Ser Glu Arg Thr Arg Asn Ile Ile Asp Ser
        275                 280                 285

Pro Met Glu Tyr Leu Gly Asn Ile Leu Leu Leu Ile Val Gly Gly Asn
    290                 295                 300

Asp Thr Thr Arg Asn Ser Ile Ser Gly Gly Val Leu Ala Leu Asn Gln
305                 310                 315                 320

Asn Pro Gly Glu Tyr Glu Lys Leu Arg Gln Asn Pro Gly Leu Ile Pro
                325                 330                 335

Asn Met Val Ser Glu Ile Ile Arg Trp Gln Thr Pro Ile Leu Ala Met
            340                 345                 350

Arg Arg Arg Ala Lys Glu Asp Thr Glu Leu Phe Gly Lys Pro Ile Arg
        355                 360                 365

Lys Asn Asp Lys Val Val Met Trp Tyr Val Ser Gly Asn Arg Asp Asp
    370                 375                 380

Glu Val Ile Asp Asp Pro Met Ser Phe Arg Ile Asp Arg Glu Asn Ala
385                 390                 395                 400

Arg His His Leu Ser Phe Gly Phe Gly Ile His Arg Cys Met Gly Asn
                405                 410                 415

Arg Leu Ala Glu Leu Gln Leu Arg Ile Leu Trp Glu Glu Leu Leu Pro
            420                 425                 430

Arg Leu Glu Asn Ile Glu Val Ile Gly Glu Pro Glu Arg Val Gln Ser
        435                 440                 445

Asn Phe Val Arg Gly Tyr Ser Lys Met Met Val Lys Leu Thr Ala Lys
    450                 455                 460

Lys
465

<210> 23
<211> 460
<212> PRT
<213> Artificial Sequence

<220>
<223> Description of Artificial Sequence:hybrid protein

<400> 23
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Ala Gln Arg Lys Val Val Ser
145                 150                 155                 160

Pro Val Val Ala Pro Asn Asn Leu Glu Thr Leu Glu Pro Ile Ile Arg
                165                 170                 175

Glu Arg Ala Ala Ala Ile Leu Asp Ser Leu Pro Val Gly Glu Glu Ile
            180                 185                 190

Asp Trp Val Asp Lys Val Ser Ile Glu Leu Thr Thr Met Thr Leu Ala
        195                 200                 205

Thr Leu Phe Asp Phe Pro Trp Glu Glu Arg Arg Lys Leu Thr Arg Trp
    210                 215                 220

Ser Asp Val Leu Thr Ala Ser Pro Gly Asn Gly Val Asp Ser Trp Glu
225                 230                 235                 240

Gln Trp Gln Gln Glu Leu Thr Glu Cys Gly Met Tyr Phe Lys Asn Leu
                245                 250                 255

Trp Asp Gln Arg Val Gly Gly Glu Gln Lys His Asp Leu Ile Ser Met
            260                 265                 270

Leu Ala Gly Ser Pro Ala Thr Pro Asn Met Pro Phe Gln Glu Phe Leu
        275                 280                 285

Gly Asn Leu Met Leu Leu Ile Val Gly Gly Asn Asp Thr Thr Arg Asn
    290                 295                 300

Ser Ile Ser Gly Gly Val Leu Ala Leu Asn Gln Asn Pro Lys Glu Tyr
305                 310                 315                 320

Ala Lys Leu Met Asn Asp Pro Gly Leu Ile Pro Gly Met Val Ser Glu
                325                 330                 335

Ile Ile Arg Trp Gln Ser Pro Ile Thr Ser Met Met Arg Thr Ala Leu
            340                 345                 350

Cys Asp Ala Glu Val Gly Gly Lys Lys Ile Arg Glu Gly Asp Lys Val
        355                 360                 365

Val Met Trp Tyr Leu Ser Gly Asn Arg Asp Ser Glu Val Ile Glu Arg
    370                 375                 380

Ala Asp Glu Phe Leu Ile Asp Arg Lys Asn Pro Arg Gln His Leu Ser
385                 390                 395                 400

Phe Gly Phe Gly Ile His Arg Cys Met Gly Asn Arg Leu Ala Glu Leu
                405                 410                 415

Gln Leu Arg Ile Leu Trp Glu Glu Leu Leu Pro Arg Phe Glu Asn Ile
            420                 425                 430

Glu Val Ile Gly Glu Pro Glu Arg Val Gln Ser Asn Phe Val Arg Gly
        435                 440                 445

Tyr Ser Lys Met Met Val Lys Leu Thr Ala Lys Lys
    450                 455                 460

<210> 24
<211> 461
<212> PRT
<213> Artificial Sequence

<220>
<223> Description of Artificial Sequence:hybrid protein

<400> 24
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Ala Gln Arg Lys Ala Val Ser
145                 150                 155                 160

Pro Ala Val Ala Pro Ser Asn Leu Val Leu Leu Glu Pro Ile Ile Arg
                165                 170                 175

Glu Arg Ala Gly Leu Ile Leu Asp Ser Leu Pro Val Gly Glu Glu Ile
            180                 185                 190

Asp Trp Ile Asp Arg Val Ser Ile Glu Leu Thr Thr Met Thr Leu Ala
        195                 200                 205

Thr Leu Phe Asp Phe Pro Trp Glu Glu Arg Arg Lys Leu Thr Arg Trp
    210                 215                 220

Ser Asp Val Ala Thr Ala Thr Pro Glu Thr Gly Val Val Ser Ser Phe
225                 230                 235                 240

Glu Gln Arg Arg Glu Glu Leu Leu Glu Cys Ala Gln Tyr Phe Lys Gly

                245                 250                 255

Leu Trp Asp Lys Arg Ala Asn Glu Pro Pro Lys Pro Asp Leu Ile Ser
            260                 265                 270

Met Met Val His Ser Pro Ala Thr Arg Asp Met Pro Tyr Leu Glu Phe
        275                 280                 285

Leu Gly Asn Leu Ile Leu Leu Ile Val Gly Gly Asn Asp Thr Thr Arg
    290                 295                 300

Asn Ser Ile Ser Gly Gly Val Leu Ala Leu Asn Gln Asn Pro Ala Glu
305                 310                 315                 320

Tyr Arg Lys Leu Met Ala Asp Pro Gly Leu Ile Pro Lys Met Ile Pro
                325                 330                 335

Glu Ile Ile Arg Trp Gln Thr Pro Leu Thr His Met Arg Arg Thr Ala
            340                 345                 350

Leu Met Asp Ala Glu Ile Gly Gly Lys Lys Ile Arg Lys Gly Asp Lys
        355                 360                 365

Val Val Met Trp Tyr Leu Ser Gly Asn Arg Asp Asp Glu Val Ile Asp
    370                 375                 380

Arg Pro Asn Glu Phe Ile Ile Asp Arg Pro Asn Ser Arg His His Leu
385                 390                 395                 400

Ser Phe Gly Phe Gly Val His Arg Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460

<210> 25
<211> 461
<212> PRT
<213> Artificial Sequence

<220>

<223> Description of Artificial Sequence:hybrid protein

<400> 25
Met Ser Thr Ser Ser Ser Thr Ser Asn Asp Ile Gln Ala Lys Ile Ile
1               5                   10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Cys Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Pro Ser Val Glu Met Phe
    130                 135                 140

Ile Ala Met Asp Pro Pro Lys His Asp Gln Gln Arg Lys Ala Val Thr
145                 150                 155                 160

Pro Ala Val Ala Pro Ser Asn Leu Leu Leu Leu Glu Pro Thr Ile Arg
                165                 170                 175

Glu Arg Ala Cys Gln Ile Leu Asp Asp Leu Pro Ile Gly Glu Glu Phe
            180                 185                 190

Asp Trp Val Asp Lys Val Ser Val Glu Leu Thr Thr Met Thr Leu Ala
        195                 200                 205

Thr Leu Phe Asp Phe Pro Trp Glu Glu Arg Arg Lys Leu Thr Arg Trp
    210                 215                 220

Ser Asp Val Thr Thr Ala Ala Pro Glu Thr Gly Ile Val Glu Ser Tyr
225                 230                 235                 240

Asp Ala Arg Arg Glu Glu Leu Ile Glu Cys Ala Met Tyr Phe Lys Gly
                245                 250                 255

Leu Trp Glu Gln Arg Ile Asn Glu Glu Pro Lys Asn Asp Leu Ile Ser
            260                 265                 270

Ile Met Ala His Ser Pro Ala Thr Arg Asp Met Pro Phe Leu Glu Phe
        275                 280                 285

Leu Gly Asn Leu Leu Leu Leu Ile Val Gly Gly Asn Asp Thr Thr Arg
    290                 295                 300

Asn Ser Ile Ser Gly Gly Val Leu Ala Leu Asn Gln Asn Pro Asp Glu
305                 310                 315                 320

Tyr Arg Lys Leu Asn Asp Asp Pro Ser Leu Ile Ala Ser Met Val Pro
                325                 330                 335

Glu Ile Ile Arg Trp Gln Thr Pro Leu Thr His Met Arg Arg Thr Ala
            340                 345                 350

Leu Gln Asp Trp Glu Ile Gly Gly Lys Gln Ile Lys Lys Gly Asp Lys
        355                 360                 365

Val Val Met Trp Tyr Leu Ser Gly Asn Arg Asp Asp Thr Val Ile Glu
    370                 375                 380

Gln Ala Asp Arg Phe Ile Ile Asp Arg Lys Asn Pro Arg His His Leu
385                 390                 395                 400

Ser Phe Gly Tyr Gly Ile His Arg Cys Met Gly Asn Arg Leu Ala Glu
                405                 410                 415

Leu Gln Leu Arg Ile Leu Trp Glu Glu Leu Leu Pro Arg Phe Glu Asn
            420                 425                 430

Ile Glu Val Ile Gly Glu Pro Glu Arg Val Gln Ser Asn Phe Val Arg
        435                 440                 445

Gly Tyr Ser Lys Met Met Val Lys Leu Thr Ala Lys Lys
    450                 455                 460

<210> 26
<211> 1413
<212> DNA
<213> Artificial Sequence

<220>
<223> Description of Artificial Sequence:hybrid gene

<220>
<221> CDS
<222> (1)..(1410)

<400> 26
atg tca acg agt tca agt aca agt aat gac atc cag gca aaa ata att   48
Met Ser Thr Ser Ser Ser Thr Ser Asn Asp Ile Gln Ala Lys Ile Ile
 1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
        35                  40                  45

cag atg cac ttt gtt gaa acc gat ttg cct gac gtc aat gat ttg gcg   192
Gln Met His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

att gcc atg gac ccg ccg cag cat gat gag cag cga aac gcc gta cag   480
Ile Ala Met Asp Pro Pro Gln His Asp Glu Gln Arg Asn Ala Val Gln
145                 150                 155                 160

gga gtc gtg gcg ccg aag aac ctc aag gag atg gag agc ctg atc cgc 528
Gly Val Val Ala Pro Lys Asn Leu Lys Glu Met Glu Ser Leu Ile Arg
                165                 170                 175

tct cgc gtc cgc gag gtg ctc gac gga ctt ccg gtt ggc gag ccg ttc 576
Ser Arg Val Arg Glu Val Leu Asp Gly Leu Pro Val Gly Glu Pro Phe
            180                 185                 190

aac tgg gtt gac aag gtc tcg att gaa ctg acc ggt cgc atg ctg gcg 624
Asn Trp Val Asp Lys Val Ser Ile Glu Leu Thr Gly Arg Met Leu Ala
        195                 200                 205

acc ctg ctg gac tac ctg tac gag gat cgt cga gag ctc acg gag att 672
Thr Leu Leu Asp Tyr Leu Tyr Glu Asp Arg Arg Glu Leu Thr Glu Ile
    210                 215                 220

tcg gac ctg gca tcg ggt gtg ccg ggt gcg acc ggt ggc gag tcc gac 720
Ser Asp Leu Ala Ser Gly Val Pro Gly Ala Thr Gly Gly Glu Ser Asp
225                 230                 235                 240

ctc gat gag acg ttc tcc ctg gcg gtt ccg cta ctg acg aag ttc ctg 768
Leu Asp Glu Thr Phe Ser Leu Ala Val Pro Leu Leu Thr Lys Phe Leu
                245                 250                 255

cgc ctg tgg aac gag aag aag gcg cgt ttc gaa gcc ggc cag ccg ctg 816
Arg Leu Trp Asn Glu Lys Lys Ala Arg Phe Glu Ala Gly Gln Pro Leu
            260                 265                 270

ggc ttc gat ctg atc agc ctc atg ctg gcc gac ccg gcg acg cgg aac 864
Gly Phe Asp Leu Ile Ser Leu Met Leu Ala Asp Pro Ala Thr Arg Asn
        275                 280                 285

ctc atc gat gaa cgc ccg atg gag ttc ctc gga aac gcg gtg ctg ctg 912
Leu Ile Asp Glu Arg Pro Met Glu Phe Leu Gly Asn Ala Val Leu Leu
    290                 295                 300

atc gtc ggt ggc aac gac acg acc cgt aac tcg atg tcg ggt ggc gtg 960
Ile Val Gly Gly Asn Asp Thr Thr Arg Asn Ser Met Ser Gly Gly Val
305                 310                 315                 320

tac gcg ctg aac aag tgg ccg gat cag ttc gcc aag ctc aag gac aac 1008
Tyr Ala Leu Asn Lys Trp Pro Asp Gln Phe Ala Lys Leu Lys Asp Asn
                325                 330                 335

ccg aag ctg atc ccg aac atg gtt tcg gag atc atc cgc tgg cag acg 1056
Pro Lys Leu Ile Pro Asn Met Val Ser Glu Ile Ile Arg Trp Gln Thr
            340                 345                 350

ccg ctc gcc tac atg gcg cgt cgg gcc aag gaa gac acc gtt gtc ggt 1104
Pro Leu Ala Tyr Met Ala Arg Arg Ala Lys Glu Asp Thr Val Val Gly
        355                 360                 365

ggt cag gag atc aag aag ggc gag aag ctc atc atg tgg tac tcc tcg 1152
Gly Gln Glu Ile Lys Lys Gly Glu Lys Leu Ile Met Trp Tyr Ser Ser
    370                 375                 380

gcc aac cgg gac gag cgc cac ttc gag aac ccg gaa cag ctg atc atc 1200
Ala Asn Arg Asp Glu Arg His Phe Glu Asn Pro Glu Gln Leu Ile Ile
385                 390                 395                 400

gac cgc aag aat gcg cgt aac cac att gcg ttc ggc ttc ggc att cac 1248
Asp Arg Lys Asn Ala Arg Asn His Ile Ala Phe Gly Phe Gly Ile His
                405                 410                 415

cgc tgc atg ggg aac agg ttt gcc gaa ctg cag ctg cgt att ctg tgg 1296
Arg Cys Met Gly Asn Arg Phe Ala Glu Leu Gln Leu Arg Ile Leu Trp
            420                 425                 430

gaa gag ctt ctc cct cgc ttt gaa aac atc gaa gtg atc ggt gag ccg 1344
Glu Glu Leu Leu Pro Arg Phe Glu Asn Ile Glu Val Ile Gly Glu Pro
        435                 440                 445

gag cgc gtg caa tcg aac ttt gtg cgg ggc tat tcc aag atg atg gtt 1392
Glu Arg Val Gln Ser Asn Phe Val Arg Gly Tyr Ser Lys Met Met Val
    450                 455                 460

aag ttg acg gct aaa aaa taa 1413
Lys Leu Thr Ala Lys Lys
465                 470

<210> 27
<211> 1413
<212> DNA
<213> Artificial Sequence

<220>
<223> Description of Artificial Sequence:hybrid gene

<220>
<221> CDS
<222> (1)..(1410)

<400> 27
atg tca acg agt tca agt aca agt aat gac atc cag gca aaa ata att 48
Met Ser Thr Ser Ser Ser Thr Ser Asn Asp Ile Gln Ala Lys Ile Ile

1               5                   10                  15

aac gcc aca tcc aaa gtc gtg cca atg cat cta cag atc aag gca cta   96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

att gct atg gac ccc ccc aaa cac gat gtg cag cgt cgc tcg gtt cag   480
Ile Ala Met Asp Pro Pro Lys His Asp Val Gln Arg Arg Ser Val Gln
145                 150                 155                 160

ggc gtg gtg gca ccg caa aac ctg aag gaa atg gag ggc cta atc cgt   528
Gly Val Val Ala Pro Gln Asn Leu Lys Glu Met Glu Gly Leu Ile Arg
                165                 170                 175

tct cgt acc ggg gaa gtg ctc gat ggc ctg ccc ctg ggc gaa gcc ttt   576
Ser Arg Thr Gly Glu Val Leu Asp Gly Leu Pro Leu Gly Glu Ala Phe
            180                 185                 190

aac tgg gtg ccg gcc gta tcc atc gag ctg acg gga cgc atg ctg gcc   624
Asn Trp Val Pro Ala Val Ser Ile Glu Leu Thr Gly Arg Met Leu Ala

195                 200                 205

act ctg ctg gat ttt cct tac gaa gag cgc cac aag ctg acg tac tgg   672
Thr Leu Leu Asp Phe Pro Tyr Glu Glu Arg His Lys Leu Thr Tyr Trp
    210                 215                 220

tcg gac cgg ctg gcc aac acg gcg gag acg acc ggc ggc gag ttt gac   720
Ser Asp Arg Leu Ala Asn Thr Ala Glu Thr Thr Gly Gly Glu Phe Asp
225                 230                 235                 240

gac gac gat gga ctg ttt gac acc gcc gcc gac atg gcc cgg gcc ttc   768
Asp Asp Asp Gly Leu Phe Asp Thr Ala Ala Asp Met Ala Arg Ala Phe
                245                 250                 255

tcc gaa ctg tgg cgc gac aag cag gcg cgc cgg gcg gcg ggg gag gcg   816
Ser Glu Leu Trp Arg Asp Lys Gln Ala Arg Arg Ala Ala Gly Glu Ala
            260                 265                 270

ccc agc ttt gat ctg atc agc ctg atg cag agc agc gaa gac acc gca   864
Pro Ser Phe Asp Leu Ile Ser Leu Met Gln Ser Ser Glu Asp Thr Ala
        275                 280                 285

gat atg atc aag cgg ccg atg gaa ttc ctc ggc aat ctg gcg ctg ctc   912
Asp Met Ile Lys Arg Pro Met Glu Phe Leu Gly Asn Leu Ala Leu Leu
    290                 295                 300

atc atc ggc ggc aat gac acc acg cga aat tcc atg agc ggc ggc gtg   960
Ile Ile Gly Gly Asn Asp Thr Thr Arg Asn Ser Met Ser Gly Gly Val
305                 310                 315                 320

ctg gct ttg aac cag ttc ccg aag gaa ttc gcc aag ctc aaa gcc aat   1008
Leu Ala Leu Asn Gln Phe Pro Lys Glu Phe Ala Lys Leu Lys Ala Asn
                325                 330                 335

ccg gag ctg att ccg aac atg gtt tcg gaa atc atc cgc tgg cag acg   1056
Pro Glu Leu Ile Pro Asn Met Val Ser Glu Ile Ile Arg Trp Gln Thr
            340                 345                 350

ccg ctc gcg cat atg cgc cgg atc gcc acg cag gac gtg gaa ctg tgc   1104
Pro Leu Ala His Met Arg Arg Ile Ala Thr Gln Asp Val Glu Leu Cys
        355                 360                 365

ggt cag acc atc aag aag ggc gat cgc gtc ttg atg tgg tac gcc tcg   1152
Gly Gln Thr Ile Lys Lys Gly Asp Arg Val Leu Met Trp Tyr Ala Ser
    370                 375                 380

ggc aac cgg gac gaa cgc aag ttc gaa aat ccc gat gac ttc atc atc   1200
Gly Asn Arg Asp Glu Arg Lys Phe Glu Asn Pro Asp Asp Phe Ile Ile

385                 390                 395                 400

gat cgc gag ggc gcg cgg cac cac atg tcg ttt ggc tac ggc atc cac   1248
Asp Arg Glu Gly Ala Arg His His Met Ser Phe Gly Tyr Gly Ile His
                405                 410                 415

cgc tgc atg ggt aac cgc ctt gcc gaa ctg cag ctg cgt att ctg tgg   1296
Arg Cys Met Gly Asn Arg Leu Ala Glu Leu Gln Leu Arg Ile Leu Trp
            420                 425                 430

gaa gag ctt ctc cct cgc ttt gaa aac atc gaa gtg atc ggt gag ccg   1344
Glu Glu Leu Leu Pro Arg Phe Glu Asn Ile Glu Val Ile Gly Glu Pro
        435                 440                 445

gag cgc gtg caa tcg aac ttt gtg cgg ggc tat tcc aag atg atg gtt   1392
Glu Arg Val Gln Ser Asn Phe Val Arg Gly Tyr Ser Lys Met Met Val
    450                 455                 460

aag ttg acg gct aaa aaa taa                                       1413
Lys Leu Thr Ala Lys Lys
465                 470

<210> 28
<211> 1413
<212> DNA
<213> Artificial Sequence

<220>
<223> Description of Artificial Sequence:hybrid gene

<220>
<221> CDS
<222> (1)..(1410)

<400> 28
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

atc gct atg gac ccc ccc aag cac gac gtg cag cgc cgc tcg gtg cag   480
Ile Ala Met Asp Pro Pro Lys His Asp Val Gln Arg Arg Ser Val Gln
145                 150                 155                 160

aac gtg gtg gca ccg cag aac ctg aag gag atg gaa ggg ttg atc cgc   528
Asn Val Val Ala Pro Gln Asn Leu Lys Glu Met Glu Gly Leu Ile Arg
                165                 170                 175

acg cgc acc gcg gag gtg ctc gaa agc ctg ccc cgg aac gaa gtc ttc   576
Thr Arg Thr Ala Glu Val Leu Glu Ser Leu Pro Arg Asn Glu Val Phe
            180                 185                 190

aac tgg gtg ccg gcg gta tcg aaa gag ctg acg ggc cga atg ctg gcc   624
Asn Trp Val Pro Ala Val Ser Lys Glu Leu Thr Gly Arg Met Leu Ala
        195                 200                 205

acg ctg ctg gat ttt cct tat gag gag cgt cac aag ctg atc gaa tgg   672
Thr Leu Leu Asp Phe Pro Tyr Glu Glu Arg His Lys Leu Ile Glu Trp
    210                 215                 220

tcg gat cag atg gcc ggc acg gcc gca gcc acc ggc ggc gag ttc gag   720
Ser Asp Gln Met Ala Gly Thr Ala Ala Ala Thr Gly Gly Glu Phe Glu
225                 230                 235                 240

gat gaa gag agc atg ttc gag gcc gcc gcg gat atg gct tgg tcc ttc 768
Asp Glu Glu Ser Met Phe Glu Ala Ala Ala Asp Met Ala Trp Ser Phe
                245                 250                 255

tcc aga ctg tgg cgc gac aag aag gcc cgt cgc gcg gcg ggt gaa gca 816
Ser Arg Leu Trp Arg Asp Lys Lys Ala Arg Arg Ala Ala Gly Glu Ala
            260                 265                 270

ccc ggg ttt gat ttg atc agc ctg ctg cag agc ggt gaa gac acg cag 864
Pro Gly Phe Asp Leu Ile Ser Leu Leu Gln Ser Gly Glu Asp Thr Gln
        275                 280                 285

gat ctg att aac cgt ccg atg gag ttc atc ggc aat ctc gcg ctg ctc 912
Asp Leu Ile Asn Arg Pro Met Glu Phe Ile Gly Asn Leu Ala Leu Leu
    290                 295                 300

atc gtg gga ggc aat gac acc acg cgc aac tcc atg agc ggc ggc gtg 960
Ile Val Gly Gly Asn Asp Thr Thr Arg Asn Ser Met Ser Gly Gly Val
305                 310                 315                 320

ctg gcc atg aac caa ttc ccc gcg gaa ttc gcc aaa ctg aag gcg aat 1008
Leu Ala Met Asn Gln Phe Pro Ala Glu Phe Ala Lys Leu Lys Ala Asn
                325                 330                 335

ccg aag ttg ctg ccg aac atg gtc tcg gaa atc atc cgc tgg cag acc 1056
Pro Lys Leu Leu Pro Asn Met Val Ser Glu Ile Ile Arg Trp Gln Thr
            340                 345                 350

ccg ctg gcg cac atg cga cgg gtc gcc acg cag gac gta gag cta cgc 1104
Pro Leu Ala His Met Arg Arg Val Ala Thr Gln Asp Val Glu Leu Arg
        355                 360                 365

ggt cag act atc aaa aag ggg gat cgc gtg ctg atg tgg tac gcc tcg 1152
Gly Gln Thr Ile Lys Lys Gly Asp Arg Val Leu Met Trp Tyr Ala Ser
    370                 375                 380

ggc aat cgg gac gaa cgc aag ttc gaa aag ccc gat gac ttc atc atc 1200
Gly Asn Arg Asp Glu Arg Lys Phe Glu Lys Pro Asp Asp Phe Ile Ile
385                 390                 395                 400

gat cgt gag ggc gcg cgg aac cat atc gcg ttc ggc tac ggc atc cac 1248
Asp Arg Glu Gly Ala Arg Asn His Ile Ala Phe Gly Tyr Gly Ile His
                405                 410                 415

cgg tgc atg ggt aat agg ctt gcc gaa ctg cag ctg cgt att ctg tgg 1296
Arg Cys Met Gly Asn Arg Leu Ala Glu Leu Gln Leu Arg Ile Leu Trp
            420                 425                 430

gaa gag ctt ctc cct cgc ttt gaa aac atc gaa gtg atc ggt gag ccg   1344
Glu Glu Leu Leu Pro Arg Phe Glu Asn Ile Glu Val Ile Gly Glu Pro
        435                 440                 445

gag cgc gtg caa tcg aac ttt gtg cgg ggc tat tcc aag atg atg gtt   1392
Glu Arg Val Gln Ser Asn Phe Val Arg Gly Tyr Ser Lys Met Met Val
    450                 455                 460

aag ttg acg gct aaa aaa taa                                       1413
Lys Leu Thr Ala Lys Lys
465                 470

<210> 29
<211> 1413
<212> DNA
<213> Artificial Sequence

<220>
<223> Description of Artificial Sequence:hybrid gene

<220>
<221> CDS
<222> (1)..(1410)

<400> 29
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr

                85                  90                  95

cag aag aac agt gct ttc ggg ccg ttc tgg tcg gta aca cgc tac gaa 336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc 384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc 432
Gln Ile Ile Leu Gly Asp Pro Pro Glu Gly Leu Ser Val Glu Met Phe
    130                 135                 140

atc gcg atg gac cct ccc aaa cat gat gta cag cgc cga gcg gtg cag 480
Ile Ala Met Asp Pro Pro Lys His Asp Val Gln Arg Arg Ala Val Gln
145                 150                 155                 160

ggc gtc gtt gct ccg aaa aac ctt aaa gaa atg gaa ggg ctg att cgt 528
Gly Val Val Ala Pro Lys Asn Leu Lys Glu Met Glu Gly Leu Ile Arg
                165                 170                 175

tct cgt gcg gcg gaa gtg cta gac agc ttg cct ctg gat aaa cct ttc 576
Ser Arg Ala Ala Glu Val Leu Asp Ser Leu Pro Leu Asp Lys Pro Phe
            180                 185                 190

gat tgg gtg ccg gcg gtg tcc aaa gaa ctg act ggc cgt atg ctc gct 624
Asp Trp Val Pro Ala Val Ser Lys Glu Leu Thr Gly Arg Met Leu Ala
        195                 200                 205

acg ttg ctg gat ttc cct tat gag gat cgt cac aaa ctc gtt gaa tgg 672
Thr Leu Leu Asp Phe Pro Tyr Glu Asp Arg His Lys Leu Val Glu Trp
    210                 215                 220

tct gat cgt ttg tct ggc gcg gca tcc gcc acg ggt gga gaa ttt acc 720
Ser Asp Arg Leu Ser Gly Ala Ala Ser Ala Thr Gly Gly Glu Phe Thr
225                 230                 235                 240

gac gaa gat gtc atg ttt gat gac gcg gca gat atg gcg agg gcg ttc 768
Asp Glu Asp Val Met Phe Asp Asp Ala Ala Asp Met Ala Arg Ala Phe
                245                 250                 255

tcc agg ctg tgg cga gac aag gag gcg cga cgt gct tcc ggc gaa gaa 816
Ser Arg Leu Trp Arg Asp Lys Glu Ala Arg Arg Ala Ser Gly Glu Glu
            260                 265                 270

ccc ggt ttc gat ttg atc agt atg ctg cag agc aac gac gat aca aaa 864
Pro Gly Phe Asp Leu Ile Ser Met Leu Gln Ser Asn Asp Asp Thr Lys

        275                 280                 285

gac ctt atc aat cga ccg atg gag ttt atc ggg aat ctg gct ctg ctt 912
Asp Leu Ile Asn Arg Pro Met Glu Phe Ile Gly Asn Leu Ala Leu Leu
    290                 295                 300

atc gtt ggc ggc aat gac acc aca agg aat tcc atg agt ggt ggt gtt 960
Ile Val Gly Gly Asn Asp Thr Thr Arg Asn Ser Met Ser Gly Gly Val
305                 310                 315                 320

ttg gcc ttg aat caa ttc ccc gag gaa ttc tcc aag ctg aag gcg aat 1008
Leu Ala Leu Asn Gln Phe Pro Glu Glu Phe Ser Lys Leu Lys Ala Asn
                325                 330                 335

ccg gaa ctg att cct aac atg gtt tcg gaa atc att cgt tgg caa act 1056
Pro Glu Leu Ile Pro Asn Met Val Ser Glu Ile Ile Arg Trp Gln Thr
            340                 345                 350

cca ctg gcc aac atg cgt agg gtg gcg aca cag gat gta gaa ctt cgt 1104
Pro Leu Ala Asn Met Arg Arg Val Ala Thr Gln Asp Val Glu Leu Arg
        355                 360                 365

ggc cag acc atc aag aaa ggc gac cgg gtg tta atg tgg tat gcc tcg 1152
Gly Gln Thr Ile Lys Lys Gly Asp Arg Val Leu Met Trp Tyr Ala Ser
    370                 375                 380

ggt aat cgg gac gaa cgc aag ttt gat aac ccc gac caa ctc att att 1200
Gly Asn Arg Asp Glu Arg Lys Phe Asp Asn Pro Asp Gln Leu Ile Ile
385                 390                 395                 400

gat cgc aag gac gca cgg aac cat att tct ttt ggc tac ggt att cat 1248
Asp Arg Lys Asp Ala Arg Asn His Ile Ser Phe Gly Tyr Gly Ile His
                405                 410                 415

cgg tgc atg ggg aac agc ctt gcc gaa ctg cag ctg cgt att ctg tgg 1296
Arg Cys Met Gly Asn Ser Leu Ala Glu Leu Gln Leu Arg Ile Leu Trp
            420                 425                 430

gaa gag ctt ctc cct cgc ttt gaa aac atc gaa gtg atc ggt gag ccg 1344
Glu Glu Leu Leu Pro Arg Phe Glu Asn Ile Glu Val Ile Gly Glu Pro
        435                 440                 445

gag cgc gtg caa tcg aac ttt gtg cgg ggc tat tcc aag atg atg gtt 1392
Glu Arg Val Gln Ser Asn Phe Val Arg Gly Tyr Ser Lys Met Met Val
    450                 455                 460

aag ttg acg gct aaa aaa taa 1413
Lys Leu Thr Ala Lys Lys

465                 470

<210> 30
<211> 1413
<212> DNA
<213> Artificial Sequence

<220>
<223> Description of Artificial Sequence:hybrid gene

<220>
<221> CDS
<222> (1)..(1410)

<400> 30
atg tca acg agt tca agt aca agt aat gac atc cag gca aaa ata att    48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta    96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct    144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg    192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag    240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat    288
Ala Asn Ala Tyr Phe Lys Arg Leu Arg Asp Glu Ala Pro Val His Tyr
                 85                  90                  95

cag aag aac agt gct ttc ggg ccg ttc tgg tcg gta aca cgc tac gaa    336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc    384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

atc gcg atg gac ccg ccc aaa cat gat gta cag cgc cga gcg gtg cag   480
Ile Ala Met Asp Pro Pro Lys His Asp Val Gln Arg Arg Ala Val Gln
145                 150                 155                 160

ggc gtc gtt gct ccg aaa aac ctt aaa gaa atg gaa ggg ctg att cgt   528
Gly Val Val Ala Pro Lys Asn Leu Lys Glu Met Glu Gly Leu Ile Arg
                165                 170                 175

tct cgt gcg gcg gaa gtg cta gac agc ttg cct ctg gat aaa cct ttc   576
Ser Arg Ala Ala Glu Val Leu Asp Ser Leu Pro Leu Asp Lys Pro Phe
            180                 185                 190

gat tgg gtg ccg gcg gtg tcc aaa gaa ctg act ggc cgt atg ctc gct   624
Asp Trp Val Pro Ala Val Ser Lys Glu Leu Thr Gly Arg Met Leu Ala
        195                 200                 205

acg ttg ctg gat ttc cct tat gag gat cgt cac aaa ctc gtt gaa tgg   672
Thr Leu Leu Asp Phe Pro Tyr Glu Asp Arg His Lys Leu Val Glu Trp
    210                 215                 220

tct gat cgt ttg tct ggc gcg gca tcc gcc acg ggt gga gaa ttt acc   720
Ser Asp Arg Leu Ser Gly Ala Ala Ser Ala Thr Gly Gly Glu Phe Thr
225                 230                 235                 240

gac gaa gat gtc atg ttt gat gac gcg gca gat atg gcg agg gcg ttc   768
Asp Glu Asp Val Met Phe Asp Asp Ala Ala Asp Met Ala Arg Ala Phe
                245                 250                 255

tcc agg ctg tgg cga gac aag gag gcg cga cgt gct tcc ggc gaa gaa   816
Ser Arg Leu Trp Arg Asp Lys Glu Ala Arg Arg Ala Ser Gly Glu Glu
            260                 265                 270

ccc ggt ttc gat ttg atc agt atg ctg cag agc aac gaa gat aca aaa   864
Pro Gly Phe Asp Leu Ile Ser Met Leu Gln Ser Asn Glu Asp Thr Lys
        275                 280                 285

gac ctg atc aat cgg ccg atg gag ttt atc ggg aat ctg gcg ctg ctt   912
Asp Leu Ile Asn Arg Pro Met Glu Phe Ile Gly Asn Leu Ala Leu Leu
    290                 295                 300

att gtt ggt gga aat gat acc act cgc aac tcg atg agt ggt ggc gtg   960
Ile Val Gly Gly Asn Asp Thr Thr Arg Asn Ser Met Ser Gly Gly Val
305                 310                 315                 320

ctg gct ttg aat cag ttt ccc gag gaa ttt aga aaa ctg aag gca aaa   1008
Leu Ala Leu Asn Gln Phe Pro Glu Glu Phe Arg Lys Leu Lys Ala Lys
            325                 330                 335

ccg gag ttg att ccg aat atg gtt tcg gaa atc atc cgt tgg caa acc   1056
Pro Glu Leu Ile Pro Asn Met Val Ser Glu Ile Ile Arg Trp Gln Thr
        340                 345                 350

ccg ttg gcg aat atg cgc agg gtt gct acc cag gat gtt gag ctt cgt   1104
Pro Leu Ala Asn Met Arg Arg Val Ala Thr Gln Asp Val Glu Leu Arg
    355                 360                 365

ggt cag acc atc aag aaa ggg gac cgg gtg tta atg tgg tat gcc tcg   1152
Gly Gln Thr Ile Lys Lys Gly Asp Arg Val Leu Met Trp Tyr Ala Ser
  370                 375                 380

gga aac cgg gac gaa cgc aag ttc gaa aac cct gat caa ctt att att   1200
Gly Asn Arg Asp Glu Arg Lys Phe Glu Asn Pro Asp Gln Leu Ile Ile
385                 390                 395                 400

gat cgc aag gac gcg cga aac cat atc tct ttt ggt tac ggc att cac   1248
Asp Arg Lys Asp Ala Arg Asn His Ile Ser Phe Gly Tyr Gly Ile His
                405                 410                 415

cga tgc atg ggt aac cgc ctt gcc gaa ctg cag ctg cgt att ctg tgg   1296
Arg Cys Met Gly Asn Arg Leu Ala Glu Leu Gln Leu Arg Ile Leu Trp
            420                 425                 430

gaa gag ctt ctc cct cgc ttt gaa aac atc gaa gtg atc ggt gag ccg   1344
Glu Glu Leu Leu Pro Arg Phe Glu Asn Ile Glu Val Ile Gly Glu Pro
        435                 440                 445

gag cgc gtg caa tcg aac ttt gtg cgg ggc tat tcc aag atg atg gtt   1392
Glu Arg Val Gln Ser Asn Phe Val Arg Gly Tyr Ser Lys Met Met Val
    450                 455                 460

aag ttg acg gct aaa aaa taa                                       1413
Lys Leu Thr Ala Lys Lys
465                 470


<210> 31
<211> 1410
<212> DNA
<213> Artificial Sequence

<220>
<223> Description of Artificial Sequence:hybrid gene

<220>
<221> CDS
<222> (1)..(1407)

<400> 31
atg tca acg agt tca agt aca agt aat gac atc cag gca aaa ata att   48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta   96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

ata gcc atg gac ccg cca aaa cat gat gag caa cgc aag gcg gtc cag   480
Ile Ala Met Asp Pro Pro Lys His Asp Glu Gln Arg Lys Ala Val Gln
145                 150                 155                 160

aat gtg gtg gca ccg aag aat ctc aag gaa atg gaa tcg ctg atc cgt   528
Asn Val Val Ala Pro Lys Asn Leu Lys Glu Met Glu Ser Leu Ile Arg

                165                 170                 175

gag cga acc cgg gaa gtg ctg gac agt ctg ccc gtg gat caa cct ttc 576
Glu Arg Thr Arg Glu Val Leu Asp Ser Leu Pro Val Asp Gln Pro Phe
            180                 185                 190

gac tgg gta caa caa gtc tcc att gag ctg acg gct cgt atg ctg gcg 624
Asp Trp Val Gln Gln Val Ser Ile Glu Leu Thr Ala Arg Met Leu Ala
        195                 200                 205

acg ctg ctt gat ttt cct tat gag cag cgc cac aag ctg gtg tac tgg 672
Thr Leu Leu Asp Phe Pro Tyr Glu Gln Arg His Lys Leu Val Tyr Trp
    210                 215                 220

tca gat gta ccc act gcc agc ccg gaa acc acc ggt gac aag gtt ggc 720
Ser Asp Val Pro Thr Ala Ser Pro Glu Thr Thr Gly Asp Lys Val Gly
225                 230                 235                 240

ccg gat gag atc ttc gcg gcg gca gcg gat gcc ggg aag cac ttt gta 768
Pro Asp Glu Ile Phe Ala Ala Ala Ala Asp Ala Gly Lys His Phe Val
                245                 250                 255

agc ttg tgg cat gag aag gct gcc cga aaa gca gcg gga gaa gcg ccg 816
Ser Leu Trp His Glu Lys Ala Ala Arg Lys Ala Ala Gly Glu Ala Pro
            260                 265                 270

ggt ttt gat ctg atc agc ctg atg cag gcc aac gaa gaa acc aaa gac 864
Gly Phe Asp Leu Ile Ser Leu Met Gln Ala Asn Glu Glu Thr Lys Asp
        275                 280                 285

atg gtc aag cgg ccc atg gag ttc atg ggg aat ctg gtc ttg ctg atc 912
Met Val Lys Arg Pro Met Glu Phe Met Gly Asn Leu Val Leu Leu Ile
    290                 295                 300

gtg ggc ggt aat gac acc acg cgt aat tcc atg acg ggc ggg gtg ctg 960
Val Gly Gly Asn Asp Thr Thr Arg Asn Ser Met Thr Gly Gly Val Leu
305                 310                 315                 320

gcg cta aat cag ttc cct ggg gag ttt atc aag ctc aag gaa aac cct 1008
Ala Leu Asn Gln Phe Pro Gly Glu Phe Ile Lys Leu Lys Glu Asn Pro
                325                 330                 335

gac ctg att ccc aac atg gta tct gag att atc cgc tgg cag acg ccg 1056
Asp Leu Ile Pro Asn Met Val Ser Glu Ile Ile Arg Trp Gln Thr Pro
            340                 345                 350

tta gcg tat atg cgg cgc atc gcg aag cag gat gta gag ctc aac ggg 1104
Leu Ala Tyr Met Arg Arg Ile Ala Lys Gln Asp Val Glu Leu Asn Gly

        355                 360                 365

gag act att cgc aag ggc gac aag gtg gtg atg tgg tat gcc tcg ggt 1152
Glu Thr Ile Arg Lys Gly Asp Lys Val Val Met Trp Tyr Ala Ser Gly
    370                 375                 380

aat cgt gat gag cgc aag att gaa aac ccg gat gtc ttc cag atc gac 1200
Asn Arg Asp Glu Arg Lys Ile Glu Asn Pro Asp Val Phe Gln Ile Asp
385                 390                 395                 400

cgc aaa ggc gcg cgc aat cac ctt tcc ttt ggc ttt ggc gtc cac cgt 1248
Arg Lys Gly Ala Arg Asn His Leu Ser Phe Gly Phe Gly Val His Arg
                405                 410                 415

tgt atg ggt aac cgc ctt gcc gaa ctg cag ctg cgt att ctg tgg gaa 1296
Cys Met Gly Asn Arg Leu Ala Glu Leu Gln Leu Arg Ile Leu Trp Glu
            420                 425                 430

gag ctt ctc cct cgc ttt gaa aac atc gaa gtg atc ggt gag ccg gag 1344
Glu Leu Leu Pro Arg Phe Glu Asn Ile Glu Val Ile Gly Glu Pro Glu
        435                 440                 445

cgc gtg caa tcg aac ttt gtg cgg ggc tat tcc aag atg atg gtt aag 1392
Arg Val Gln Ser Asn Phe Val Arg Gly Tyr Ser Lys Met Met Val Lys
    450                 455                 460

ttg acg gct aaa aaa taa 1410
Leu Thr Ala Lys Lys
465

<210> 32
<211> 1410
<212> DNA
<213> Artificial Sequence

<220>
<223> Description of Artificial Sequence:hybrid gene

<220>
<221> CDS
<222> (1)..(1407)

<400> 32
atg tca acg agt tca agt aca agt aat gac atc cag gca aaa ata att 48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta   96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

atc gct atg gac cct cca aaa cat gat gag caa cgc aag gcg gtc cag   480
Ile Ala Met Asp Pro Pro Lys His Asp Glu Gln Arg Lys Ala Val Gln
145                 150                 155                 160

aat gtg gtg gca ccg aag aat ctc aag gaa atg gaa tcg ctg atc cgt   528
Asn Val Val Ala Pro Lys Asn Leu Lys Glu Met Glu Ser Leu Ile Arg
                165                 170                 175

gag cga acc cgg gaa gtg ctg gac agt ttg ccc gtg gat caa cct ttc   576
Glu Arg Thr Arg Glu Val Leu Asp Ser Leu Pro Val Asp Gln Pro Phe
            180                 185                 190

gac tgg gta caa caa gtc tcc att gaa ctg acg gct cgt atg ctg gcg   624
Asp Trp Val Gln Gln Val Ser Ile Glu Leu Thr Ala Arg Met Leu Ala
        195                 200                 205

acg ctg ctt gat ttt cct tat gag cag cgc cac aag ctg gtg tac tgg 672
Thr Leu Leu Asp Phe Pro Tyr Glu Gln Arg His Lys Leu Val Tyr Trp
    210                 215                 220

tca gat gta tcc act gcc agc ccg gaa acc acc ggt ggc aag gtt ggc 720
Ser Asp Val Ser Thr Ala Ser Pro Glu Thr Thr Gly Gly Lys Val Gly
225                 230                 235                 240

ccg gat gag atc ttc gcg gcg gca gcg gat gcc ggg aag cac ttt gtc 768
Pro Asp Glu Ile Phe Ala Ala Ala Ala Asp Ala Gly Lys His Phe Val
                245                 250                 255

agc ttg tgg cat gag aag gct gcc cga aaa gca gcg gga gaa gcg ccg 816
Ser Leu Trp His Glu Lys Ala Ala Arg Lys Ala Ala Gly Glu Ala Pro
            260                 265                 270

ggt ttt gat ctg atc agc ctg atg cag gcc aac gaa gaa acc aaa gac 864
Gly Phe Asp Leu Ile Ser Leu Met Gln Ala Asn Glu Glu Thr Lys Asp
        275                 280                 285

atg gtc aag cgg ccc atg gag ttc atg ggg aat ctg gtc ttg ctg atc 912
Met Val Lys Arg Pro Met Glu Phe Met Gly Asn Leu Val Leu Leu Ile
    290                 295                 300

gtg ggc ggt aat gac acc acg cgt aat tcc atg acg ggc ggg gtg ctg 960
Val Gly Gly Asn Asp Thr Thr Arg Asn Ser Met Thr Gly Gly Val Leu
305                 310                 315                 320

gcg cta aat cag ttc cct ggg gag ttt atc aag ctc aag gaa aac cct 1008
Ala Leu Asn Gln Phe Pro Gly Glu Phe Ile Lys Leu Lys Glu Asn Pro
                325                 330                 335

gac ctg att ccc aac atg gta tct gag att atc cgc tgg cag acg ccg 1056
Asp Leu Ile Pro Asn Met Val Ser Glu Ile Ile Arg Trp Gln Thr Pro
            340                 345                 350

ttg gcg tat atg cgg cgc atc gcg aag cag gat gta gag ctc aac ggg 1104
Leu Ala Tyr Met Arg Arg Ile Ala Lys Gln Asp Val Glu Leu Asn Gly
        355                 360                 365

gag act att cgc aag ggc gac aag gtg gtg atg tgg tat gcc tcg ggt 1152
Glu Thr Ile Arg Lys Gly Asp Lys Val Val Met Trp Tyr Ala Ser Gly
    370                 375                 380

aat cgt gat gag cgc aag att gaa aac ccg gat gtc ttc cag atc gac 1200
Asn Arg Asp Glu Arg Lys Ile Glu Asn Pro Asp Val Phe Gln Ile Asp
385                 390                 395                 400

cgc aaa ggc gcg cgc aat cac ctt tcc ttt ggc ttt ggc gtc cac cga 1248
Arg Lys Gly Ala Arg Asn His Leu Ser Phe Gly Phe Gly Val His Arg
                405                 410                 415

tgt atg gga aac agc ttt gcc gaa ctg cag ctg cgt att ctg tgg gaa 1296
Cys Met Gly Asn Ser Phe Ala Glu Leu Gln Leu Arg Ile Leu Trp Glu
            420                 425                 430

gag ctt ctc cct cgc ttt gaa aac atc gaa gtg atc ggt gag ccg gag 1344
Glu Leu Leu Pro Arg Phe Glu Asn Ile Glu Val Ile Gly Glu Pro Glu
        435                 440                 445

cgc gtg caa tcg aac ttt gtg cgg ggc tat tcc aag atg atg gtt aag 1392
Arg Val Gln Ser Asn Phe Val Arg Gly Tyr Ser Lys Met Met Val Lys
    450                 455                 460

ttg acg gct aaa aaa taa 1410
Leu Thr Ala Lys Lys
465

<210> 33
<211> 1410
<212> DNA
<213> Artificial Sequence

<220>
<223> Description of Artificial Sequence:hybrid gene

<220>
<221> CDS
<222> (1)..(1407)

<400> 33
atg tca acg agt tca agt aca agt aat gac atc cag gca aaa ata att 48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta 96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct 144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg 192
Gln Val His Phe Val Glu Thr Asp Leu Pro Asp Val Asn Asp Leu Ala

50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                 85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttt   432
Gln Ile Ile Leu Gly Asp Pro Pro Glu Gly Leu Ser Val Glu Met Phe
    130                 135                 140

atc gcg atg gac cct ccg gaa cac gac agg cag cgg gcc gct gtc caa   480
Ile Ala Met Asp Pro Pro Glu His Asp Arg Gln Arg Ala Ala Val Gln
145                 150                 155                 160

ggg gtg gtc gcc ccg aag aac ctg cgt gag atg gag agc ctc atc cgc   528
Gly Val Val Ala Pro Lys Asn Leu Arg Glu Met Glu Ser Leu Ile Arg
                165                 170                 175

gag cgg gcc cgg gat gtg ctc gac ggt ctc ccg ctc ggc gaa ccg ttc   576
Glu Arg Ala Arg Asp Val Leu Asp Gly Leu Pro Leu Gly Glu Pro Phe
            180                 185                 190

aac tgg gtg cag cac gtc tcc atc gag cta acc gcg cgg atg ctc gcc   624
Asn Trp Val Gln His Val Ser Ile Glu Leu Thr Ala Arg Met Leu Ala
        195                 200                 205

aca ctg ctg gac ttc ccg ttc gag cag cgg cgc aag ctc gtc gaa tgg   672
Thr Leu Leu Asp Phe Pro Phe Glu Gln Arg Arg Lys Leu Val Glu Trp
    210                 215                 220

tcc gat ctc gcc acc tct atg gag caa gcc gtc ggt gca ccc tcg gac   720
Ser Asp Leu Ala Thr Ser Met Glu Gln Ala Val Gly Ala Pro Ser Asp
225                 230                 235                 240

aac gat gag ata ttt cgt ggg atg gtc gat atg gct cga ggt ctc agc   768
Asn Asp Glu Ile Phe Arg Gly Met Val Asp Met Ala Arg Gly Leu Ser

                245                 250                 255

gct cac tgg cgg gac aag gca gcc cgg aca gct gcc ggg gag gag ccc 816
Ala His Trp Arg Asp Lys Ala Ala Arg Thr Ala Ala Gly Glu Glu Pro
            260                 265                 270

ggg ttc gat ctg atc acc atg ttg cag cgc gac gag agc acc aag gac 864
Gly Phe Asp Leu Ile Thr Met Leu Gln Arg Asp Glu Ser Thr Lys Asp
        275                 280                 285

ctg ctc gac cgc ccg atg gag ttc ctg ggc aac ttg acg ttg ctc atc 912
Leu Leu Asp Arg Pro Met Glu Phe Leu Gly Asn Leu Thr Leu Leu Ile
    290                 295                 300

gtg ggc ggc aac gac aca acg cgc aat tcc atg agc ggt ggt gtc ctc 960
Val Gly Gly Asn Asp Thr Thr Arg Asn Ser Met Ser Gly Gly Val Leu
305                 310                 315                 320

gcg ctg aac cag ttc cct gac cag ttc gag aag ctc aag gcg aac ccc 1008
Ala Leu Asn Gln Phe Pro Asp Gln Phe Glu Lys Leu Lys Ala Asn Pro
                325                 330                 335

ggc ctg atc ccc aac atg gtc tcg gag atc atc cgt tgg caa acc ccg 1056
Gly Leu Ile Pro Asn Met Val Ser Glu Ile Ile Arg Trp Gln Thr Pro
            340                 345                 350

ctc gcc cat atg cgc cgg atc gcc aag gcc gac acc atg ctg aac ggg 1104
Leu Ala His Met Arg Arg Ile Ala Lys Ala Asp Thr Met Leu Asn Gly
        355                 360                 365

cag ttc atc cgc aag ggc gac aag gtt ctg atg tgg tac gcc tcg ggt 1152
Gln Phe Ile Arg Lys Gly Asp Lys Val Leu Met Trp Tyr Ala Ser Gly
    370                 375                 380

aac cgc gac gaa cgg gtt ttc gaa cgg ccc gac gag ctg atc atc gat 1200
Asn Arg Asp Glu Arg Val Phe Glu Arg Pro Asp Glu Leu Ile Ile Asp
385                 390                 395                 400

cga ccc aac gcc cgt aac cat gtc tcc ttc ggt ttc ggc att cat cgt 1248
Arg Pro Asn Ala Arg Asn His Val Ser Phe Gly Phe Gly Ile His Arg
                405                 410                 415

tgt atg ggt aat agc ctt gcc gaa ctg cag ctg cgt att ctg tgg gaa 1296
Cys Met Gly Asn Ser Leu Ala Glu Leu Gln Leu Arg Ile Leu Trp Glu
            420                 425                 430

gag ctt ctc cct cgc ttt gaa aac atc gaa gtg atc ggt gag ccg gag 1344
Glu Leu Leu Pro Arg Phe Glu Asn Ile Glu Val Ile Gly Glu Pro Glu

435                 440                 445

cgc gtg caa tcg aac ttt gtg cgg ggc tat tcc aag atg atg gtt aag   1392
Arg Val Gln Ser Asn Phe Val Arg Gly Tyr Ser Lys Met Met Val Lys
    450                 455                 460

ttg acg gct aaa aaa taa                                           1410
Leu Thr Ala Lys Lys
465

<210> 34
<211> 1410
<212> DNA
<213> Artificial Sequence

<220>
<223> Description of Artificial Sequence:hybrid gene

<220>
<221> CDS
<222> (1)..(1407)

<400> 34
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                 85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttt   432
Gln Ile Ile Leu Gly Asp Pro Pro Glu Gly Leu Ser Val Glu Met Phe
    130                 135                 140

atc gct atg gat ccg ccc aaa cac gac atc cag cgc cag gcc gtg cag   480
Ile Ala Met Asp Pro Pro Lys His Asp Ile Gln Arg Gln Ala Val Gln
145                 150                 155                 160

ggt gta gtg gct ccc aag aac ctg aag gag atg gaa ggg ctg atc cgc   528
Gly Val Val Ala Pro Lys Asn Leu Lys Glu Met Glu Gly Leu Ile Arg
                165                 170                 175

cag cgt gtg cag gat gtg ctt gac aag ttg ccg gtc gac cag ccc ttt   576
Gln Arg Val Gln Asp Val Leu Asp Lys Leu Pro Val Asp Gln Pro Phe
            180                 185                 190

gac tgg gtc aat cgc gtc tcc att gag cta acc tcg cga atg ctt gcc   624
Asp Trp Val Asn Arg Val Ser Ile Glu Leu Thr Ser Arg Met Leu Ala
        195                 200                 205

acc ttg ctc gat ttc cct tac gag cag cgg cat aag ctc gtg tat tgg   672
Thr Leu Leu Asp Phe Pro Tyr Glu Gln Arg His Lys Leu Val Tyr Trp
    210                 215                 220

tcg gat atg gcg gct gcc agc gcg gaa acc act ggg ggc tcc ggc gat   720
Ser Asp Met Ala Ala Ala Ser Ala Glu Thr Thr Gly Gly Ser Gly Asp
225                 230                 235                 240

atg gac gag ctc ttt cac ggt ata gcc gac atg gct cgc cac ttc tcg   768
Met Asp Glu Leu Phe His Gly Ile Ala Asp Met Ala Arg His Phe Ser
                245                 250                 255

gcg ctc tgg cac gac aag gcg gct cgg agg gct gcc gga gag gaa ccc   816
Ala Leu Trp His Asp Lys Ala Ala Arg Arg Ala Ala Gly Glu Glu Pro
            260                 265                 270

ggc ttc gat ctg atc acc atg ctg cag agc aac gag agc acc aag gat   864
Gly Phe Asp Leu Ile Thr Met Leu Gln Ser Asn Glu Ser Thr Lys Asp
        275                 280                 285

ctg atc aaa cga ccg atg gag ttc tta ggc aac ctt gtg ctg ctg atc 912
Leu Ile Lys Arg Pro Met Glu Phe Leu Gly Asn Leu Val Leu Leu Ile
    290                 295                 300

gtc gga ggc aac gac acc acc cgg aac tcg atg agc ggt ggt gtt ctc 960
Val Gly Gly Asn Asp Thr Thr Arg Asn Ser Met Ser Gly Gly Val Leu
305                 310                 315                 320

gcg ttg aac cgg tac cca gat cag ttc gag aag ctg aag gcg aac ccc 1008
Ala Leu Asn Arg Tyr Pro Asp Gln Phe Glu Lys Leu Lys Ala Asn Pro
                325                 330                 335

gag ctg atc ccc aat atg gtc tcg gag gtc atc cgg tgg cag aca ccg 1056
Glu Leu Ile Pro Asn Met Val Ser Glu Val Ile Arg Trp Gln Thr Pro
            340                 345                 350

ttg gcc tat atg cgc cgg atc gcc aag gcg gac act atg ctg agc ggg 1104
Leu Ala Tyr Met Arg Arg Ile Ala Lys Ala Asp Thr Met Leu Ser Gly
        355                 360                 365

cag ttc att cgc aag ggc gac aag gtc gtg atg tgg tac gcg tcg ggc 1152
Gln Phe Ile Arg Lys Gly Asp Lys Val Val Met Trp Tyr Ala Ser Gly
    370                 375                 380

aac cgc gac gag cgc gtg ttc gat cgg ccc gat gac ctg atc atc gat 1200
Asn Arg Asp Glu Arg Val Phe Asp Arg Pro Asp Asp Leu Ile Ile Asp
385                 390                 395                 400

cgg gcc aac gcc cgc aac cac atc tcc ttc ggc ttt ggc atc cac cgg 1248
Arg Ala Asn Ala Arg Asn His Ile Ser Phe Gly Phe Gly Ile His Arg
                405                 410                 415

tgc atg ggt aat cgg ctt gcc gaa ctg cag ctg cgt att ctg tgg gaa 1296
Cys Met Gly Asn Arg Leu Ala Glu Leu Gln Leu Arg Ile Leu Trp Glu
            420                 425                 430

gag ctt ctc cct cgc ttt gaa aac atc gaa gtg atc ggt gag ccg gag 1344
Glu Leu Leu Pro Arg Phe Glu Asn Ile Glu Val Ile Gly Glu Pro Glu
        435                 440                 445

cgc gtg caa tcg aac ttt gtg cgg ggc tat tcc aag atg atg gtt aag 1392
Arg Val Gln Ser Asn Phe Val Arg Gly Tyr Ser Lys Met Met Val Lys
    450                 455                 460

ttg acg gct aaa aaa taa 1410
Leu Thr Ala Lys Lys
465

<210> 35
<211> 1410
<212> DNA
<213> Artificial Sequence

<220>
<223> Description of Artificial Sequence:hybrid gene

<220>
<221> CDS
<222> (1)..(1407)

<400> 35
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                 85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttt   432
Gln Ile Ile Leu Gly Asp Pro Pro Glu Gly Leu Ser Val Glu Met Phe

130                 135                 140

atc gca atg gac cca cca cgc cac gac aag cag cgg gcc gct gtt caa    480
Ile Ala Met Asp Pro Pro Arg His Asp Lys Gln Arg Ala Ala Val Gln
145                 150                 155                 160

ggg gtg gtc gcg ccc aag aac ctg cgt gag atg gag ggt ctg att cga    528
Gly Val Val Ala Pro Lys Asn Leu Arg Glu Met Glu Gly Leu Ile Arg
                165                 170                 175

tcg cgc gtg cag gag gtg ctg gac aac ctg ccc gtg gat cag ccg ttc    576
Ser Arg Val Gln Glu Val Leu Asp Asn Leu Pro Val Asp Gln Pro Phe
            180                 185                 190

gac tgg ata cag aat gtt tcg atc gag ttg acc gct cgg atg ctt gcg    624
Asp Trp Ile Gln Asn Val Ser Ile Glu Leu Thr Ala Arg Met Leu Ala
        195                 200                 205

acc ctc ctg gac ttc ccc tac gag cag cgt cgc aaa ctc gtc gag tgg    672
Thr Leu Leu Asp Phe Pro Tyr Glu Gln Arg Arg Lys Leu Val Glu Trp
    210                 215                 220

tca gat ctg gca acc tcg atg gag caa gcc aat ggc ggt ccc tcg gac    720
Ser Asp Leu Ala Thr Ser Met Glu Gln Ala Asn Gly Gly Pro Ser Asp
225                 230                 235                 240

ctt gac gac acg ttc gca ggc atg cgc gac atg gcg cga ggc ctc agc    768
Leu Asp Asp Thr Phe Ala Gly Met Arg Asp Met Ala Arg Gly Leu Ser
                245                 250                 255

gag cac tgg cac gac aag gcg gcc cgg agg gct gcc gga gag gaa ccc    816
Glu His Trp His Asp Lys Ala Ala Arg Arg Ala Ala Gly Glu Glu Pro
            260                 265                 270

ggc ttc gat ctg atc acc atg ctg cag agc aac gag agc acc aag gat    864
Gly Phe Asp Leu Ile Thr Met Leu Gln Ser Asn Glu Ser Thr Lys Asp
        275                 280                 285

ctg atc aaa cga ccg atg gag ttc ttg ggc aac ctt gtg ctg ctg atc    912
Leu Ile Lys Arg Pro Met Glu Phe Leu Gly Asn Leu Val Leu Leu Ile
    290                 295                 300

gtc gga ggc aac gac acc acc cgg aac tcg atg agc ggc ggt gtt ctc    960
Val Gly Gly Asn Asp Thr Thr Arg Asn Ser Met Ser Gly Gly Val Leu
305                 310                 315                 320

gcg ttg aac cgg tac cca gat cag ttc gag aag ctg aag gca aac ccc    1008
Ala Leu Asn Arg Tyr Pro Asp Gln Phe Glu Lys Leu Lys Ala Asn Pro

325                 330                 335

gac ctg atc ccc aac atg gtc tcg gag gtc atc cgg tgg cag aca ccg    1056
Asp Leu Ile Pro Asn Met Val Ser Glu Val Ile Arg Trp Gln Thr Pro
        340                 345                 350

ttg gcc tat atg cgc cgg atc gcc aag gcc gac act atg ctg aac ggg    1104
Leu Ala Tyr Met Arg Arg Ile Ala Lys Ala Asp Thr Met Leu Asn Gly
    355                 360                 365

cag ttc att cgc aag ggc gac aag gtc gtg atg tgg tac gcg tcg ggc    1152
Gln Phe Ile Arg Lys Gly Asp Lys Val Val Met Trp Tyr Ala Ser Gly
  370                 375                 380

aac cgc gac gag cgc gtg ttc gat cgg ccc gac gac ttg atc atc gac    1200
Asn Arg Asp Glu Arg Val Phe Asp Arg Pro Asp Asp Leu Ile Ile Asp
385                 390                 395                 400

cgg gcc aac gcc cgc aac cac atc tcc ttc ggc ttt ggc atc cat cgt    1248
Arg Ala Asn Ala Arg Asn His Ile Ser Phe Gly Phe Gly Ile His Arg
                405                 410                 415

tgt atg ggg aac cga ctt gcc gaa ctg cag ctg cgt att ctg tgg gaa    1296
Cys Met Gly Asn Arg Leu Ala Glu Leu Gln Leu Arg Ile Leu Trp Glu
        420                 425                 430

gag ctt ctc cct cgc ttt gaa aac atc gaa gtg atc ggt gag ccg gag    1344
Glu Leu Leu Pro Arg Phe Glu Asn Ile Glu Val Ile Gly Glu Pro Glu
    435                 440                 445

cgc gtg caa tcg aac ttt gtg cgg ggc tat tcc aag atg atg gtt aag    1392
Arg Val Gln Ser Asn Phe Val Arg Gly Tyr Ser Lys Met Met Val Lys
  450                 455                 460

ttg acg gct aaa aaa taa                                            1410
Leu Thr Ala Lys Lys
465

<210> 36
<211> 1410
<212> DNA
<213> Artificial Sequence

<220>
<223> Description of Artificial Sequence:hybrid gene

<220>

<221> CDS
<222> (1)..(1407)

<400> 36
atg tca acg agt tca agt aca agt aat gac atc cag gca aaa ata att   48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta   96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

atc gca atg gat cca cca aaa cac gac cgg cag cgg gcc gct gtc caa   480
Ile Ala Met Asp Pro Pro Lys His Asp Arg Gln Arg Ala Ala Val Gln
145                 150                 155                 160

ggg gtg gtt gcg ccc aag aac ctg cgt gag atg gag ggt ctg att cga   528
Gly Val Val Ala Pro Lys Asn Leu Arg Glu Met Glu Gly Leu Ile Arg
                165                 170                 175

tcg cgc gtg cag gag gtg ctg gac aac ctg ccc gtg gat cag ccg ttc   576
Ser Arg Val Gln Glu Val Leu Asp Asn Leu Pro Val Asp Gln Pro Phe
            180                 185                 190

gac tgg gtg cag aat gtt tcg atc gag ttg acc gcc cgg atg ctt gca   624
Asp Trp Val Gln Asn Val Ser Ile Glu Leu Thr Ala Arg Met Leu Ala
        195                 200                 205

acc ctc ctg gac ttc ccg tac gag cag cgt cgc aaa ctc gtc gag tgg   672
Thr Leu Leu Asp Phe Pro Tyr Glu Gln Arg Arg Lys Leu Val Glu Trp
    210                 215                 220

tca gat ctg gca acc tcg atg gag caa gcc aat ggc ggt ccc tcg gac   720
Ser Asp Leu Ala Thr Ser Met Glu Gln Ala Asn Gly Gly Pro Ser Asp
225                 230                 235                 240

ctt gac gag acg ttt gta ggc atg cgc gac atg gcg cga ggc ctc agc   768
Leu Asp Glu Thr Phe Val Gly Met Arg Asp Met Ala Arg Gly Leu Ser
                245                 250                 255

gag cac tgg cac gac aag gcg gct cgg agg gct gcc gga gag gaa ccc   816
Glu His Trp His Asp Lys Ala Ala Arg Arg Ala Ala Gly Glu Glu Pro
            260                 265                 270

ggc ttc gat ctg atc acc atg ctg cag agc aac gag agc acc aag gat   864
Gly Phe Asp Leu Ile Thr Met Leu Gln Ser Asn Glu Ser Thr Lys Asp
        275                 280                 285

ctg atc aaa cga ccg atg gag ttc tta ggc aac ctt gtg ctg ctg atc   912
Leu Ile Lys Arg Pro Met Glu Phe Leu Gly Asn Leu Val Leu Leu Ile
    290                 295                 300

gtc gga ggc aac gac acc acc cgg aac tcg atg agc ggt ggt gtt ctc   960
Val Gly Gly Asn Asp Thr Thr Arg Asn Ser Met Ser Gly Gly Val Leu
305                 310                 315                 320

gcg ttg aac cgg tac cca gat cag ttc gag aag ctg aag gcg aac ccc   1008
Ala Leu Asn Arg Tyr Pro Asp Gln Phe Glu Lys Leu Lys Ala Asn Pro
                325                 330                 335

gag ctg atc ccc aat atg gtc tcg gag gtc atc cgg tgg cag aca ccg   1056
Glu Leu Ile Pro Asn Met Val Ser Glu Val Ile Arg Trp Gln Thr Pro
            340                 345                 350

ttg gcc tat atg cgc cgg atc gcc aag gcg gac act atg ctg aac ggg   1104
Leu Ala Tyr Met Arg Arg Ile Ala Lys Ala Asp Thr Met Leu Asn Gly
        355                 360                 365

cag ttc att cgc aag ggc gac aag gtc gtg atg tgg tac gcg tcg ggc 1152
Gln Phe Ile Arg Lys Gly Asp Lys Val Val Met Trp Tyr Ala Ser Gly
    370                 375                 380

aac cgc gac gag cgc gtg ttc gat cgg ccc gat gac ctg atc atc gat 1200
Asn Arg Asp Glu Arg Val Phe Asp Arg Pro Asp Asp Leu Ile Ile Asp
385                 390                 395                 400

cgg gcc aac gcc cgc aac cac atc tcc ttc ggc ttt ggc atc cac cgc 1248
Arg Ala Asn Ala Arg Asn His Ile Ser Phe Gly Phe Gly Ile His Arg
                405                 410                 415

tgt atg ggc aac agt ctt gcc gaa ctg cag ctg cgt att ctg tgg gaa 1296
Cys Met Gly Asn Ser Leu Ala Glu Leu Gln Leu Arg Ile Leu Trp Glu
            420                 425                 430

gag ctt ctc cct cgc ttt gaa aac atc gaa gtg atc ggt gag ccg gag 1344
Glu Leu Leu Pro Arg Phe Glu Asn Ile Glu Val Ile Gly Glu Pro Glu
        435                 440                 445

cgc gtg caa tcg aac ttt gtg cgg ggc tat tcc aag atg atg gtt aag 1392
Arg Val Gln Ser Asn Phe Val Arg Gly Tyr Ser Lys Met Met Val Lys
    450                 455                 460

ttg acg gct aaa aaa taa 1410
Leu Thr Ala Lys Lys
465

<210> 37
<211> 1386
<212> DNA
<213> Artificial Sequence

<220>
<223> Description of Artificial Sequence:hybrid gene

<220>
<221> CDS
<222> (1)..(1383)

<400> 37
atg tca acg agt tca agt aca agt aat gac atc cag gca aaa ata att 48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta 96
Asn Ala Thr Ser Lys Val Val Pro Met His Leu Gln Ile Lys Ala Leu

            20                  25                  30

aaa aac ttg atg aag gtg aag cgg aag acc att ggc act tcc cgc cct 144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg 192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag 240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat 288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa 336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc 384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc 432
Gln Ile Ile Leu Gly Asp Pro Pro Glu Gly Leu Ser Val Glu Met Phe
    130                 135                 140

atc gca atg gac ccc ccc aag cac gac gtg cag cgc aag acc gtc tcg 480
Ile Ala Met Asp Pro Pro Lys His Asp Val Gln Arg Lys Thr Val Ser
145                 150                 155                 160

ccg atc gtc agc ccg cac agc ctg atg cag ctg gag ccg atc atc cgc 528
Pro Ile Val Ser Pro His Ser Leu Met Gln Leu Glu Pro Ile Ile Arg
                165                 170                 175

gag cgc gcc gcc ggc ctg ctc gac agc ctg ccg atc ggc gag acc ttc 576
Glu Arg Ala Ala Gly Leu Leu Asp Ser Leu Pro Ile Gly Glu Thr Phe
            180                 185                 190

gac tgg gtc gac cgc atc tcc atc gaa ctg acc acc cag atg ctg gtg 624
Asp Trp Val Asp Arg Ile Ser Ile Glu Leu Thr Thr Gln Met Leu Val
        195                 200                 205

acg ctg ttc gac ttc ccg ttc cat gat cgc cgc aag ctg acc cgc tgg 672
Thr Leu Phe Asp Phe Pro Phe His Asp Arg Arg Lys Leu Thr Arg Trp

210                 215                 220

tct gac gtg gcc acg gcc gcg ccg ggc atg ggg gtg atc gag acc gag    720
Ser Asp Val Ala Thr Ala Ala Pro Gly Met Gly Val Ile Glu Thr Glu
225                 230                 235                 240

gaa cag cgc cgc gag gaa ctg ttc gag tgc ctc gcc tac ttc acc aac    768
Glu Gln Arg Arg Glu Glu Leu Phe Glu Cys Leu Ala Tyr Phe Thr Asn
                245                 250                 255

ctg tgg aac gag cgg gtc aac gcc ccg ccg gcc aac aac ctg atc tcg    816
Leu Trp Asn Glu Arg Val Asn Ala Pro Pro Ala Asn Asn Leu Ile Ser
            260                 265                 270

ctg ctg gcc cat ggc gag cag acc cgc aac atg gag ccg atg gag tat    864
Leu Leu Ala His Gly Glu Gln Thr Arg Asn Met Glu Pro Met Glu Tyr
        275                 280                 285

ctg ggg aac ctg atc ctg ctc atc gtc ggc ggc aac gac acc acc cgc    912
Leu Gly Asn Leu Ile Leu Leu Ile Val Gly Gly Asn Asp Thr Thr Arg
    290                 295                 300

aac acc atg acc ggc tcg gtg ctg gcg ctg aac cag aac cca gat cag    960
Asn Thr Met Thr Gly Ser Val Leu Ala Leu Asn Gln Asn Pro Asp Gln
305                 310                 315                 320

tac gcc aag ctg aag gcc aac ccc gag ctg atc ccg tcg atg gtc tcc    1008
Tyr Ala Lys Leu Lys Ala Asn Pro Glu Leu Ile Pro Ser Met Val Ser
                325                 330                 335

gag acc atc cgc tgg cag acg ccg ctg gcc cac atg cgc cgc acg gcg    1056
Glu Thr Ile Arg Trp Gln Thr Pro Leu Ala His Met Arg Arg Thr Ala
            340                 345                 350

ctg gcc gac tac gag atc ggc ggc aag acc atc aag aag ggc gac aag    1104
Leu Ala Asp Tyr Glu Ile Gly Gly Lys Thr Ile Lys Lys Gly Asp Lys
        355                 360                 365

gtc gcc atg tgg tac gtc tcg ggc aac cgc gac gag acg gcg atc gag    1152
Val Ala Met Trp Tyr Val Ser Gly Asn Arg Asp Glu Thr Ala Ile Glu
    370                 375                 380

aat ccg gac gcc tac atc atc gac cgc gag cgt ccc cgc cag cac ctg    1200
Asn Pro Asp Ala Tyr Ile Ile Asp Arg Glu Arg Pro Arg Gln His Leu
385                 390                 395                 400

tcc ttc ggc ttc ggc atc cac cga tgc atg ggc aac agg ctt gcc gaa    1248
Ser Phe Gly Phe Gly Ile His Arg Cys Met Gly Asn Arg Leu Ala Glu

405                 410                 415

ctg cag ctg cgt att ctg tgg gaa gag ctt ctc cct cgc ttt gaa aac   1296
Leu Gln Leu Arg Ile Leu Trp Glu Glu Leu Leu Pro Arg Phe Glu Asn
        420                 425                 430

atc gaa gtg atc ggt gag ccg gag cgc gtg caa tcg aac ttt gtg cgg   1344
Ile Glu Val Ile Gly Glu Pro Glu Arg Val Gln Ser Asn Phe Val Arg
    435                 440                 445

ggc tat tcc aag atg atg gtt aag ttg acg gct aaa aaa taa          1386
Gly Tyr Ser Lys Met Met Val Lys Leu Thr Ala Lys Lys
450                 455                 460

<210> 38
<211> 1395
<212> DNA
<213> Artificial Sequence

<220>
<223> Description of Artificial Sequence:hybrid gene

<220>
<221> CDS
<222> (1)..(1392)

<400> 38
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat 288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa 336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc 384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc 432
Gln Ile Ile Leu Gly Asp Pro Pro Glu Gly Leu Ser Val Glu Met Phe
    130                 135                 140

att gcg atg gac cct ccc aag cac gac gtc cag cgc aag acc gtc agc 480
Ile Ala Met Asp Pro Pro Lys His Asp Val Gln Arg Lys Thr Val Ser
145                 150                 155                 160

ccg att gtc tcg ccg gcc aac ctc cac ctg atg gag ccg ttg atc cgc 528
Pro Ile Val Ser Pro Ala Asn Leu His Leu Met Glu Pro Leu Ile Arg
                165                 170                 175

agc cgc atc acc aag acc ctg gac gaa cta ccg atc ggc gag ccc ttc 576
Ser Arg Ile Thr Lys Thr Leu Asp Glu Leu Pro Ile Gly Glu Pro Phe
            180                 185                 190

gac tgg gtc gat cgg gtc tcc atc gaa ctc acc acc caa atg ctg gcc 624
Asp Trp Val Asp Arg Val Ser Ile Glu Leu Thr Thr Gln Met Leu Ala
        195                 200                 205

acc ctg ttc gac ttc ccc tgg gat gaa cgg cgc aaa cta acc cgc tgg 672
Thr Leu Phe Asp Phe Pro Trp Asp Glu Arg Arg Lys Leu Thr Arg Trp
    210                 215                 220

tcc gac atc gcc acc gcg ggc ccc caa tcc ggc ctg ttc ctc acg gat 720
Ser Asp Ile Ala Thr Ala Gly Pro Gln Ser Gly Leu Phe Leu Thr Asp
225                 230                 235                 240

gac tac gag acc gag cgc agg atg gaa ctg ttc gcc tgc gtg gac tat 768
Asp Tyr Glu Thr Glu Arg Arg Met Glu Leu Phe Ala Cys Val Asp Tyr
                245                 250                 255

ttc acg cga ctc tgg aac gag cgg gtc aac gcc ccg ccc aag ggc gat 816
Phe Thr Arg Leu Trp Asn Glu Arg Val Asn Ala Pro Pro Lys Gly Asp
            260                 265                 270

ctg atc tcc atg ctg gcc cat ggc gag gcc acg cgg aac atg gac cgc 864
Leu Ile Ser Met Leu Ala His Gly Glu Ala Thr Arg Asn Met Asp Arg
        275                 280                 285

atg gag tac ctc ggg aat ctg ctc cta ctg atc atc ggc ggc aat gac 912
Met Glu Tyr Leu Gly Asn Leu Leu Leu Leu Ile Ile Gly Gly Asn Asp
    290                 295                 300

acc acc cga aat acg atg acc ggc tcg atc tta gcg atg aac gag aac 960
Thr Thr Arg Asn Thr Met Thr Gly Ser Ile Leu Ala Met Asn Glu Asn
305                 310                 315                 320

ccc gag cag cta cgt aaa ctg cag gcg aag cca gac ctg atc ccc tcc 1008
Pro Glu Gln Leu Arg Lys Leu Gln Ala Lys Pro Asp Leu Ile Pro Ser
                325                 330                 335

atg gtt tcg gag acc att cgc tgg cag acc ccg ctc tcc aac atg cgg 1056
Met Val Ser Glu Thr Ile Arg Trp Gln Thr Pro Leu Ser Asn Met Arg
            340                 345                 350

cgc acc gcc acc caa gat ttc gag ctt ggc ggc aag ctc atc aag aag 1104
Arg Thr Ala Thr Gln Asp Phe Glu Leu Gly Gly Lys Leu Ile Lys Lys
        355                 360                 365

ggc gac aag gtg ctg atc tgg tac gcc tca ggc aac cgg gac gag gag 1152
Gly Asp Lys Val Leu Ile Trp Tyr Ala Ser Gly Asn Arg Asp Glu Glu
    370                 375                 380

gcg atc gag aac cct gaa gct tac atc atc gac cga gag cgg ccg cgc 1200
Ala Ile Glu Asn Pro Glu Ala Tyr Ile Ile Asp Arg Glu Arg Pro Arg
385                 390                 395                 400

aac cac ctg tcg ttc ggg ttc ggg att cac cgg tgc atg ggc aat cgc 1248
Asn His Leu Ser Phe Gly Phe Gly Ile His Arg Cys Met Gly Asn Arg
                405                 410                 415

ctt gcc gaa ctg cag ctg cgt att ctg tgg gaa gag ctt ctc cct cgc 1296
Leu Ala Glu Leu Gln Leu Arg Ile Leu Trp Glu Glu Leu Leu Pro Arg
            420                 425                 430

ttt gaa aac atc gaa gtg atc ggt gag ccg gag cgc gtg caa tcg aac 1344
Phe Glu Asn Ile Glu Val Ile Gly Glu Pro Glu Arg Val Gln Ser Asn
        435                 440                 445

ttt gtg cgg ggc tat tcc aag atg atg gtt aag ttg acg gct aaa aaa 1392
Phe Val Arg Gly Tyr Ser Lys Met Met Val Lys Leu Thr Ala Lys Lys
    450                 455                 460

taa                                                          1395

<210> 39
<211> 1395
<212> DNA
<213> Artificial Sequence

<220>
<223> Description of Artificial Sequence:hybrid gene

<220>
<221> CDS
<222> (1)..(1392)

<400> 39
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                 85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc 432
Gln Ile Ile Leu Gly Asp Pro Pro Glu Gly Leu Ser Val Glu Met Phe
    130                 135                 140

atc gct atg gac ccg ccc aag cat gac atc cag cgc aag acc gtc agc 480
Ile Ala Met Asp Pro Pro Lys His Asp Ile Gln Arg Lys Thr Val Ser
145                 150                 155                 160

ccg atc gtc tcg ccg cag aac ctg cac ctg ctg gag ccg ctg atc cgg 528
Pro Ile Val Ser Pro Gln Asn Leu His Leu Leu Glu Pro Leu Ile Arg
                165                 170                 175

tcg cgc atc acc aag acc ctt gat gaa ctg ccc atc ggc cag ccc ttc 576
Ser Arg Ile Thr Lys Thr Leu Asp Glu Leu Pro Ile Gly Gln Pro Phe
            180                 185                 190

gac tgg gtg gac cgg gtg tcc atc gaa ctg acc acg cag atg ctg gcg 624
Asp Trp Val Asp Arg Val Ser Ile Glu Leu Thr Thr Gln Met Leu Ala
        195                 200                 205

acc ctg ttc gac ttc ccc ttc gag gag cgc cgc aag ctg acc ttc tgg 672
Thr Leu Phe Asp Phe Pro Phe Glu Glu Arg Arg Lys Leu Thr Phe Trp
    210                 215                 220

tcg gac gtg gcc acc gcc ccg ccg gag tcc aag ctg ttc aag acg gac 720
Ser Asp Val Ala Thr Ala Pro Pro Glu Ser Lys Leu Phe Lys Thr Asp
225                 230                 235                 240

gac ccg gaa acc gag cgc aag atg atc ctg ttc gag tgc gtg gac tac 768
Asp Pro Glu Thr Glu Arg Lys Met Ile Leu Phe Glu Cys Val Asp Tyr
                245                 250                 255

ttc acg cgc ctg tgg aac gag cgg gtg aat gcg ccg ccg aag ggc gac 816
Phe Thr Arg Leu Trp Asn Glu Arg Val Asn Ala Pro Pro Lys Gly Asp
            260                 265                 270

ctg atc tcc atg ctg gcg cac ggc gaa gcc acc cgg aac atg gac cgg 864
Leu Ile Ser Met Leu Ala His Gly Glu Ala Thr Arg Asn Met Asp Arg
        275                 280                 285

atg gag tat ctc ggg aac ctg atc ctg ctg atc gtc ggc ggc aac gac 912
Met Glu Tyr Leu Gly Asn Leu Ile Leu Leu Ile Val Gly Gly Asn Asp
    290                 295                 300

acc acg cgc aac acc atg tcc ggc tcg atc gtg gcc atg cac cag aac 960
Thr Thr Arg Asn Thr Met Ser Gly Ser Ile Val Ala Met His Gln Asn
305                 310                 315                 320

ccg gac cag ctg gcc aag ctt cag gcc gac cac tcg ctg gtg ccg tcg    1008
Pro Asp Gln Leu Ala Lys Leu Gln Ala Asp His Ser Leu Val Pro Ser
            325                 330                 335

atg gtg tcg gaa acc atc cgc tgg cag acc ccg ctg gct cac atg cgc    1056
Met Val Ser Glu Thr Ile Arg Trp Gln Thr Pro Leu Ala His Met Arg
        340                 345                 350

cgc acc gcc acc cag gac ttc gaa ctg ggc ggc aag acc atc aag aag    1104
Arg Thr Ala Thr Gln Asp Phe Glu Leu Gly Gly Lys Thr Ile Lys Lys
    355                 360                 365

ggc gac aag gtc gtc atg tgg tac gtc tcg ggc aac cgc gac gac gag    1152
Gly Asp Lys Val Val Met Trp Tyr Val Ser Gly Asn Arg Asp Asp Glu
370                 375                 380

gtg atc gag cgc gcg gac gaa ttt ctg atc gac cgc aaa aac ccg cgt    1200
Val Ile Glu Arg Ala Asp Glu Phe Leu Ile Asp Arg Lys Asn Pro Arg
385                 390                 395                 400

cat cac gcg tcc ttt ggc ttt ggc att cac agt tgc atg ggt aac cgg    1248
His His Ala Ser Phe Gly Phe Gly Ile His Ser Cys Met Gly Asn Arg
            405                 410                 415

ctt gcc gaa ctg cag ctg cgt att ctg tgg gaa gag ctt ctc cct cgc    1296
Leu Ala Glu Leu Gln Leu Arg Ile Leu Trp Glu Glu Leu Leu Pro Arg
        420                 425                 430

ttt gaa aac atc gaa gtg atc ggt gag ccg gag cgc gtg caa tcg aac    1344
Phe Glu Asn Ile Glu Val Ile Gly Glu Pro Glu Arg Val Gln Ser Asn
    435                 440                 445

ttt gtg cgg ggc tat tcc aag atg atg gtt aag ttg acg gct aaa aaa    1392
Phe Val Arg Gly Tyr Ser Lys Met Met Val Lys Leu Thr Ala Lys Lys
450                 455                 460

taa                                                                1395

<210> 40
<211> 1386
<212> DNA
<213> Artificial Sequence

<220>
<223> Description of Artificial Sequence:hybrid gene

<220>

<221> CDS
<222> (1)..(1383)

<400> 40
atg tca acg agt tca agt aca agt aat gac atc cag gca aaa ata att   48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta   96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

atc gcg atg gat ccg ccc aag cac gac gac cag cgc aag gta gta tcg   480
Ile Ala Met Asp Pro Pro Lys His Asp Asp Gln Arg Lys Val Val Ser
145                 150                 155                 160

ccg gca ctg ggg ccc gag aat ctc gcc aag ctg gcc gat ggc att cgc   528
Pro Ala Leu Gly Pro Glu Asn Leu Ala Lys Leu Ala Asp Gly Ile Arg
                165                 170                 175

acg cgc acc ggt caa ctg ctc gac caa ttg ccg atc aac gaa acc ttt 576
Thr Arg Thr Gly Gln Leu Leu Asp Gln Leu Pro Ile Asn Glu Thr Phe
            180                 185                 190

gac tgg gtt gat cgc gtc tcc atc gaa ctg acg acg cag atg ctc gcg 624
Asp Trp Val Asp Arg Val Ser Ile Glu Leu Thr Thr Gln Met Leu Ala
        195                 200                 205

atc ctg ttc gat ttt ccc tgg gag gag cgc cgc aag cta acc cac tgg 672
Ile Leu Phe Asp Phe Pro Trp Glu Glu Arg Arg Lys Leu Thr His Trp
    210                 215                 220

tcc gat acc gcc acc gcg atg acg gcc cat ccc acg gac gcg gag aag 720
Ser Asp Thr Ala Thr Ala Met Thr Ala His Pro Thr Asp Ala Glu Lys
225                 230                 235                 240

gca gcc ggc ttg gcc gaa ctc ggc gag tgc gcg gcg tat ttc acg cga 768
Ala Ala Gly Leu Ala Glu Leu Gly Glu Cys Ala Ala Tyr Phe Thr Arg
                245                 250                 255

ctg tgg aac gag cgg gtg aac gcg gag ccg cgc gcc gat ctg ctg tcg 816
Leu Trp Asn Glu Arg Val Asn Ala Glu Pro Arg Ala Asp Leu Leu Ser
            260                 265                 270

atg ctg gct cac tcc gag gcg acc cgc aac atg ggg ccg aag gag ttt 864
Met Leu Ala His Ser Glu Ala Thr Arg Asn Met Gly Pro Lys Glu Phe
        275                 280                 285

ctg ggc aat ctg att ctg ctc att gtc ggc ggc aac gac act acg cgc 912
Leu Gly Asn Leu Ile Leu Leu Ile Val Gly Gly Asn Asp Thr Thr Arg
    290                 295                 300

aat tcg atg acc ggc ggc ctg ctg gcg ctg cac gac aat ccc gat caa 960
Asn Ser Met Thr Gly Gly Leu Leu Ala Leu His Asp Asn Pro Asp Gln
305                 310                 315                 320

tac cgc aag ctg tgc gaa aac ccg aaa ctg gtc gat tcc ttg gtg ccg 1008
Tyr Arg Lys Leu Cys Glu Asn Pro Lys Leu Val Asp Ser Leu Val Pro
                325                 330                 335

gag atc atc cgc tgg caa acg ccg gta acc agc atg cga cgc acc gcg 1056
Glu Ile Ile Arg Trp Gln Thr Pro Val Thr Ser Met Arg Arg Thr Ala
            340                 345                 350

ctg cgg gat acc gtg ctc ggc ggc aag gtc att cac gag ggt gat cgc 1104
Leu Arg Asp Thr Val Leu Gly Gly Lys Val Ile His Glu Gly Asp Arg
        355                 360                 365

gtg atc atg tgg tac gcg tcg gcc aat cgc gat ccc gat gcc atc gaa   1152
Val Ile Met Trp Tyr Ala Ser Ala Asn Arg Asp Pro Asp Ala Ile Glu
    370                 375                 380

aat ccc aat gcc ttc atc atc gac cgg gca cga cca cgg cag cac ctt   1200
Asn Pro Asn Ala Phe Ile Ile Asp Arg Ala Arg Pro Arg Gln His Leu
385                 390                 395                 400

tcc ttc ggt ttt ggc atc cac cga tgc atg ggc aac cgg ctt gcc gaa   1248
Ser Phe Gly Phe Gly Ile His Arg Cys Met Gly Asn Arg Leu Ala Glu
                405                 410                 415

ctg cag ctg cgt att ctg tgg gaa gag ctt ctc cct cgc ttt gaa aac   1296
Leu Gln Leu Arg Ile Leu Trp Glu Glu Leu Leu Pro Arg Phe Glu Asn
            420                 425                 430

atc gaa gtg atc ggt gag ccg gag cgc gtg caa tcg aac ttt gtg cgg   1344
Ile Glu Val Ile Gly Glu Pro Glu Arg Val Gln Ser Asn Phe Val Arg
        435                 440                 445

ggc tat tcc aag atg atg gtt aag ttg acg gct aaa aaa taa           1386
Gly Tyr Ser Lys Met Met Val Lys Leu Thr Ala Lys Lys
    450                 455                 460

<210> 41
<211> 1386
<212> DNA
<213> Artificial Sequence

<220>
<223> Description of Artificial Sequence:hybrid gene

<220>
<221> CDS
<222> (1)..(1383)

<400> 41
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro

35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg cca gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

atc gcg atg gac ccg ccc aag cac gac atc cag cgc aag acg gtc agc   480
Ile Ala Met Asp Pro Pro Lys His Asp Ile Gln Arg Lys Thr Val Ser
145                 150                 155                 160

ccg atc gtc tcg ccg atg aac ctg gcc aag cta gag ggc gtg atc cgc   528
Pro Ile Val Ser Pro Met Asn Leu Ala Lys Leu Glu Gly Val Ile Arg
                165                 170                 175

gag cgg gcc ggg ctg atc ctt gac ggc ctg ccc atc ggg gag ccc ttc   576
Glu Arg Ala Gly Leu Ile Leu Asp Gly Leu Pro Ile Gly Glu Pro Phe
            180                 185                 190

gac tgg gtc gac cgg gta tcg atc gaa ctg acc acc cag atg ctg gcc   624
Asp Trp Val Asp Arg Val Ser Ile Glu Leu Thr Thr Gln Met Leu Ala
        195                 200                 205

acc ctg ttc gac ttc cct tgg gaa gac cgt gcc aag ctg acg cgc tgg   672
Thr Leu Phe Asp Phe Pro Trp Glu Asp Arg Ala Lys Leu Thr Arg Trp
    210                 215                 220

tcg gat gtc gca acg gcg ctg gtc ggc acg ggc att att gat tcg gaa   720
Ser Asp Val Ala Thr Ala Leu Val Gly Thr Gly Ile Ile Asp Ser Glu

225                 230                 235                 240

gag cag cgc atg gag gag ctc aag ggg tgc gcg caa tac atg acc cgg   768
Glu Gln Arg Met Glu Glu Leu Lys Gly Cys Ala Gln Tyr Met Thr Arg
                245                 250                 255

ctg tgg aac gag cgc gtc aat gtg cca ccg ggc aat gat ctg ata tcg   816
Leu Trp Asn Glu Arg Val Asn Val Pro Pro Gly Asn Asp Leu Ile Ser
            260                 265                 270

atg atg gcg cac acc gag tcc atg cgc aac atg aca ccg gaa gag ttt   864
Met Met Ala His Thr Glu Ser Met Arg Asn Met Thr Pro Glu Glu Phe
        275                 280                 285

ctg ggc aac ctc att ttg ctg atc gtc ggc ggc aat gac acg acc cgc   912
Leu Gly Asn Leu Ile Leu Leu Ile Val Gly Gly Asn Asp Thr Thr Arg
    290                 295                 300

aac tcg atg acc ggc ggc gtg ctg gcg ctc aac gaa aat ccg gac gaa   960
Asn Ser Met Thr Gly Gly Val Leu Ala Leu Asn Glu Asn Pro Asp Glu
305                 310                 315                 320

tac cgc aag ctg tgc gcc aac ccg gcg ctg atc gcc tcc atg gtg ccg   1008
Tyr Arg Lys Leu Cys Ala Asn Pro Ala Leu Ile Ala Ser Met Val Pro
                325                 330                 335

gag atc gtg cgt tgg cag aca ccg ctg tcg cac atg cgg cgc acc gcg   1056
Glu Ile Val Arg Trp Gln Thr Pro Leu Ser His Met Arg Arg Thr Ala
            340                 345                 350

ctg cag gac acc gag ctc ggc ggc aag tcc att cgc aag ggt gac aag   1104
Leu Gln Asp Thr Glu Leu Gly Gly Lys Ser Ile Arg Lys Gly Asp Lys
        355                 360                 365

gtc atc atg tgg tat gtc tcc ggc aac cgt gat ccc gaa gcg att gaa   1152
Val Ile Met Trp Tyr Val Ser Gly Asn Arg Asp Pro Glu Ala Ile Glu
    370                 375                 380

aat ccg gac gcg ttc atc att gat cgc gcc agg ccg cgc cat cac ctc   1200
Asn Pro Asp Ala Phe Ile Ile Asp Arg Ala Arg Pro Arg His His Leu
385                 390                 395                 400

tcg ttc ggt ttc ggc att cac cgc tgt atg ggt aac agc ctt gcc gaa   1248
Ser Phe Gly Phe Gly Ile His Arg Cys Met Gly Asn Ser Leu Ala Glu
                405                 410                 415

ctg cag ctg cgt att ctg tgg gaa gag ctt ctc cct cgc ttt gaa aac   1296
Leu Gln Leu Arg Ile Leu Trp Glu Glu Leu Leu Pro Arg Phe Glu Asn

420                 425                 430

atc gaa gtg atc ggt gag ccg gag cgc gtg caa tcg aac ttt gtg cgg   1344
Ile Glu Val Ile Gly Glu Pro Glu Arg Val Gln Ser Asn Phe Val Arg
        435                 440                 445

ggc tat tcc aag atg atg gtt aag ttg acg gct aaa aaa taa           1386
Gly Tyr Ser Lys Met Met Val Lys Leu Thr Ala Lys Lys
    450                 455                 460

<210> 42
<211> 1386
<212> DNA
<213> Artificial Sequence

<220>
<223> Description of Artificial Sequence:hybrid gene

<220>
<221> CDS
<222> (1)..(1383)

<400> 42
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                 85                  90                  95

cag aag aac agt gct ttc ggg ccg ttc tgg tcg gta aca cgc tac gaa 336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc 384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc 432
Gln Ile Ile Leu Gly Asp Pro Pro Glu Gly Leu Ser Val Glu Met Phe
    130                 135                 140

atc gcc atg gac ccg ccc aag cac gac gtg cag cgc atg gcg gtc agt 480
Ile Ala Met Asp Pro Pro Lys His Asp Val Gln Arg Met Ala Val Ser
145                 150                 155                 160

ccg atc gtg gcg ccg gag aac ctt gcc aag ctc gaa ggt ctg atc cgc 528
Pro Ile Val Ala Pro Glu Asn Leu Ala Lys Leu Glu Gly Leu Ile Arg
                165                 170                 175

gag cgt acc ggt cgt gcg ctg gat ggc ctg ccg atc aac gag acc ttt 576
Glu Arg Thr Gly Arg Ala Leu Asp Gly Leu Pro Ile Asn Glu Thr Phe
            180                 185                 190

gac tgg gtc aag cgc gtt tcg atc aac ctg acg acg cag atg ctg gcg 624
Asp Trp Val Lys Arg Val Ser Ile Asn Leu Thr Thr Gln Met Leu Ala
        195                 200                 205

acg ctg ttt gat ttc cct tgg gaa gac cgt gcc aag ctg acg cgc tgg 672
Thr Leu Phe Asp Phe Pro Trp Glu Asp Arg Ala Lys Leu Thr Arg Trp
    210                 215                 220

tcg gat gtc gca acg gcg ctg gtc ggc acg ggc att att gat tcg gaa 720
Ser Asp Val Ala Thr Ala Leu Val Gly Thr Gly Ile Ile Asp Ser Glu
225                 230                 235                 240

gag cag cgc atg gag gag ctc aag ggg tgc gcg caa tac atg acc cgg 768
Glu Gln Arg Met Glu Glu Leu Lys Gly Cys Ala Gln Tyr Met Thr Arg
                245                 250                 255

ctg tgg aac gag cgc gtc aat gtg cca ccg ggc aat gat ctg ata tcg 816
Leu Trp Asn Glu Arg Val Asn Val Pro Pro Gly Asn Asp Leu Ile Ser
            260                 265                 270

atg atg gcg cac acc gag tcc atg cgc aac atg acg ccg gaa gag ttt 864
Met Met Ala His Thr Glu Ser Met Arg Asn Met Thr Pro Glu Glu Phe
        275                 280                 285

ctg ggc aac ctc ctt ttg ctg atc gtc ggc ggc aat gac acg acc cgc 912
Leu Gly Asn Leu Leu Leu Leu Ile Val Gly Gly Asn Asp Thr Thr Arg
    290                 295                 300

aac tcg atg acc ggc ggc gtg ctg gcg ctc aac gaa aat ccg gac gaa 960
Asn Ser Met Thr Gly Gly Val Leu Ala Leu Asn Glu Asn Pro Asp Glu
305                 310                 315                 320

tac cgc aag ctg tgc gcc aac ccg gcg ctg atc gcc tcc atg gtg ccg 1008
Tyr Arg Lys Leu Cys Ala Asn Pro Ala Leu Ile Ala Ser Met Val Pro
                325                 330                 335

gag atc gtt cgc tgg cag aca ccg ctg gcg cac atg cgg cgc acc gcg 1056
Glu Ile Val Arg Trp Gln Thr Pro Leu Ala His Met Arg Arg Thr Ala
            340                 345                 350

ctg caa gac acc gag ctc ggc ggc aag tcc att cgc aag ggt gac aaa 1104
Leu Gln Asp Thr Glu Leu Gly Gly Lys Ser Ile Arg Lys Gly Asp Lys
        355                 360                 365

gtc atc atg tgg tac gtc tcc ggc aac cgt gat ccc gaa gcg att gaa 1152
Val Ile Met Trp Tyr Val Ser Gly Asn Arg Asp Pro Glu Ala Ile Glu
    370                 375                 380

aat ccg gaa gcg ttc atc att gat cgc gcc aag ccg cgc cat cac ctc 1200
Asn Pro Glu Ala Phe Ile Ile Asp Arg Ala Lys Pro Arg His His Leu
385                 390                 395                 400

tcg ttc ggt ttc ggc att cac agc tgc atg ggg aat cgc ctt gcc gaa 1248
Ser Phe Gly Phe Gly Ile His Ser Cys Met Gly Asn Arg Leu Ala Glu
                405                 410                 415

ctg cag ctg cgt att ctg tgg gaa gag ctt ctc cct cgc ttt gaa aac 1296
Leu Gln Leu Arg Ile Leu Trp Glu Glu Leu Leu Pro Arg Phe Glu Asn
            420                 425                 430

atc gaa gtg atc ggt gag ccg gag cgc gtg caa tcg aac ttt gtg cgg 1344
Ile Glu Val Ile Gly Glu Pro Glu Arg Val Gln Ser Asn Phe Val Arg
        435                 440                 445

ggc tat tcc aag atg atg gtt aag ttg acg gct aaa aaa taa 1386
Gly Tyr Ser Lys Met Met Val Lys Leu Thr Ala Lys Lys
    450                 455                 460

<210> 43
<211> 1386

<212> DNA
<213> Artificial Sequence

<220>
<223> Description of Artificial Sequence:hybrid gene

<220>
<221> CDS
<222> (1)..(1383)

<400> 43
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                 85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

atc gcc atg gat ccg ccc aag cac gac gtg cag cgc atg gcg gtc agt   480

Ile Ala Met Asp Pro Pro Lys His Asp Val Gln Arg Met Ala Val Ser
145                 150                 155                 160

ccg atc gtg gcg ccg gag aac ctc gcc aag ctc gaa ggt ctg atc cgc     528
Pro Ile Val Ala Pro Glu Asn Leu Ala Lys Leu Glu Gly Leu Ile Arg
                165                 170                 175

gag cgt acc ggt cgt gcg ctg gat ggc ctg ccg atc aac gag acc ttt     576
Glu Arg Thr Gly Arg Ala Leu Asp Gly Leu Pro Ile Asn Glu Thr Phe
            180                 185                 190

gac tgg gtc aag cgc gtc tcg atc aac ctg acg acg cag atg ctg gcg     624
Asp Trp Val Lys Arg Val Ser Ile Asn Leu Thr Thr Gln Met Leu Ala
        195                 200                 205

acg ctg ttt gat ttc cct tgg gaa gac cgt gcc aag ctg acg cgc tgg     672
Thr Leu Phe Asp Phe Pro Trp Glu Asp Arg Ala Lys Leu Thr Arg Trp
    210                 215                 220

tcg gat gtc gca acg gcg ctg gtc ggc acg ggc att att gat tcg gaa     720
Ser Asp Val Ala Thr Ala Leu Val Gly Thr Gly Ile Ile Asp Ser Glu
225                 230                 235                 240

gag cag cgc atg gag gag ctc aag ggg tgc gcg caa tac atg acc cgg     768
Glu Gln Arg Met Glu Glu Leu Lys Gly Cys Ala Gln Tyr Met Thr Arg
                245                 250                 255

ctg tgg aac gag cgc gtc aat gtg cca ccg ggc aat gat ctg ata tcg     816
Leu Trp Asn Glu Arg Val Asn Val Pro Pro Gly Asn Asp Leu Ile Ser
            260                 265                 270

atg atg gcg cac acc gag tcc atg cgc aac atg acg ccg gaa gag ttt     864
Met Met Ala His Thr Glu Ser Met Arg Asn Met Thr Pro Glu Glu Phe
        275                 280                 285

ctg ggc aac ctc att ttg ctg atc gtc ggc ggc aat gac acg acc cgc     912
Leu Gly Asn Leu Ile Leu Leu Ile Val Gly Gly Asn Asp Thr Thr Arg
    290                 295                 300

aac tcg atg acc ggc ggc gtg ctg gcg atc aac gaa aat ccg gac gaa     960
Asn Ser Met Thr Gly Gly Val Leu Ala Ile Asn Glu Asn Pro Asp Glu
305                 310                 315                 320

tac cgc aag ctg tgc gcc aac ccg gcg ctg atc gcc tcc atg gtg ccg     1008
Tyr Arg Lys Leu Cys Ala Asn Pro Ala Leu Ile Ala Ser Met Val Pro
                325                 330                 335

gag atc gtt cgt tgg cag aca ccg ctg gcg cac atg cgg cgc acc gcg     1056

Glu Ile Val Arg Trp Gln Thr Pro Leu Ala His Met Arg Arg Thr Ala
        340                 345                 350

ctg cag gac acc gag ctc ggc ggc aag tcc att cgc aag ggt gac aag    1104
Leu Gln Asp Thr Glu Leu Gly Gly Lys Ser Ile Arg Lys Gly Asp Lys
    355                 360                 365

gtc atc atg tgg tat gtc tcc ggc aac cgt gat ccc gaa gcg att gaa    1152
Val Ile Met Trp Tyr Val Ser Gly Asn Arg Asp Pro Glu Ala Ile Glu
370                 375                 380

aat ccg gac gcg ttc atc att gat cgc gcc agg ccg cgc cat cac ctc    1200
Asn Pro Asp Ala Phe Ile Ile Asp Arg Ala Arg Pro Arg His His Leu
385                 390                 395                 400

tcg ttc ggt ttc ggc att cac cgc tgc atg ggt aat cgg ctt gcc gaa    1248
Ser Phe Gly Phe Gly Ile His Arg Cys Met Gly Asn Arg Leu Ala Glu
                405                 410                 415

ctg cag ctg cgt att ctg tgg gaa gag ctt ctc cct cgc ttt gaa aac    1296
Leu Gln Leu Arg Ile Leu Trp Glu Glu Leu Leu Pro Arg Phe Glu Asn
            420                 425                 430

atc gaa gtg atc ggt gag ccg gag cgc gtg caa tcg aac ttt gtg cgg    1344
Ile Glu Val Ile Gly Glu Pro Glu Arg Val Gln Ser Asn Phe Val Arg
        435                 440                 445

ggc tat tcc aag atg atg gtt aag ttg acg gct aaa aaa taa            1386
Gly Tyr Ser Lys Met Met Val Lys Leu Thr Ala Lys Lys
    450                 455                 460

<210> 44
<211> 1386
<212> DNA
<213> Artificial Sequence

<220>
<223> Description of Artificial Sequence:hybrid gene

<220>
<221> CDS
<222> (1)..(1383)

<400> 44
atg tca acg agt tca agt aca agt aat gac atc cag gca aaa ata att    48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta 96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct 144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg 192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag 240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat 288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa 336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc 384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc 432
Gln Ile Ile Leu Gly Asp Pro Pro Glu Gly Leu Ser Val Glu Met Phe
    130                 135                 140

atc gca atg gac ccg ccc aag cac gac gtg cag cgc atg gcg gtc agt 480
Ile Ala Met Asp Pro Pro Lys His Asp Val Gln Arg Met Ala Val Ser
145                 150                 155                 160

ccg atc gtg gcg ccg gag aac ctc gcc aag ctc gaa ggt ctg atc cgc 528
Pro Ile Val Ala Pro Glu Asn Leu Ala Lys Leu Glu Gly Leu Ile Arg
                165                 170                 175

gag cgt acc ggt cgt gcg ctg gat ggc ctg ccg atc aac gag acc ttt 576
Glu Arg Thr Gly Arg Ala Leu Asp Gly Leu Pro Ile Asn Glu Thr Phe
            180                 185                 190

gac tgg gtc aag cgc gtc tcg atc aac ctg acg acg cag atg ctg gcg 624
Asp Trp Val Lys Arg Val Ser Ile Asn Leu Thr Thr Gln Met Leu Ala
        195                 200                 205

acg ctg ttt gat ttc cct tgg gaa gac cgt gcc aag ctg acg cgc tgg 672
Thr Leu Phe Asp Phe Pro Trp Glu Asp Arg Ala Lys Leu Thr Arg Trp
    210                 215                 220

tcg gat gtc gca acg gcg ctg gtc ggc acg ggc att att gat tcg gaa 720
Ser Asp Val Ala Thr Ala Leu Val Gly Thr Gly Ile Ile Asp Ser Glu
225                 230                 235                 240

gag cag cgc atg gag gag ctc aag ggg tgc gcg caa tac atg acc cgg 768
Glu Gln Arg Met Glu Glu Leu Lys Gly Cys Ala Gln Tyr Met Thr Arg
                245                 250                 255

ctg tgg aac gag cgc gtc aat gtg cca ccg ggc aat gat ctg ata tcg 816
Leu Trp Asn Glu Arg Val Asn Val Pro Pro Gly Asn Asp Leu Ile Ser
            260                 265                 270

atg atg gcg cac acc gag tcc atg cgc aac atg acg ccg gaa gag ttt 864
Met Met Ala His Thr Glu Ser Met Arg Asn Met Thr Pro Glu Glu Phe
        275                 280                 285

ctg ggc aac ctc att ttg ctg atc gtc ggc ggc aat gac acg acc cgc 912
Leu Gly Asn Leu Ile Leu Leu Ile Val Gly Gly Asn Asp Thr Thr Arg
    290                 295                 300

aac tcg atg acc ggc ggc gtg ctg gcg atc aac gaa aat ccg gac gaa 960
Asn Ser Met Thr Gly Gly Val Leu Ala Ile Asn Glu Asn Pro Asp Glu
305                 310                 315                 320

tac cgc aag ctg tgc gcc aac ccg gcg ctg atc gcc tcc atg gtg ccg 1008
Tyr Arg Lys Leu Cys Ala Asn Pro Ala Leu Ile Ala Ser Met Val Pro
                325                 330                 335

gag atc gtg cgt tgg cag aca ccg ctg tcg cac atg cgg cgc acc gcg 1056
Glu Ile Val Arg Trp Gln Thr Pro Leu Ser His Met Arg Arg Thr Ala
            340                 345                 350

ctg cag gac acc gag ctc ggc ggc aag tcc att cgc aag ggt gac aag 1104
Leu Gln Asp Thr Glu Leu Gly Gly Lys Ser Ile Arg Lys Gly Asp Lys
        355                 360                 365

gtc atc atg tgg tat gtc tcc ggc aac cgt gat ccc gaa gcg att gaa 1152
Val Ile Met Trp Tyr Val Ser Gly Asn Arg Asp Pro Glu Ala Ile Glu
    370                 375                 380

aat ccg gaa gcg ttc atc att gat cgc gcc agg ccg cgc cat cac ctc 1200
Asn Pro Glu Ala Phe Ile Ile Asp Arg Ala Arg Pro Arg His His Leu
385                 390                 395                 400

tcg ttc ggt ttc ggc att cac cgc tgc atg ggg aat agg ctt gcc gaa 1248
Ser Phe Gly Phe Gly Ile His Arg Cys Met Gly Asn Arg Leu Ala Glu
                405                 410                 415

ctg cag ctg cgt att ctg tgg gaa gag ctt ctc cct cgc ttt gaa aac 1296
Leu Gln Leu Arg Ile Leu Trp Glu Glu Leu Leu Pro Arg Phe Glu Asn
            420                 425                 430

atc gaa gtg atc ggt gag ccg gag cgc gtg caa tcg aac ttt gtg cgg 1344
Ile Glu Val Ile Gly Glu Pro Glu Arg Val Gln Ser Asn Phe Val Arg
        435                 440                 445

ggc tat tcc aag atg atg gtt aag ttg acg gct aaa aaa taa 1386
Gly Tyr Ser Lys Met Met Val Lys Leu Thr Ala Lys Lys
    450                 455                 460

<210> 45
<211> 1386
<212> DNA
<213> Artificial Sequence

<220>
<223> Description of Artificial Sequence:hybrid gene

<220>
<221> CDS
<222> (1)..(1383)

<400> 45
atg tca acg agt tca agt aca agt aat gac atc cag gca aaa ata att 48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta 96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct 144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg 192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag 240

Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat    288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa    336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc    384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc    432
Gln Ile Ile Leu Gly Asp Pro Pro Glu Gly Leu Ser Val Glu Met Phe
    130                 135                 140

att gct atg gat cca ccc aag cac gac gtg cag cgc atg gcg gtc agt    480
Ile Ala Met Asp Pro Pro Lys His Asp Val Gln Arg Met Ala Val Ser
145                 150                 155                 160

ccg atc gtg gcg ccg gag aac ctc gcc aag ctc gaa ggt ctg atc cgc    528
Pro Ile Val Ala Pro Glu Asn Leu Ala Lys Leu Glu Gly Leu Ile Arg
                165                 170                 175

gag cgt acc tgc cgt gcg ctg gat ggc ctg ccg atc aac gag acc ttt    576
Glu Arg Thr Cys Arg Ala Leu Asp Gly Leu Pro Ile Asn Glu Thr Phe
            180                 185                 190

gac tgg gtc aag ctc gtt tcg atc aac ctg acg acg cag atg ctg gcg    624
Asp Trp Val Lys Leu Val Ser Ile Asn Leu Thr Thr Gln Met Leu Ala
        195                 200                 205

acg ctg ttt gat ttc cct tgg gaa gac cgt gcc aag ctg acg cgc tgg    672
Thr Leu Phe Asp Phe Pro Trp Glu Asp Arg Ala Lys Leu Thr Arg Trp
    210                 215                 220

tcg gat gtc gca acg gcg ctg gtc ggc acg ggc att att gat tcg gaa    720
Ser Asp Val Ala Thr Ala Leu Val Gly Thr Gly Ile Ile Asp Ser Glu
225                 230                 235                 240

gag cag cgc atg gag gag ctc aag ggg tgc gcg caa tac atg acc cgg    768
Glu Gln Arg Met Glu Glu Leu Lys Gly Cys Ala Gln Tyr Met Thr Arg
                245                 250                 255

ctg tgg aac gag cgc gtc aat gtg cca ccg ggc aat gat ctg ata tcg    816

Leu Trp Asn Glu Arg Val Asn Val Pro Pro Gly Asn Asp Leu Ile Ser
            260                 265                 270

atg atg gcg cac acc gag tcc atg cgc aac atg acg ccg gaa gag ttt 864
Met Met Ala His Thr Glu Ser Met Arg Asn Met Thr Pro Glu Glu Phe
        275                 280                 285

ctg ggc aac ctc att ttg ctg atc gtc ggc ggc aat gac acg acc cgc 912
Leu Gly Asn Leu Ile Leu Leu Ile Val Gly Gly Asn Asp Thr Thr Arg
    290                 295                 300

aac tcg atg acc ggc ggc gtg ccg gcg atc aac gaa aat ccg gac gaa 960
Asn Ser Met Thr Gly Gly Val Pro Ala Ile Asn Glu Asn Pro Asp Glu
305                 310                 315                 320

tac cgc aag ctg tgc gcc aac ccg gcg ctg atc gcc tcc atg gtg ccg 1008
Tyr Arg Lys Leu Cys Ala Asn Pro Ala Leu Ile Ala Ser Met Val Pro
                325                 330                 335

gag atc gtg cgt tgg cag aca ccg ctg tcg cac atg cgg cgc acc gcg 1056
Glu Ile Val Arg Trp Gln Thr Pro Leu Ser His Met Arg Arg Thr Ala
            340                 345                 350

ctg cag gac acc gag ctc ggc ggc aag tcc att cgc aag ggt gac aag 1104
Leu Gln Asp Thr Glu Leu Gly Gly Lys Ser Ile Arg Lys Gly Asp Lys
        355                 360                 365

gtc atc atg tgg tat gtc tcc ggc aac cgt gat ccc gaa gcg att gaa 1152
Val Ile Met Trp Tyr Val Ser Gly Asn Arg Asp Pro Glu Ala Ile Glu
    370                 375                 380

aat ccg aac gcg ttc atc att gat cgc gcc aag ccg cgc cat cac ctc 1200
Asn Pro Asn Ala Phe Ile Ile Asp Arg Ala Lys Pro Arg His His Leu
385                 390                 395                 400

tcg ttc ggt ttc ggc att cac cgt tgc atg ggg aac cgg ctt gcc gaa 1248
Ser Phe Gly Phe Gly Ile His Arg Cys Met Gly Asn Arg Leu Ala Glu
                405                 410                 415

ctg cag ctg cgt att ctg tgg gaa gag ctt ctc cct cgc ttt gaa aac 1296
Leu Gln Leu Arg Ile Leu Trp Glu Glu Leu Leu Pro Arg Phe Glu Asn
            420                 425                 430

atc gaa gtg atc ggt gag ccg gag cgc gtg caa tcg aac ttt gtg cgg 1344
Ile Glu Val Ile Gly Glu Pro Glu Arg Val Gln Ser Asn Phe Val Arg
        435                 440                 445

ggc tat tcc aag atg atg gtt aag ttg acg gct aaa aaa taa 1386

Gly Tyr Ser Lys Met Met Val Lys Leu Thr Ala Lys Lys
450                 455                 460

<210> 46
<211> 1386
<212> DNA
<213> Artificial Sequence

<220>
<223> Description of Artificial Sequence:hybrid gene

<220>
<221> CDS
<222> (1)..(1383)

<400> 46
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac atg atg aag gtg aag cgg aag acc att ggc act tcc cgc cct   144
Lys Asn Met Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                 85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttt 432
Gln Ile Ile Leu Gly Asp Pro Pro Glu Gly Leu Ser Val Glu Met Phe
    130                 135                 140

atc gcc atg gac ccc ccc aag cac gac gtg cag cgc atg gcg gtc agt 480
Ile Ala Met Asp Pro Pro Lys His Asp Val Gln Arg Met Ala Val Ser
145                 150                 155                 160

ccg atc gtg gcg ccg gag aac ctc gcc aag ctc gaa ggt ctg atc cgc 528
Pro Ile Val Ala Pro Glu Asn Leu Ala Lys Leu Glu Gly Leu Ile Arg
                165                 170                 175

gag cgt acc tgc cgt gcg ctg gat ggc ctg ccg atc aac gag acc ttt 576
Glu Arg Thr Cys Arg Ala Leu Asp Gly Leu Pro Ile Asn Glu Thr Phe
            180                 185                 190

gac tgg gtc aag ctc gtt tcg atc aac ctg acg acg cag atg ctg gcg 624
Asp Trp Val Lys Leu Val Ser Ile Asn Leu Thr Thr Gln Met Leu Ala
        195                 200                 205

acg ctg ttt gat ttc cct tgg gaa gac cgt gcc aag ctg acg cgc tgg 672
Thr Leu Phe Asp Phe Pro Trp Glu Asp Arg Ala Lys Leu Thr Arg Trp
    210                 215                 220

tcg gat gtc gca acg gcg ctg gtc ggc acg ggc att att gat tcg gaa 720
Ser Asp Val Ala Thr Ala Leu Val Gly Thr Gly Ile Ile Asp Ser Glu
225                 230                 235                 240

gag cag cgc atg gag gag ctc aag ggg tgc gcg caa tac atg acc cgg 768
Glu Gln Arg Met Glu Glu Leu Lys Gly Cys Ala Gln Tyr Met Thr Arg
                245                 250                 255

ctg tgg aac gag cgc gtc aat gtg cca ccg ggc aat gat ctg ata tcg 816
Leu Trp Asn Glu Arg Val Asn Val Pro Pro Gly Asn Asp Leu Ile Ser
            260                 265                 270

atg atg gcg cac acc gag tcc atg cgc aac atg acg ccg gaa gag ttt 864
Met Met Ala His Thr Glu Ser Met Arg Asn Met Thr Pro Glu Glu Phe
        275                 280                 285

ctg ggc aac ctc att ttg ctg atc gtc ggc ggc aat gac acg acc cgc 912
Leu Gly Asn Leu Ile Leu Leu Ile Val Gly Gly Asn Asp Thr Thr Arg
    290                 295                 300

aac tcg atg acc ggc ggc gtg ctg gcg atc aac gaa aat ccg gac gaa 960
Asn Ser Met Thr Gly Gly Val Leu Ala Ile Asn Glu Asn Pro Asp Glu
305                 310                 315                 320

tac cgc aag ctg tgc gcc aac ccg gcg ctg atc gcc tcc atg gtg ccg   1008
Tyr Arg Lys Leu Cys Ala Asn Pro Ala Leu Ile Ala Ser Met Val Pro
            325                 330                 335

gag atc gtg cgt tgg cag aca ccg ctg tcg cac atg cgg cgc acc gcg   1056
Glu Ile Val Arg Trp Gln Thr Pro Leu Ser His Met Arg Arg Thr Ala
        340                 345                 350

ctg cag gac acc gag ctc ggc ggc aag tcc att cgc aag ggt gac aag   1104
Leu Gln Asp Thr Glu Leu Gly Gly Lys Ser Ile Arg Lys Gly Asp Lys
    355                 360                 365

gtc atc atg tgg tat gtc tcc ggc aac cgt gat ccc gaa gcg att gaa   1152
Val Ile Met Trp Tyr Val Ser Gly Asn Arg Asp Pro Glu Ala Ile Glu
370                 375                 380

aat ccg gaa gcg ttc atc att gat cgc gcc agg ccg cgc cat cac ctc   1200
Asn Pro Glu Ala Phe Ile Ile Asp Arg Ala Arg Pro Arg His His Leu
385                 390                 395                 400

tcg ttc ggt ttc ggc att cac cgg tgc atg ggt aac cgt ctt gcc gaa   1248
Ser Phe Gly Phe Gly Ile His Arg Cys Met Gly Asn Arg Leu Ala Glu
            405                 410                 415

ctg cag ctg cgt att ctg tgg gaa gag ctt ctc cct cgc ttt gaa aac   1296
Leu Gln Leu Arg Ile Leu Trp Glu Glu Leu Leu Pro Arg Phe Glu Asn
        420                 425                 430

atc gaa gtg atc ggt gag ccg gag cgc gtg caa tcg aac ttt gtg cgg   1344
Ile Glu Val Ile Gly Glu Pro Glu Arg Val Gln Ser Asn Phe Val Arg
    435                 440                 445

ggc tat tcc aag atg atg gtt aag ttg acg gct aaa aaa taa           1386
Gly Tyr Ser Lys Met Met Val Lys Leu Thr Ala Lys Lys
450                 455                 460

<210> 47
<211> 1398
<212> DNA
<213> Artificial Sequence

<220>
<223> Description of Artificial Sequence:hybrid gene

<220>
<221> CDS

<222> (1)..(1395)

<400> 47
atg tca acg agt tca agt aca agt aat gac atc cag gca aaa ata att   48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta   96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc   432
Gln Ile Ile Leu Gly Asp Pro Pro Glu Gly Leu Ser Val Glu Met Phe
    130                 135                 140

atc gcg atg gac ccg ccc aaa cac gac gtg cac cgc gcc gcc gtg cag   480
Ile Ala Met Asp Pro Pro Lys His Asp Val His Arg Ala Ala Val Gln
145                 150                 155                 160

ccg gtg gtg gcg cca gcc aac ctt gcc aag ctc gag ccg atc att cgc   528
Pro Val Val Ala Pro Ala Asn Leu Ala Lys Leu Glu Pro Ile Ile Arg
                165                 170                 175

gag cgc gcg ggc cgt atc ttg gac gag ctg ccg atc ggc gag acc ctc   576

Glu Arg Ala Gly Arg Ile Leu Asp Glu Leu Pro Ile Gly Glu Thr Leu
            180                 185                 190

aac tgg gtg gac cgg gta tcg atc gaa ctg acc acg cag atg ctc gcc 624
Asn Trp Val Asp Arg Val Ser Ile Glu Leu Thr Thr Gln Met Leu Ala
        195                 200                 205

acg ctg ttc gat ttt ccg ttc gag caa cgg cgc aag ctc acg ttc tgg 672
Thr Leu Phe Asp Phe Pro Phe Glu Gln Arg Arg Lys Leu Thr Phe Trp
    210                 215                 220

tcg gat gcc acc acc ggc gcg ccg gac atc agc ggc ggc gat ttc att 720
Ser Asp Ala Thr Thr Gly Ala Pro Asp Ile Ser Gly Gly Asp Phe Ile
225                 230                 235                 240

acg ccc gag gaa cgc aac aag gcg ctg gcc gag tgc ggt gag acc ttc 768
Thr Pro Glu Glu Arg Asn Lys Ala Leu Ala Glu Cys Gly Glu Thr Phe
                245                 250                 255

acc cgc ctg tgg tac gag cgc gtc aac ggg ccg ccg ggc aac gac ctc 816
Thr Arg Leu Trp Tyr Glu Arg Val Asn Gly Pro Pro Gly Asn Asp Leu
            260                 265                 270

att tcc atg ttg gcg cac tcc gag cgc acc cgg aac atc atc gac tcg 864
Ile Ser Met Leu Ala His Ser Glu Arg Thr Arg Asn Ile Ile Asp Ser
        275                 280                 285

ccg atg gag tac ctc ggc aac atc ctg ttg ctg atc gtc ggc ggc aac 912
Pro Met Glu Tyr Leu Gly Asn Ile Leu Leu Leu Ile Val Gly Gly Asn
    290                 295                 300

gac acc acg cgc aac tcc att tct ggt ggc gtg cta gcg ctg aac cag 960
Asp Thr Thr Arg Asn Ser Ile Ser Gly Gly Val Leu Ala Leu Asn Gln
305                 310                 315                 320

aac ccc ggt gag tac gaa aaa ctg cgc cag aac ccc ggc ttg atc ccc 1008
Asn Pro Gly Glu Tyr Glu Lys Leu Arg Gln Asn Pro Gly Leu Ile Pro
                325                 330                 335

aat atg gtg tcc gag atc atc cgc tgg cag acg ccg atc ctg gcc atg 1056
Asn Met Val Ser Glu Ile Ile Arg Trp Gln Thr Pro Ile Leu Ala Met
            340                 345                 350

cgc cgg cgc gcc aag gag gac acg gag ctg ttc ggc aag ccg atc cgc 1104
Arg Arg Arg Ala Lys Glu Asp Thr Glu Leu Phe Gly Lys Pro Ile Arg
        355                 360                 365

aag aat gac aaa gtc gtc atg tgg tac gtc tcg ggt aac cgc gac gac 1152

Lys Asn Asp Lys Val Val Met Trp Tyr Val Ser Gly Asn Arg Asp Asp
    370                 375                 380

gag gtc atc gac gac ccg atg agc ttt cgg atc gac cgc gag aac gcc 1200
Glu Val Ile Asp Asp Pro Met Ser Phe Arg Ile Asp Arg Glu Asn Ala
385                 390                 395                 400

cgc cac cac ctg tcg ttc ggc ttc ggc atc cac cgc tgc atg ggt aat 1248
Arg His His Leu Ser Phe Gly Phe Gly Ile His Arg Cys Met Gly Asn
                405                 410                 415

cgt ctt gcc gaa ctg cag ctg cgt att ctg tgg gaa gag ctt ctc cct 1296
Arg Leu Ala Glu Leu Gln Leu Arg Ile Leu Trp Glu Glu Leu Leu Pro
            420                 425                 430

cgc ctt gaa aac atc gaa gtg atc ggt gag ccg gag cgc gtg caa tcg 1344
Arg Leu Glu Asn Ile Glu Val Ile Gly Glu Pro Glu Arg Val Gln Ser
        435                 440                 445

aac ttt gtg cgg ggc tat tcc aag atg atg gtt aag ttg acg gct aaa 1392
Asn Phe Val Arg Gly Tyr Ser Lys Met Met Val Lys Leu Thr Ala Lys
    450                 455                 460

aaa taa 1398
Lys
465

<210> 48
<211> 1383
<212> DNA
<213> Artificial Sequence

<220>
<223> Description of Artificial Sequence:hybrid gene

<220>
<221> CDS
<222> (1)..(1380)

<400> 48
atg tca acg agt tca agt aca agt aat gac atc cag gca aaa ata att 48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta 96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
        35                  40                  45

cag gtg cac ttt gtt gaa acc gat ctg cct gac gtc aat gat ttg gcg   192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat   288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa   336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc   384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttt   432
Gln Ile Ile Leu Gly Asp Pro Pro Glu Gly Leu Ser Val Glu Met Phe
    130                 135                 140

atc gcg atg gac ccc ccc aag cat gat gca cag cgc aag gtg gta agc   480
Ile Ala Met Asp Pro Pro Lys His Asp Ala Gln Arg Lys Val Val Ser
145                 150                 155                 160

ccg gtt gtc gcg cca aat aat ctt gaa acg ctg gag ccg atc att cgc   528
Pro Val Val Ala Pro Asn Asn Leu Glu Thr Leu Glu Pro Ile Ile Arg
                165                 170                 175

gag cgc gcc gcc gcc atc ctc gat tcg ctg ccg gtt ggg gaa gaa atc   576
Glu Arg Ala Ala Ala Ile Leu Asp Ser Leu Pro Val Gly Glu Glu Ile
            180                 185                 190

gac tgg gtt gac aag gtt tcc atc gag ctg acc acc atg acg ctg gcc   624
Asp Trp Val Asp Lys Val Ser Ile Glu Leu Thr Thr Met Thr Leu Ala
        195                 200                 205

act ctg ttc gat ttt ccc tgg gaa gaa cgg cgc aag ctg acc cgc tgg   672
Thr Leu Phe Asp Phe Pro Trp Glu Glu Arg Arg Lys Leu Thr Arg Trp
    210                 215                 220

tcc gac gtt ttg acc gca tca ccg ggc aat ggc gtc gat agc tgg gag 720
Ser Asp Val Leu Thr Ala Ser Pro Gly Asn Gly Val Asp Ser Trp Glu
225                 230                 235                 240

cag tgg cag cag gaa ttg acg gaa tgc ggc atg tat ttc aaa aat ctg 768
Gln Trp Gln Gln Glu Leu Thr Glu Cys Gly Met Tyr Phe Lys Asn Leu
                245                 250                 255

tgg gat cag cgc gtg ggc ggc gaa cag aaa cat gat ctg att tcc atg 816
Trp Asp Gln Arg Val Gly Gly Glu Gln Lys His Asp Leu Ile Ser Met
            260                 265                 270

ctc gcc ggt tca ccc gcc acg ccc aac atg ccg ttc cag gaa ttt ctg 864
Leu Ala Gly Ser Pro Ala Thr Pro Asn Met Pro Phe Gln Glu Phe Leu
        275                 280                 285

ggc aat ctg atg ctg ctg atc gtc ggc ggc aac gac aca acg cgc aat 912
Gly Asn Leu Met Leu Leu Ile Val Gly Gly Asn Asp Thr Thr Arg Asn
    290                 295                 300

tcc atc agt ggc ggc gtc ctg gcg ctt aac cag aac ccg aag gaa tac 960
Ser Ile Ser Gly Gly Val Leu Ala Leu Asn Gln Asn Pro Lys Glu Tyr
305                 310                 315                 320

gcc aag ctg atg aat gac cca ggc ctt atc ccc ggc atg gtg tcg gaa 1008
Ala Lys Leu Met Asn Asp Pro Gly Leu Ile Pro Gly Met Val Ser Glu
                325                 330                 335

atc atc cgc tgg cag agc ccc atc acg agc atg atg cgc aca gcc tta 1056
Ile Ile Arg Trp Gln Ser Pro Ile Thr Ser Met Met Arg Thr Ala Leu
            340                 345                 350

tgc gat gcc gag gtt ggc ggc aaa aag atc cgt gaa ggc gat aag gtc 1104
Cys Asp Ala Glu Val Gly Gly Lys Lys Ile Arg Glu Gly Asp Lys Val
        355                 360                 365

gtc atg tgg tat ctg tcc ggc aac cgt gac agc gag gtg atc gag cgg 1152
Val Met Trp Tyr Leu Ser Gly Asn Arg Asp Ser Glu Val Ile Glu Arg
    370                 375                 380

gcc gac gaa ttc ctg atc gac cgc aaa aac ccg cgt cag cat ctg tcc 1200
Ala Asp Glu Phe Leu Ile Asp Arg Lys Asn Pro Arg Gln His Leu Ser
385                 390                 395                 400

ttc ggt ttc ggc att cac cga tgc atg gga aac cgc ctt gcc gaa ctg 1248
Phe Gly Phe Gly Ile His Arg Cys Met Gly Asn Arg Leu Ala Glu Leu
                405                 410                 415

cag ctg cgt att ctg tgg gaa gag ctt ctc cct cgc ttt gaa aac atc   1296
Gln Leu Arg Ile Leu Trp Glu Glu Leu Leu Pro Arg Phe Glu Asn Ile
        420                 425                 430

gaa gtg atc ggt gag ccg gag cgc gtg caa tcg aac ttt gtg cgg ggc   1344
Glu Val Ile Gly Glu Pro Glu Arg Val Gln Ser Asn Phe Val Arg Gly
    435                 440                 445

tat tcc aag atg atg gtt aag ttg acg gct aaa aaa taa               1383
Tyr Ser Lys Met Met Val Lys Leu Thr Ala Lys Lys
  450                 455                 460

<210> 49
<211> 1386
<212> DNA
<213> Artificial Sequence

<220>
<223> Description of Artificial Sequence:hybrid gene

<220>
<221> CDS
<222> (1)..(1383)

<400> 49
atg tca acg agt tca agt aca agt aat gac atc cag gca aaa ata att   48
Met Ser Thr Ser Ser Ser Thr Ser Asn Asp Ile Gln Ala Lys Ile Ile
  1               5                  10                  15

aac gcc aca tcc aaa gtc gtg cca atg cat cta cag atc aag gca cta   96
Asn Ala Thr Ser Lys Val Val Pro Met His Leu Gln Ile Lys Ala Leu
             20                  25                  30

aaa aac ttg atg aag gtg aag cgg aag acc att ggc act tcc cgc cct   144
Lys Asn Leu Met Lys Val Lys Arg Lys Thr Ile Gly Thr Ser Arg Pro
         35                  40                  45

cag gtg cac ttt gtt gaa acc gat ttg cct gac gtc aat gat ttg gcg   192
Gln Val His Phe Val Glu Thr Asp Leu Pro Asp Val Asn Asp Leu Ala
     50                  55                  60

ata gaa gat atc gat acg agt aac cct ttt tta tac cga caa ggt aag   240
Ile Glu Asp Ile Asp Thr Ser Asn Pro Phe Leu Tyr Arg Gln Gly Lys
 65                  70                  75                  80

gcg aat gcg tac ttt aag cgg ttg cgt gat gaa gcg ccg gtg cac tat   288

Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa 336
Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc 384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc 432
Gln Ile Ile Leu Gly Asp Pro Pro Glu Gly Leu Ser Val Glu Met Phe
    130                 135                 140

atc gca atg gac ccg ccc aag cat gac gct cag cgc aag gca gtc agt 480
Ile Ala Met Asp Pro Pro Lys His Asp Ala Gln Arg Lys Ala Val Ser
145                 150                 155                 160

ccg gct gtc gcc ccg tcc aac ctt gtt ctg ctg gag ccc atc atc cgc 528
Pro Ala Val Ala Pro Ser Asn Leu Val Leu Leu Glu Pro Ile Ile Arg
                165                 170                 175

gag cgc gcg ggg ctt att ctc gat tcg ctt ccc gtc ggc gag gag atc 576
Glu Arg Ala Gly Leu Ile Leu Asp Ser Leu Pro Val Gly Glu Glu Ile
            180                 185                 190

gac tgg att gac cgg gtt tcc atc gag ctt acg acg atg acg ctg gcg 624
Asp Trp Ile Asp Arg Val Ser Ile Glu Leu Thr Thr Met Thr Leu Ala
        195                 200                 205

aca ctg ttc gac ttc ccc tgg gag gag cgg cgc aag ctc act cgc tgg 672
Thr Leu Phe Asp Phe Pro Trp Glu Glu Arg Arg Lys Leu Thr Arg Trp
    210                 215                 220

tcg gat gtt gcg acc gcg acg ccg gag acg ggc gtg gtt tct tcg ttt 720
Ser Asp Val Ala Thr Ala Thr Pro Glu Thr Gly Val Val Ser Ser Phe
225                 230                 235                 240

gag cag cgc cgc gag gag ctc ctc gaa tgt gcg caa tac ttc aag ggc 768
Glu Gln Arg Arg Glu Glu Leu Leu Glu Cys Ala Gln Tyr Phe Lys Gly
                245                 250                 255

ctt tgg gac aag cgg gcc aac gag ccg ccg aag ccc gat ctg atc tcg 816
Leu Trp Asp Lys Arg Ala Asn Glu Pro Pro Lys Pro Asp Leu Ile Ser
            260                 265                 270

atg atg gtg cat tcg ccc gcg acg cgg gac atg ccg tat ctc gaa ttt 864

Met Met Val His Ser Pro Ala Thr Arg Asp Met Pro Tyr Leu Glu Phe
        275                 280                 285

ctg ggc aac ctg att ctc ctg atc gtc ggc ggt aac gac acc acg cgc 912
Leu Gly Asn Leu Ile Leu Leu Ile Val Gly Gly Asn Asp Thr Thr Arg
    290                 295                 300

aat tcc atc agc ggc ggg gtg ctc gcc ctt aat cag aac ccg gcc gaa 960
Asn Ser Ile Ser Gly Gly Val Leu Ala Leu Asn Gln Asn Pro Ala Glu
305                 310                 315                 320

tac cgc aag ctc atg gcc gat cca ggc ctc atc ccg aaa atg atc ccc 1008
Tyr Arg Lys Leu Met Ala Asp Pro Gly Leu Ile Pro Lys Met Ile Pro
                325                 330                 335

gag atc atc cgc tgg cag acg ccg ctg acg cat atg cgt cgg acg gca 1056
Glu Ile Ile Arg Trp Gln Thr Pro Leu Thr His Met Arg Arg Thr Ala
            340                 345                 350

ctc atg gac gcc gaa atc ggt ggc aag aag atc aga aag ggc gac aaa 1104
Leu Met Asp Ala Glu Ile Gly Gly Lys Lys Ile Arg Lys Gly Asp Lys
        355                 360                 365

gtc gtt atg tgg tac ctc tcg gga aac cgg gac gac gag gta att gac 1152
Val Val Met Trp Tyr Leu Ser Gly Asn Arg Asp Asp Glu Val Ile Asp
    370                 375                 380

cgc ccg aac gag ttc att atc gac cgc ccg aat tcc cgt cac cat ctt 1200
Arg Pro Asn Glu Phe Ile Ile Asp Arg Pro Asn Ser Arg His His Leu
385                 390                 395                 400

tcc ttc ggt ttc ggc gtc cac cgg tgc atg ggg aat agg ctt gcc gaa 1248
Ser Phe Gly Phe Gly Val His Arg Cys Met Gly Asn Arg Leu Ala Glu
                405                 410                 415

ctg cag ctg cgt att ctg tgg gaa gag ctt ctc cct cgc ttt gaa aac 1296
Leu Gln Leu Arg Ile Leu Trp Glu Glu Leu Leu Pro Arg Phe Glu Asn
            420                 425                 430

atc gaa gtg atc ggt gag ccg gag cgc gtg caa tcg aac ttt gtg cgg 1344
Ile Glu Val Ile Gly Glu Pro Glu Arg Val Gln Ser Asn Phe Val Arg
        435                 440                 445

ggc tat tcc aag atg atg gtt aag ttg acg gct aaa aaa taa 1386
Gly Tyr Ser Lys Met Met Val Lys Leu Thr Ala Lys Lys
    450                 455                 460

<210> 50
<211> 1386
<212> DNA
<213> Artificial Sequence

<220>
<223> Description of Artificial Sequence:hybrid gene

<220>
<221> CDS
<222> (1)..(1383)

<400> 50
atg tca acg agt tca agt aca agt aat gac atc cag gca aaa ata att 48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta 96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct 144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg 192
Gln Val His Phe Val Glu Thr Asp Leu Pro Asp Val Asn Asp Leu Ala
    50                  55                  60

ata gag gat atc gat acg agt aac cct ttt tta tac cga caa ggt aag 240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat 288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca tgc tac gaa 336
Gln Lys Asn Ser Ala Phe Gly Pro Phe Trp Ser Val Thr Cys Tyr Glu
            100                 105                 110

gat atc gtc ttc gtg gac aag agc cat gat ttg ttt tcc gcc gaa ccc 384
Asp Ile Val Phe Val Asp Lys Ser His Asp Leu Phe Ser Ala Glu Pro
        115                 120                 125

caa att atc ttg ggt gat cct ccg gaa ggc ccg tcg gtt gaa atg ttc 432
Gln Ile Ile Leu Gly Asp Pro Pro Glu Gly Pro Ser Val Glu Met Phe
    130                 135                 140

atc gct atg gat ccc cca aag cac gac cag caa cgc aag gcc gtc acc 480
Ile Ala Met Asp Pro Pro Lys His Asp Gln Gln Arg Lys Ala Val Thr
145                 150                 155                 160

ccc gct gta gcg ccg tcc aac ttg ctg ttg ctc gaa ccg aca atc cgc 528
Pro Ala Val Ala Pro Ser Asn Leu Leu Leu Leu Glu Pro Thr Ile Arg
                165                 170                 175

gaa cga gca tgt cag att ctc gat gat ttg ccg ata gga gag gaa ttt 576
Glu Arg Ala Cys Gln Ile Leu Asp Asp Leu Pro Ile Gly Glu Glu Phe
            180                 185                 190

gat tgg gtt gac aag gtc tcg gtt gaa ttg acc acg atg acc ctt gcc 624
Asp Trp Val Asp Lys Val Ser Val Glu Leu Thr Thr Met Thr Leu Ala
        195                 200                 205

acc cta ttc gat ttc ccg tgg gaa gaa cgc cgc aag ctc acg cgc tgg 672
Thr Leu Phe Asp Phe Pro Trp Glu Glu Arg Arg Lys Leu Thr Arg Trp
    210                 215                 220

tct gac gtc acc acc gcc gct cct gaa acc ggc atc gtc gaa tcg tac 720
Ser Asp Val Thr Thr Ala Ala Pro Glu Thr Gly Ile Val Glu Ser Tyr
225                 230                 235                 240

gac gcg agg cgt gaa gaa ctc atc gag tgc gcg atg tat ttc aaa ggc 768
Asp Ala Arg Arg Glu Glu Leu Ile Glu Cys Ala Met Tyr Phe Lys Gly
                245                 250                 255

ctg tgg gag cag cgc atc aac gaa gaa ccg aag aac gat ctg att tcc 816
Leu Trp Glu Gln Arg Ile Asn Glu Glu Pro Lys Asn Asp Leu Ile Ser
            260                 265                 270

ata atg gcg cat tcc ccg gcg aca cgg gat atg ccc ttc ctt gaa ttt 864
Ile Met Ala His Ser Pro Ala Thr Arg Asp Met Pro Phe Leu Glu Phe
        275                 280                 285

ttg ggc aat ctc ctt ctg ctg atc gtg ggt ggc aac gac acc acg cgc 912
Leu Gly Asn Leu Leu Leu Leu Ile Val Gly Gly Asn Asp Thr Thr Arg
    290                 295                 300

aat tcg ata agc gga ggt gtg ctg gcg ctc aat cag aac ccc gat gaa 960
Asn Ser Ile Ser Gly Gly Val Leu Ala Leu Asn Gln Asn Pro Asp Glu
305                 310                 315                 320

tat cgc aaa ctg aat gac gac cct tcg ttg atc gcc agc atg gtg ccg 1008
Tyr Arg Lys Leu Asn Asp Asp Pro Ser Leu Ile Ala Ser Met Val Pro
                325                 330                 335

gaa atc atc cgg tgg cag acg ccg ctg act cac atg cgc cgc acc gcg   1056
Glu Ile Ile Arg Trp Gln Thr Pro Leu Thr His Met Arg Arg Thr Ala
            340                 345                 350

ctt cag gat tgg gag atc ggc ggc aag cag atc aag aag ggc gac aag   1104
Leu Gln Asp Trp Glu Ile Gly Gly Lys Gln Ile Lys Lys Gly Asp Lys
        355                 360                 365

gtc gtg atg tgg tac ctg tcg ggt aac cgc gac gac acg gtt atc gaa   1152
Val Val Met Trp Tyr Leu Ser Gly Asn Arg Asp Asp Thr Val Ile Glu
    370                 375                 380

cag gcc gac cgc ttc atc att gac cgc aag aat ccg cgc cat cac ctg   1200
Gln Ala Asp Arg Phe Ile Ile Asp Arg Lys Asn Pro Arg His His Leu
385                 390                 395                 400

tca ttc ggt tat ggc atc cac cgt tgt atg ggt aac cga ctt gcc gaa   1248
Ser Phe Gly Tyr Gly Ile His Arg Cys Met Gly Asn Arg Leu Ala Glu
                405                 410                 415

ctg cag ctg cgt att ctg tgg gaa gag ctt ctc cct cgc ttt gaa aac   1296
Leu Gln Leu Arg Ile Leu Trp Glu Glu Leu Leu Pro Arg Phe Glu Asn
            420                 425                 430

atc gaa gtg atc ggt gag ccg gag cgc gtg caa tcg aac ttt gtg cgg   1344
Ile Glu Val Ile Gly Glu Pro Glu Arg Val Gln Ser Asn Phe Val Arg
        435                 440                 445

ggc tat tcc aag atg atg gtt aag ttg acg gct aaa aaa taa           1386
Gly Tyr Ser Lys Met Met Val Lys Leu Thr Ala Lys Lys
    450                 455                 460


<210> 51
<211> 8
<212> PRT
<213> Artificial Sequence

<220>
<223> Description of Artificial Sequence: conserved amino acid sequence

<400> 51
Met Phe Ile Ala Met Asp Pro Pro
  1               5


<210> 52

<211> 8
<212> PRT
<213> Artificial Sequence

<220>
<223> Description of Artificial Sequence: conserved amino acid sequence

<400> 52
His Arg Cys Met Gly Asn Arg Leu
 1               5


<210> 53
<211> 22
<212> DNA
<213> Artificial Sequence

<220>
<223> Description of Artificial Sequence:primer

<400> 53
atgttyathg cnatggaycc nc                                          22


<210> 54
<211> 22
<212> DNA
<213> Artificial Sequence

<220>
<223> Description of Artificial Sequence:primer

<400> 54
gnggrtccat ngcdatraac at                                          22


<210> 55
<211> 24
<212> DNA
<213> Artificial Sequence

<220>
<223> Description of Artificial Sequence:primer

<400> 55
narnckrttn cccatrcanc krtg                                        24

<210> 56
<211> 23
<212> DNA
<213> Artificial Sequence

<220>
<223> Description of Artificial Sequence:primer

<400> 56
caymgntgya tgggnaaymg nyt                                          23


<210> 57
<211> 470
<212> PRT
<213> Alcanivorax borkumensis

<400> 57
Met Ser Thr Ser Ser Ser Thr Ser Asn Asp Ile Gln Ala Lys Ile Ile
 1               5                  10                  15

Asn Ala Thr Ser Lys Val Val Pro Met His Leu Gln Ile Lys Ala Leu
            20                  25                  30

Lys Asn Leu Met Lys Val Lys Arg Lys Thr Ile Gly Thr Ser Arg Pro
        35                  40                  45

Gln Val His Phe Val Glu Thr Asp Leu Pro Asp Val Asn Asp Leu Ala
    50                  55                  60

Ile Glu Asp Ile Asp Thr Ser Asn Pro Phe Leu Tyr Arg Gln Gly Lys
65                  70                  75                  80

Ala Asn Ala Tyr Phe Lys Arg Leu Arg Asp Glu Ala Pro Val His Tyr
                85                  90                  95

Gln Lys Asn Ser Ala Phe Gly Pro Phe Trp Ser Val Thr Arg Tyr Glu
            100                 105                 110

Asp Ile Val Phe Val Asp Lys Ser His Asp Leu Phe Ser Ala Glu Pro
        115                 120                 125

Gln Ile Ile Leu Gly Asp Pro Pro Glu Gly Leu Ser Val Glu Met Phe
    130                 135                 140

Ile Ala Met Asp Pro Pro Lys His Asp Val Gln Arg Arg Ala Val Gln
145                 150                 155                 160

Gly Val Val Ala Pro Lys Asn Leu Lys Glu Met Glu Gly Leu Ile Arg
                165                 170                 175

Lys Arg Thr Gly Asp Val Leu Asp Ser Leu Pro Leu Asp Thr Pro Phe
            180                 185                 190

Asn Trp Val Pro Val Val Ser Lys Glu Leu Thr Gly Arg Met Leu Ala
        195                 200                 205

Ser Leu Leu Asp Phe Pro Tyr Asp Glu Arg Glu Lys Leu Val Gly Trp
    210                 215                 220

Ser Asp Arg Leu Ser Gly Ala Ser Ser Ala Thr Gly Gly Glu Phe Thr
225                 230                 235                 240

Asn Glu Asp Val Phe Phe Asp Asp Ala Ala Asp Met Ala Trp Ala Phe
                245                 250                 255

Ser Lys Leu Trp Arg Asp Lys Glu Ala Arg Gln Lys Ala Gly Glu Glu
            260                 265                 270

Pro Gly Phe Asp Leu Ile Ser Met Leu Gln Ser Asn Glu Asp Thr Lys
        275                 280                 285

Asp Leu Ile Asn Arg Pro Leu Glu Phe Ile Gly Asn Leu Ala Leu Leu
    290                 295                 300

Ile Val Gly Gly Asn Asp Thr Thr Arg Asn Ser Met Ser Gly Gly Val
305                 310                 315                 320

Leu Ala Leu Asn Gln Phe Pro Glu Gln Phe Glu Lys Leu Lys Ala Asn
                325                 330                 335

Pro Lys Leu Ile Pro Asn Met Val Ser Glu Ile Ile Arg Trp Gln Thr
            340                 345                 350

Pro Leu Ala Tyr Met Arg Arg Val Ala Lys Gln Asp Val Glu Leu Asn
        355                 360                 365

Gly Gln Thr Ile Lys Lys Gly Asp Arg Val Leu Met Trp Tyr Ala Ser
    370                 375                 380

Gly Asn Gln Asp Glu Arg Lys Phe Glu Asn Pro Glu Gln Phe Ile Ile
385                 390                 395                 400

Asp Arg Lys Asp Thr Arg Asn His Val Ser Phe Gly Tyr Gly Val His
                405                 410                 415

Arg Cys Met Gly Asn Arg Leu Ala Glu Leu Gln Leu Arg Ile Leu Trp
            420                 425                 430

Glu Glu Leu Leu Pro Arg Phe Glu Asn Ile Glu Val Ile Gly Glu Pro
        435                 440                 445

Glu Arg Val Gln Ser Asn Phe Val Arg Gly Tyr Ser Lys Met Met Val
    450                 455                 460

Lys Leu Thr Ala Lys Lys
465                 470

<210> 58
<211> 1413
<212> DNA
<213> Alcanivorax borkumensis

<220>
<221> CDS
<222> (1)..(1410)

<400> 58
atg tca acg agt tca agt aca agt aat gac atc cag gca aaa ata att 48
Met Ser Thr Ser Ser Ser Thr Ser Asn Asp Ile Gln Ala Lys Ile Ile
1               5                   10                  15

aac gcc aca tcc aaa gtc gtg cca atg cat cta cag atc aag gca cta 96
Asn Ala Thr Ser Lys Val Val Pro Met His Leu Gln Ile Lys Ala Leu
            20                  25                  30

aaa aac ttg atg aag gtg aag cgg aag acc att ggc act tcc cgc cct 144
Lys Asn Leu Met Lys Val Lys Arg Lys Thr Ile Gly Thr Ser Arg Pro
        35                  40                  45

cag gtg cac ttt gtt gaa acc gat ttg cct gac gtc aat gat ttg gcg 192
Gln Val His Phe Val Glu Thr Asp Leu Pro Asp Val Asn Asp Leu Ala
    50                  55                  60

ata gaa gat atc gat acg agt aac cct ttt tta tac cga caa ggt aag 240
Ile Glu Asp Ile Asp Thr Ser Asn Pro Phe Leu Tyr Arg Gln Gly Lys
65                  70                  75                  80

gcg aat gcg tac ttt aag cgg ttg cgt gat gaa gcg ccg gtg cac tat 288
Ala Asn Ala Tyr Phe Lys Arg Leu Arg Asp Glu Ala Pro Val His Tyr
                85                  90                  95

cag aag aac agt gct ttc ggg ccg ttc tgg tcg gta aca cgc tac gaa 336

Gln Lys Asn Ser Ala Phe Gly Pro Phe Trp Ser Val Thr Arg Tyr Glu
            100                 105                 110

gat atc gtc ttc gtg gac aag agc cat gat ttg ttt tcc gcc gaa ccc 384
Asp Ile Val Phe Val Asp Lys Ser His Asp Leu Phe Ser Ala Glu Pro
        115                 120                 125

caa att atc ttg ggt gat cct ccg gaa ggc ctg tcg gtt gaa atg ttc 432
Gln Ile Ile Leu Gly Asp Pro Pro Glu Gly Leu Ser Val Glu Met Phe
    130                 135                 140

atc gct atg gat cct ccc aag cac gac gta cag cgt cgg gca gtc cag 480
Ile Ala Met Asp Pro Pro Lys His Asp Val Gln Arg Arg Ala Val Gln
145                 150                 155                 160

ggt gtt gtt gcg ccc aag aac ctg aaa gaa atg gaa gga ctg atc cgc 528
Gly Val Val Ala Pro Lys Asn Leu Lys Glu Met Glu Gly Leu Ile Arg
                165                 170                 175

aag cgc acc ggg gac gta ctg gat agc ctg ccg ttg gac act ccg ttc 576
Lys Arg Thr Gly Asp Val Leu Asp Ser Leu Pro Leu Asp Thr Pro Phe
            180                 185                 190

aac tgg gtg ccg gtg gtg tcg aaa gag ctg acc ggg cgc atg cta gcc 624
Asn Trp Val Pro Val Val Ser Lys Glu Leu Thr Gly Arg Met Leu Ala
        195                 200                 205

tca ctg tta gat ttc ccg tat gac gaa cgc gaa aaa ctg gtt ggc tgg 672
Ser Leu Leu Asp Phe Pro Tyr Asp Glu Arg Glu Lys Leu Val Gly Trp
    210                 215                 220

tcg gat cga ttg tcc ggc gcg tcc tcg gca acc ggc ggc gag ttt acg 720
Ser Asp Arg Leu Ser Gly Ala Ser Ser Ala Thr Gly Gly Glu Phe Thr
225                 230                 235                 240

aat gaa gat gtg ttt ttt gat gac gcg gca gat atg gcg tgg gct ttc 768
Asn Glu Asp Val Phe Phe Asp Asp Ala Ala Asp Met Ala Trp Ala Phe
                245                 250                 255

tcc aag ctt tgg cgt gat aaa gaa gcc cgt caa aaa gca ggt gaa gag 816
Ser Lys Leu Trp Arg Asp Lys Glu Ala Arg Gln Lys Ala Gly Glu Glu
            260                 265                 270

ccg ggt ttc gat ttg atc agc atg ctt cag tcc aat gaa gac aca aaa 864
Pro Gly Phe Asp Leu Ile Ser Met Leu Gln Ser Asn Glu Asp Thr Lys
        275                 280                 285

gat ctg atc aat cgt cct ttg gaa ttc att ggt aat ctc gcg ttg ttg 912

Asp Leu Ile Asn Arg Pro Leu Glu Phe Ile Gly Asn Leu Ala Leu Leu
    290                 295                 300

att gtt ggc ggt aat gac acc acg cgt aac tca atg agc ggg ggg gtg 960
Ile Val Gly Gly Asn Asp Thr Thr Arg Asn Ser Met Ser Gly Gly Val
305                 310                 315                 320

ctg gct tta aat cag ttc cca gag caa ttc gag aag cta aag gcg aac 1008
Leu Ala Leu Asn Gln Phe Pro Glu Gln Phe Glu Lys Leu Lys Ala Asn
                325                 330                 335

cca aag ctt atc ccc aat atg gtc tct gaa atc att cgc tgg caa acg 1056
Pro Lys Leu Ile Pro Asn Met Val Ser Glu Ile Ile Arg Trp Gln Thr
            340                 345                 350

ccg ctt gcg tat atg cgc cgg gtt gcc aag cag gat gtg gag ctg aac 1104
Pro Leu Ala Tyr Met Arg Arg Val Ala Lys Gln Asp Val Glu Leu Asn
        355                 360                 365

gga cag acc atc aag aag ggt gat cgc gtg ctg atg tgg tat gcg tcg 1152
Gly Gln Thr Ile Lys Lys Gly Asp Arg Val Leu Met Trp Tyr Ala Ser
    370                 375                 380

ggc aac cag gat gag aga aaa ttt gag aat cct gag caa ttc atc atc 1200
Gly Asn Gln Asp Glu Arg Lys Phe Glu Asn Pro Glu Gln Phe Ile Ile
385                 390                 395                 400

gac cgc aaa gat acg cgt aac cat gtg tcg ttt ggt tat ggg gtt cac 1248
Asp Arg Lys Asp Thr Arg Asn His Val Ser Phe Gly Tyr Gly Val His
                405                 410                 415

cgt tgt atg ggc aac cgc ctt gcc gaa ctg cag ctg cgt att ctg tgg 1296
Arg Cys Met Gly Asn Arg Leu Ala Glu Leu Gln Leu Arg Ile Leu Trp
            420                 425                 430

gaa gag ctt ctc cct cgc ttt gaa aac atc gaa gtg atc ggt gag ccg 1344
Glu Glu Leu Leu Pro Arg Phe Glu Asn Ile Glu Val Ile Gly Glu Pro
        435                 440                 445

gag cgc gtg caa tcg aac ttt gtg cgg ggc tat tcc aag atg atg gtt 1392
Glu Arg Val Gln Ser Asn Phe Val Arg Gly Tyr Ser Lys Met Met Val
    450                 455                 460

aag ttg acg gct aaa aaa taa 1413
Lys Leu Thr Ala Lys Lys
465                 470

<210> 59
<211> 22
<212> DNA
<213> Artificial Sequence

<220>
<223> Description of Artificial Sequence:primer

<400> 59
gccgatggag tttatgggca at                                                22


<210> 60
<211> 24
<212> DNA
<213> Artificial Sequence

<220>
<223> Description of Artificial Sequence:primer

<400> 60
gtaccacatc accaccttgt cgcc                                              24


<210> 61
<211> 23
<212> DNA
<213> Artificial Sequence

<220>
<223> Description of Artificial Sequence:primer

<400> 61
caccatgtca acgagttcaa gta                                               23


<210> 62
<211> 23
<212> DNA
<213> Artificial Sequence

<220>
<223> Description of Artificial Sequence:primer

<400> 62
tgattatttt ttagccgtca act                                               23

<210> 63
<211> 30
<212> DNA
<213> Artificial Sequence

<220>
<223> Description of Artificial Sequence:primer

<400> 63
cgtctcccat gtctaaagta gtgtatgtgt                                        30


<210> 64
<211> 30
<212> DNA
<213> Artificial Sequence

<220>
<223> Description of Artificial Sequence:primer

<400> 64
cgtctcatcg attaccattg cctatcggga                                        30


<210> 65
<211> 30
<212> DNA
<213> Artificial Sequence

<220>
<223> Description of Artificial Sequence:primer

<400> 65
cgtctcccat gaacgcaaac gacaacgtgg                                        30


<210> 66
<211> 30
<212> DNA
<213> Artificial Sequence

<220>
<223> Description of Artificial Sequence:primer

<400> 66
cgtctcatcg atcaggcact actcagttca                                        30

<210> 67
<211> 48
<212> DNA
<213> Rhdococcus sp.

<220>
<221> CDS
<222> (1)..(48)

<400> 67
gtg ctg cac cgg cat caa ccg gtc acc atc gga gaa ccc gcc gcc cgg   48
Val Leu His Arg His Gln Pro Val Thr Ile Gly Glu Pro Ala Ala Arg
1               5                   10                  15

<210> 68
<211> 16
<212> PRT
<213> > Rhdococcus sp.

<400> 68
Val Leu His Arg His Gln Pro Val Thr Ile Gly Glu Pro Ala Ala Arg
1               5                   10                  15

<210> 69
<211> 942
<212> DNA
<213> > Rhdococcus sp.

<220>
<221> CDS
<222> (1)..(939)

<400> 69
gcg gtg tcc cgc acc gtc acc gtc gag cgc ctg gac cgg atc gcc gac   48
Ala Val Ser Arg Thr Val Thr Val Glu Arg Leu Asp Arg Ile Ala Asp
1               5                   10                  15

gac gtg ctg cgc ctc gtc ctg cgc gac gcc ggc gga aag aca tta ccc   96
Asp Val Leu Arg Leu Val Leu Arg Asp Ala Gly Gly Lys Thr Leu Pro
            20                  25                  30

acg tgg act ccc ggc gcc cat atc gac ctc gac ctc ggc gcg ctg tcg   144
Thr Trp Thr Pro Gly Ala His Ile Asp Leu Asp Leu Gly Ala Leu Ser
        35                  40                  45

cgc cag tac tcc ctg tgc ggc gcg ccc gat gcg ccg agc tac gag att   192
Arg Gln Tyr Ser Leu Cys Gly Ala Pro Asp Ala Pro Ser Tyr Glu Ile
    50                  55                  60

gcc gtg cac ctg gat ccc gag agc cgc ggc ggt tcg cgc tac atc cac   240
Ala Val His Leu Asp Pro Glu Ser Arg Gly Gly Ser Arg Tyr Ile His
65                  70                  75                  80

gaa cag ctc gag gtg gga agc ccg ctc cgg atg cgc ggc cct cgg aac   288
Glu Gln Leu Glu Val Gly Ser Pro Leu Arg Met Arg Gly Pro Arg Asn
                85                  90                  95

cat ttc gcg ctc gac ccc ggc gcc gag cac tac gtg ttc gtc gcc ggc   336
His Phe Ala Leu Asp Pro Gly Ala Glu His Tyr Val Phe Val Ala Gly
            100                 105                 110

ggc atc ggc atc acc cca gtc ctg gcc atg gcc gac cac gcc cgc gcc   384
Gly Ile Gly Ile Thr Pro Val Leu Ala Met Ala Asp His Ala Arg Ala
        115                 120                 125

cgg ggg tgg agc tac gaa ctg cac tac tgc ggc cga aac cgt tcc ggc   432
Arg Gly Trp Ser Tyr Glu Leu His Tyr Cys Gly Arg Asn Arg Ser Gly
    130                 135                 140

atg gcc tat ctc gag cgt gtc gcc ggg cac ggt gac cgg gcc gcc ctg   480
Met Ala Tyr Leu Glu Arg Val Ala Gly His Gly Asp Arg Ala Ala Leu
145                 150                 155                 160

cac gtg tcc gag gaa ggc acc cgg atc gac ctc gcc gcc ctc ctc gcc   528
His Val Ser Glu Glu Gly Thr Arg Ile Asp Leu Ala Ala Leu Leu Ala
                165                 170                 175

gag ccc gcc ccc ggc gtc cag atc tac gcg tgc ggg ccc ggg cgg ctg   576
Glu Pro Ala Pro Gly Val Gln Ile Tyr Ala Cys Gly Pro Gly Arg Leu
            180                 185                 190

ctc gcc gga ctc gag gac gcg agc cgg aac tgg ccc gac ggg gcg ctg   624
Leu Ala Gly Leu Glu Asp Ala Ser Arg Asn Trp Pro Asp Gly Ala Leu
        195                 200                 205

cac gtc gag cac ttc acc tcg tcc ctc gcg gcg ctc gat ccg gac gtc   672
His Val Glu His Phe Thr Ser Ser Leu Ala Ala Leu Asp Pro Asp Val
    210                 215                 220

gag cac gcc ttc gac ctc gaa ctg cgt gac tcg ggg ctg acc gtg cgg   720
Glu His Ala Phe Asp Leu Glu Leu Arg Asp Ser Gly Leu Thr Val Arg
225                 230                 235                 240

gtc gaa ccc acc cag acc gtc ctc gac gcg ttg cgc gcc aac aac atc   768
Val Glu Pro Thr Gln Thr Val Leu Asp Ala Leu Arg Ala Asn Asn Ile
            245                 250                 255

gac gtg ccc agc gac tgc gag gaa ggc ctc tgc ggc tcg tgc gag gtc   816
Asp Val Pro Ser Asp Cys Glu Glu Gly Leu Cys Gly Ser Cys Glu Val
        260                 265                 270

gcc gtc ctc gac ggc gag gtc gac cat cgc gac acg gtg ctg acc aag   864
Ala Val Leu Asp Gly Glu Val Asp His Arg Asp Thr Val Leu Thr Lys
    275                 280                 285

gcc gag cgg gcg gcg aac cgg cag atg atg acc tgc tgc tcg cgt gcc   912
Ala Glu Arg Ala Ala Asn Arg Gln Met Met Thr Cys Cys Ser Arg Ala
290                 295                 300

tgt ggc gac cgg ctg gcc ctg cga ctc tga                           942
Cys Gly Asp Arg Leu Ala Leu Arg Leu
305                 310


<210> 70
<211> 313
<212> PRT
<213> > Rhdococcus sp.

<400> 70
Ala Val Ser Arg Thr Val Thr Val Glu Arg Leu Asp Arg Ile Ala Asp
1               5                   10                  15

Asp Val Leu Arg Leu Val Leu Arg Asp Ala Gly Gly Lys Thr Leu Pro
            20                  25                  30

Thr Trp Thr Pro Gly Ala His Ile Asp Leu Asp Leu Gly Ala Leu Ser
        35                  40                  45

Arg Gln Tyr Ser Leu Cys Gly Ala Pro Asp Ala Pro Ser Tyr Glu Ile
    50                  55                  60

Ala Val His Leu Asp Pro Glu Ser Arg Gly Gly Ser Arg Tyr Ile His
65                  70                  75                  80

Glu Gln Leu Glu Val Gly Ser Pro Leu Arg Met Arg Gly Pro Arg Asn
                85                  90                  95

His Phe Ala Leu Asp Pro Gly Ala Glu His Tyr Val Phe Val Ala Gly
            100                 105                 110

Gly Ile Gly Ile Thr Pro Val Leu Ala Met Ala Asp His Ala Arg Ala
        115                 120                 125

Arg Gly Trp Ser Tyr Glu Leu His Tyr Cys Gly Arg Asn Arg Ser Gly
    130                 135                 140

Met Ala Tyr Leu Glu Arg Val Ala Gly His Gly Asp Arg Ala Ala Leu
145                 150                 155                 160

His Val Ser Glu Glu Gly Thr Arg Ile Asp Leu Ala Ala Leu Leu Ala
                165                 170                 175

Glu Pro Ala Pro Gly Val Gln Ile Tyr Ala Cys Gly Pro Gly Arg Leu
            180                 185                 190

Leu Ala Gly Leu Glu Asp Ala Ser Arg Asn Trp Pro Asp Gly Ala Leu
        195                 200                 205

His Val Glu His Phe Thr Ser Ser Leu Ala Ala Leu Asp Pro Asp Val
    210                 215                 220

Glu His Ala Phe Asp Leu Glu Leu Arg Asp Ser Gly Leu Thr Val Arg
225                 230                 235                 240

Val Glu Pro Thr Gln Thr Val Leu Asp Ala Leu Arg Ala Asn Asn Ile
                245                 250                 255

Asp Val Pro Ser Asp Cys Glu Glu Gly Leu Cys Gly Ser Cys Glu Val
            260                 265                 270

Ala Val Leu Asp Gly Glu Val Asp His Arg Asp Thr Val Leu Thr Lys
        275                 280                 285

Ala Glu Arg Ala Ala Asn Arg Gln Met Met Thr Cys Cys Ser Arg Ala
    290                 295                 300

Cys Gly Asp Arg Leu Ala Leu Arg Leu
305                 310

<210> 71
<211> 29
<212> DNA
<213> Artificial Sequence

<220>
<223> Description of Artificial Sequence:primer

<400> 71
gggaattcgt gctgcaccgc catcaaccg                                    29


<210> 72
<211> 29
<212> DNA
<213> Artificial Sequence

<220>
<223> Description of Artificial Sequence:primer

<400> 72
gggagctctc agaggcgcag ggccaggcg                                    29


<210> 73
<211> 31
<212> DNA
<213> Artificial Sequence

<220>
<223> Description of Artificial Sequence:primer

<400> 73
cactagtcat atgtcaacga gttcaagtac a                                 31


<210> 74
<211> 29
<212> DNA
<213> Artificial Sequence

<220>
<223> Description of Artificial Sequence:primer

<400> 74
cagcaccaat tgttttttag ccgtcaactt                                   29


<210> 75
<211> 30
<212> DNA
<213> Artificial Sequence

<220>
<223> Description of Artificial Sequence:primer

<400> 75
gactagtcat atgacgactg aaaccataca                                        30

<210> 76
<211> 30
<212> DNA
<213> Artificial Sequence

<220>
<223> Description of Artificial Sequence:primer

<400> 76
gggaattcta ccgctttggt agtcgccgga                                        30

<210> 77
<211> 31
<212> DNA
<213> Artificial Sequence

<220>
<223> Description of Artificial Sequence:primer

<400> 77
cgatatacat atgttcagtt ttgaccccta t                                      31

<210> 78
<211> 26
<212> DNA
<213> Artificial Sequence

<220>
<223> Description of Artificial Sequence:primer

<400> 78
ggattgcgtt tgacgaattt cacaaa                                            26

<210> 79
<211> 26
<212> DNA
<213> Artificial Sequence

<220>
<223> Description of Artificial Sequence:primer

<400> 79
gggctcactt tgtgaaattc gtcaaa                                          26


<210> 80
<211> 26
<212> DNA
<213> Artificial Sequence

<220>
<223> Description of Artificial Sequence:primer

<400> 80
gggaattccg aaacggccaa ttccag                                          26


<210> 81
<211> 30
<212> DNA
<213> Artificial Sequence

<220>
<223> Description of Artificial Sequence:primer

<400> 81
ggactccata tgaccgatac cgccacgacg                                      30


<210> 82
<211> 32
<212> DNA
<213> Artificial Sequence

<220>
<223> Description of Artificial Sequence:primer

<400> 82
ctgaattccc aggtgaccgg gagttcgttg ac                                   32


<210> 83
<211> 30
<212> DNA
<213> Artificial Sequence

<220>
<223> Description of Artificial Sequence:primer

<400> 83
ggactccata tgacgaccgc agaacgcacc                                    30

<210> 84
<211> 32
<212> DNA
<213> Artificial Sequence

<220>
<223> Description of Artificial Sequence:primer

<400> 84
ctgaattccc aggcgatcgg cagcgagtgg ac                                 32

<210> 85
<211> 488
<212> PRT
<213> Nocardiaceae strain Hou_blue

<400> 85
Met Asn Val Ile Gly Ala Val Gln Lys Asn Leu Gln Lys Ser Val Val
 1               5                  10                  15

Gly Pro Val Arg Ser Lys Val Glu Gln Ile Val Pro Ile Asp Met Leu
            20                  25                  30

Ile Arg Gly Ala His Val Tyr Asn Arg Ala Arg Arg Val Val Asp Lys
        35                  40                  45

Arg Glu Arg Ser Pro Lys Phe Val Glu Ser Ala Ile Pro Asp Gly Ala
    50                  55                  60

Thr Ile Pro Leu Asp Glu Ile Asp Met Gly Asn Pro Phe Leu Tyr Arg
65                  70                  75                  80

Gln Gly Gln Trp Glu Ser His Phe Lys Arg Leu Arg Asp Glu Ser Pro
                85                  90                  95

Val His Phe Gln Asn Lys Ala Pro Phe Gly Pro Phe Trp Ser Val Thr
            100                 105                 110

Arg Tyr Glu Asp Ile Leu Ala Met Asp Lys Asn His His Val Phe Ser
        115                 120                 125

Ala Glu Pro Tyr Ile Ile Met Gly Lys Met Pro Ala Gly Phe Asp Ile
    130                 135                 140

Arg Met Phe Ile Ala Met Asp Pro Pro Gln His Asp Glu Gln Arg Asn
145                 150                 155                 160

Ala Val Gln Gly Val Val Ala Pro Lys Asn Leu Lys Glu Met Glu Ser
                165                 170                 175

Leu Ile Arg Ser Arg Val Arg Glu Val Leu Asp Gly Leu Pro Val Gly
            180                 185                 190

Glu Pro Phe Asn Trp Val Asp Lys Val Ser Ile Glu Leu Thr Gly Arg
        195                 200                 205

Met Leu Ala Thr Leu Leu Asp Tyr Leu Tyr Glu Asp Arg Arg Glu Leu
    210                 215                 220

Thr Glu Ile Ser Asp Leu Ala Ser Gly Val Pro Gly Ala Thr Gly Gly
225                 230                 235                 240

Glu Ser Asp Leu Asp Glu Thr Phe Ser Leu Ala Val Pro Leu Leu Thr
                245                 250                 255

Lys Phe Leu Arg Leu Trp Asn Glu Lys Lys Ala Arg Phe Glu Ala Gly
            260                 265                 270

Gln Pro Leu Gly Phe Asp Leu Ile Ser Leu Met Leu Ala Asp Pro Ala
        275                 280                 285

Thr Arg Asn Leu Ile Asp Glu Arg Pro Met Glu Phe Leu Gly Asn Ala
    290                 295                 300

Val Leu Leu Ile Val Gly Gly Asn Asp Thr Thr Arg Asn Ser Met Ser
305                 310                 315                 320

Gly Gly Val Tyr Ala Leu Asn Lys Trp Pro Asp Gln Phe Ala Lys Leu
                325                 330                 335

Lys Asp Asn Pro Lys Leu Ile Pro Asn Met Val Ser Glu Ile Ile Arg
            340                 345                 350

Trp Gln Thr Pro Leu Ala Tyr Met Ala Arg Arg Ala Lys Glu Asp Thr
        355                 360                 365

Val Val Gly Gly Gln Glu Ile Lys Lys Gly Glu Lys Leu Ile Met Trp
    370                 375                 380

Tyr Ser Ser Ala Asn Arg Asp Glu Arg His Phe Glu Asn Pro Glu Gln
385                 390                 395                 400

Leu Ile Ile Asp Arg Lys Asn Ala Arg Asn His Ile Ala Phe Gly Phe
            405                 410                 415

Gly Ile His Arg Cys Met Gly Asn Arg Leu Ala Glu Met Gln Leu Arg
        420                 425                 430

Ile Leu Trp Glu Glu Leu Leu Glu Arg Phe Asp Asp Ile Lys Val Leu
    435                 440                 445

Glu Glu Pro Glu Ile Val Gln Ser Thr Phe Val Arg Gly Tyr Ser Thr
450                 455                 460

Leu Met Val Glu Leu Thr Lys Lys Gln Ala Pro Val Glu Ser Pro Ala
465                 470                 475                 480

Val Glu Thr Glu Met Glu Gln Val
            485

<210> 86
<211> 1467
<212> DNA
<213> Nocardiaceae strain Hou_blue

<220>
<221> CDS
<222> (1)..(1464)

<400> 86
atg aac gta atc ggt gca gtt cag aag aat ctc cag aag agc gtc gtc      48
Met Asn Val Ile Gly Ala Val Gln Lys Asn Leu Gln Lys Ser Val Val
1               5                   10                  15

ggt ccg gtc cgg tcg aag gtc gag cag atc gtc ccg atc gac atg ctg      96
Gly Pro Val Arg Ser Lys Val Glu Gln Ile Val Pro Ile Asp Met Leu
            20                  25                  30

atc cgt gga gcg cac gtg tac aac cgt gcg cgc cgg gtc gta gac aag      144
Ile Arg Gly Ala His Val Tyr Asn Arg Ala Arg Arg Val Val Asp Lys
        35                  40                  45

cgg gag cgg tcg ccg aaa ttc gtc gaa tca gca att cct gac ggc gcg      192
Arg Glu Arg Ser Pro Lys Phe Val Glu Ser Ala Ile Pro Asp Gly Ala
    50                  55                  60

acc atc ccg ctc gac gag atc gac atg gga aac ccg ttc ctg tat cgc      240
Thr Ile Pro Leu Asp Glu Ile Asp Met Gly Asn Pro Phe Leu Tyr Arg

65                  70                  75                  80

cag ggg cag tgg gag tcg cac ttc aag cgt ctg cgg gac gag tcg ccg    288
Gln Gly Gln Trp Glu Ser His Phe Lys Arg Leu Arg Asp Glu Ser Pro
                85                  90                  95

gtg cac ttc cag aac aag gcg ccg ttc ggc ccg ttc tgg tcg gtc act    336
Val His Phe Gln Asn Lys Ala Pro Phe Gly Pro Phe Trp Ser Val Thr
            100                 105                 110

cgc tac gag gac atc ctc gcg atg gac aag aac cac cac gtg ttc tcc    384
Arg Tyr Glu Asp Ile Leu Ala Met Asp Lys Asn His His Val Phe Ser
        115                 120                 125

gcg gag ccg tac atc atc atg ggc aag atg ccg gcc ggc ttc gac atc    432
Ala Glu Pro Tyr Ile Ile Met Gly Lys Met Pro Ala Gly Phe Asp Ile
    130                 135                 140

cgg atg ttc att gcc atg gac ccg ccg cag cat gat gag cag cga aac    480
Arg Met Phe Ile Ala Met Asp Pro Pro Gln His Asp Glu Gln Arg Asn
145                 150                 155                 160

gcc gta cag gga gtc gtg gcg ccg aag aac ctc aag gag atg gag agc    528
Ala Val Gln Gly Val Val Ala Pro Lys Asn Leu Lys Glu Met Glu Ser
                165                 170                 175

ctg atc cgc tct cgc gtc cgc gag gtg ctc gac gga ctt ccg gtt ggc    576
Leu Ile Arg Ser Arg Val Arg Glu Val Leu Asp Gly Leu Pro Val Gly
            180                 185                 190

gag ccg ttc aac tgg gtt gac aag gtc tcg att gaa ctg acc ggt cgc    624
Glu Pro Phe Asn Trp Val Asp Lys Val Ser Ile Glu Leu Thr Gly Arg
        195                 200                 205

atg ctg gcg acc ctg ctg gac tac ctg tac gag gat cgt cga gag ctc    672
Met Leu Ala Thr Leu Leu Asp Tyr Leu Tyr Glu Asp Arg Arg Glu Leu
    210                 215                 220

acg gag att tcg gac ctg gca tcg ggt gtg ccg ggt gcg acc ggt ggc    720
Thr Glu Ile Ser Asp Leu Ala Ser Gly Val Pro Gly Ala Thr Gly Gly
225                 230                 235                 240

gag tcc gac ctc gat gag acg ttc tcc ctg gcg gtt ccg cta ctg acg    768
Glu Ser Asp Leu Asp Glu Thr Phe Ser Leu Ala Val Pro Leu Leu Thr
                245                 250                 255

aag ttc ctg cgc ctg tgg aac gag aag aag gcg cgt ttc gaa gcc ggc    816
Lys Phe Leu Arg Leu Trp Asn Glu Lys Lys Ala Arg Phe Glu Ala Gly

            260                 265                 270

cag ccg ctg ggc ttc gat ctg atc agc ctc atg ctg gcc gac ccg gcg 864
Gln Pro Leu Gly Phe Asp Leu Ile Ser Leu Met Leu Ala Asp Pro Ala
        275                 280                 285

acg cgg aac ctc atc gat gaa cgc ccg atg gag ttc ctc gga aac gcg 912
Thr Arg Asn Leu Ile Asp Glu Arg Pro Met Glu Phe Leu Gly Asn Ala
    290                 295                 300

gtg ctg ctg atc gtc ggt ggc aac gac acg acc cgt aac tcg atg tcg 960
Val Leu Leu Ile Val Gly Gly Asn Asp Thr Thr Arg Asn Ser Met Ser
305                 310                 315                 320

ggt ggc gtg tac gcg ctg aac aag tgg ccg gat cag ttc gcc aag ctc 1008
Gly Gly Val Tyr Ala Leu Asn Lys Trp Pro Asp Gln Phe Ala Lys Leu
                325                 330                 335

aag gac aac ccg aag ctg atc ccg aac atg gtt tcg gag atc atc cgc 1056
Lys Asp Asn Pro Lys Leu Ile Pro Asn Met Val Ser Glu Ile Ile Arg
            340                 345                 350

tgg cag acg ccg ctc gcc tac atg gcg cgt cgg gcc aag gaa gac acc 1104
Trp Gln Thr Pro Leu Ala Tyr Met Ala Arg Arg Ala Lys Glu Asp Thr
        355                 360                 365

gtt gtc ggt ggt cag gag atc aag aag ggc gag aag ctc atc atg tgg 1152
Val Val Gly Gly Gln Glu Ile Lys Lys Gly Glu Lys Leu Ile Met Trp
    370                 375                 380

tac tcc tcg gcc aac cgg gac gag cgc cac ttc gag aac ccg gaa cag 1200
Tyr Ser Ser Ala Asn Arg Asp Glu Arg His Phe Glu Asn Pro Glu Gln
385                 390                 395                 400

ctg atc atc gac cgc aag aat gcg cgt aac cac att gcg ttc ggc ttc 1248
Leu Ile Ile Asp Arg Lys Asn Ala Arg Asn His Ile Ala Phe Gly Phe
                405                 410                 415

ggc att cac cgc tgc atg ggg aac agg ttg gcc gag atg cag ctg cgc 1296
Gly Ile His Arg Cys Met Gly Asn Arg Leu Ala Glu Met Gln Leu Arg
            420                 425                 430

atc ctg tgg gaa gag ctg ctc gag cgt ttc gac gac atc aag gtc ctc 1344
Ile Leu Trp Glu Glu Leu Leu Glu Arg Phe Asp Asp Ile Lys Val Leu
        435                 440                 445

gaa gag ccg gag atc gtg cag tcg acc ttc gtg cgt ggc tac tcg acg 1392
Glu Glu Pro Glu Ile Val Gln Ser Thr Phe Val Arg Gly Tyr Ser Thr

450                 455                 460

ttg atg gtc gag ctc acc aag aag cag gca ccc gtt gag agc ccg gcg   1440
Leu Met Val Glu Leu Thr Lys Lys Gln Ala Pro Val Glu Ser Pro Ala
465                 470                 475                 480

gtc gag acc gag atg gaa cag gtc tag                               1467
Val Glu Thr Glu Met Glu Gln Val
                485

<210> 87
<211> 30
<212> DNA
<213> Artificial Sequence

<220>
<223> Description of Artificial Sequence:primer

<400> 87
gtaggccata tgaacgtaat cggtgcaggt                                    30

<210> 88
<211> 30
<212> DNA
<213> Artificial Sequence

<220>
<223> Description of Artificial Sequence:primer

<400> 88
catgaattcg acctgttcca tctcggtctc                                    30